# EUROPEAN PATENT SPECIFICATION

(11) **EP 3 352 795 B1**
(45) Date of publication and mention of the grant of the patent: **12.08.2020**
(21) Application number: 16849432.6
(22) Date of filing: 20.09.2016
(51) Int. Cl.: C12N 15/10

(54) **COMPOSITIONS AND METHODS FOR TARGET NUCLEIC ACID MODIFICATION**
ZUSAMMENSETZUNGEN UND VERFAHREN ZUR ZIELNUKLEINSÄUREMODIFIKATION
COMPOSITIONS ET MÉTHODES DE MODIFICATION D'ACIDES NUCLÉIQUES CIBLES

(30) Priority: 21.09.2015 US 201562221505 P; 23.10.2015 US 201562245808 P
(43) Date of publication of application: 01.08.2018
(73) Proprietor: The Regents of the University of California, Oakland, CA 94607 (US)
(72) Inventor: MURTHY, Niren, Berkeley, California 94704 (US); LEE, Kunwoo, Berkeley, CA 94703-2714 (US); CONBOY, Irina M., El Sobrante, California 94803 (US); CONBOY, Michael J., El Sobrante, California 94803 (US)
(74) Representative: Mewburn Ellis LLP
(86) International application number: PCT/US2016/052690
(87) International publication number: WO 2017/053312

(56) References cited:
- WO-A1-2014/065596
- WO-A1-2014/099744
- WO-A1-2016/123514
- WO-A2-2015/006747
- WO-A2-2015/048577
- WO-A2-2015/089419
- US-A1- 2015 283 265
- FLORIAN T. MERKLE ET AL: "Efficient CRISPR-Cas9-Mediated Generation of Knockin Human Pluripotent Stem Cells Lacking Undesired Mutations at the Targeted Locus", CELL REPORTS, vol. 11, no. 6, 30 April 2015 (2015-04-30) , pages 875-883, XP55431623, US ISSN: 2211-1247, DOI: 10.1016/j.celrep.2015.04.007
- NICOLE ALI MCNEER ET AL: "Nanoparticles that deliver triplex-forming peptide nucleic acid molecules correct F508del CFTR in airway epithelium", NATURE COMMUNICATIONS, vol. 6, 27 April 2015 (2015-04-27), page 6952, XP55372725, GB ISSN: 2041-1723, DOI: 10.1038/ncomms7952

## Description

### STATEMENT REGARDING FEDERALLY SPONSORED RESEARCH

This invention was made with government support under Grant Nos. AI119115, AI117064, and AG048316, awarded by the National Institutes of Health. The government has certain rights in the invention.

### INTRODUCTION

RNA-mediated adaptive immune systems in bacteria and archaea rely on Clustered Regularly Interspaced Short Palindromic Repeat (CRISPR) genomic loci and CRISPR-associated (Cas) proteins that function together to provide protection from invading viruses and plasmids. In Type II CRISPR-Cas systems, the Cas9 protein functions as an RNA-guided endonuclease that uses a dual-guide RNA consisting of crRNA and *trans*-activating crRNA (tracrRNA) for target recognition and cleavage by a mechanism involving two nuclease active sites that together generate double-stranded DNA breaks (DSBs).

RNA-programmed Cas9 has proven to be a versatile tool for genome engineering in multiple cell types and organisms. Guided by a dual-RNA complex or a chimeric single-guide RNA, Cas9 (or variants of Cas9 such as nickase variants) can generate site-specific DSBs or single-stranded breaks (SSBs) within target nucleic acids. Target nucleic acids can include double-stranded DNA (dsDNA) and single-stranded DNA (ssDNA) as well as RNA. When cleavage of a target nucleic acid occurs within a cell (e.g., a eukaryotic cell), the break in the target nucleic acid can be repaired by non-homologous end joining (NHEJ) or homology directed repair (HDR). F. Merkle et al., Cell Reports, 11,6, pp. 875-883, 2015; N. McNeer et al., Nature Commun.,6, pp. 6952, 2015 and WO2014/099744 A1, describe methods for modifying genomic nucleic acids.

Thus, the Cas9 system provides a facile means of modifying genomic information. In addition, catalytically inactive Cas9 alone or fused to transcriptional activator or repressor domains can be used to alter transcription levels at sites within target nucleic acids by binding to the target site without cleavage.

### SUMMARY

The present disclosure provides a complex comprising a nanoparticle; a Type II or a Type V CRISPR system comprising a site-directed DNA-modifying polypeptide and a guide RNA; and a polycation-based endosomal escape polymer. The present disclosure provides methods of making and using a complex of the present disclosure.

The present disclosure provides a complex comprising a nanoparticle; a Type II CRISPR system comprising a Cas9 polypeptide and a guide RNA; and a polycation-based endosomal escape polymer. The present disclosure provides methods of making and using a complex of the present disclosure.

The present disclosure provides a complex comprising a nanoparticle; a Type V CRISPR system comprising a Cpfl polypeptide and a guide RNA; and a polycation-based endosomal escape polymer. The present disclosure provides methods of making and using a complex of the present disclosure.

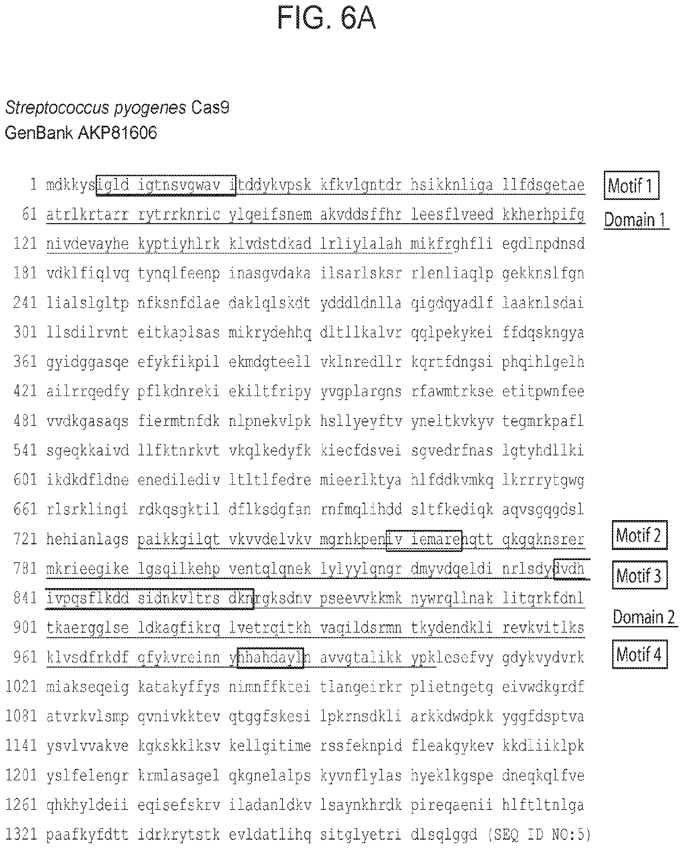

The present disclosure provides a complex (e.g., an encapsulated complex) comprising: a) nanoparticle-nucleic acid conjugate; b) a Type II CRISPR system comprising: i) a Cas9 polypeptide; and ii) a guide RNA; and c) an endosomal disruptive polymer. The nanoparticle-nucleic acid conjugate and the Type II CRISPR system (comprising: i) a Cas9 polypeptide; and ii) a guide RNA) form a complex, and are encapsulated in the endosomal disruptive polymer. In some cases, the nanoparticle is a colloidal metal nanoparticle. In some cases, the colloidal metal nanoparticle is a gold nanoparticle. In some cases, the complex further comprises a donor polynucleotide (e.g., a DNA donor template). In some cases, the encapsulated complex further comprises a silicate; for example, in some cases, the endosomal disruptive polymer and the silicate encapsulate the Type II CRISPR system. In some cases, the endosomal disruptive polymer is a cationic polymer selected from the group consisting of polyethylene imine, poly(arginine), poly(lysine), poly(histidine), poly-[2-{(2-aminoethyl)amino}-ethyl-aspartamide] (pAsp(DET)), a block co-polymer of poly(ethylene glycol) (PEG) and poly(arginine), a block co-polymer of PEG and poly(lysine), and a block co-polymer of PEG and poly{*N*-[*N*-(2-aminoethyl)-2-aminoethyl]aspartamide} (PEG-pAsp(DET)). In some cases, the endosomal disruptive polymer is poly{*N*-[*N*-(2-aminoethyl)-2-aminoethyl]aspartamide} (PAsp(DET). In some cases, the nanoparticle has a diameter in the range of 10 nm to 1000 nm. In some cases, the nanoparticle has a diameter in the range of 10 nm to 50 nm. In some cases, the Cas9 polypeptide comprises an amino acid sequence having at least 25% (e.g., at least 30%, at least 40%, at least 50%, at least 60%, at least 70%, at least 75%, at least 80%, at least 85%, at least 90%, at least 95%, or more than 95%) amino acid sequence identity to an amino acid sequence set forth in Fig. 6A-6J (SEQ ID NOs:5-14). In some cases, the Cas9 polypeptide is enzymatically active. In some cases, the Cas9 polypeptide exhibits reduced enzymatic activity relative to a wild-type Cas9 polypeptide, and wherein the Cas9 polypeptide retains target nucleic acid binding activity. In some cases, the Cas9 polypeptide comprises a nuclear localization signal. In some cases, the guide RNA is a single-molecule guide RNA. In some cases, the guide RNA is a dual-molecule guide RNA.

The present disclosure provides a complex (e.g., an encapsulated complex) comprising: a) a colloidal metal nanoparticle-nucleic acid conjugate; b) a Type II CRISPR system comprising: i) a Cas9 polypeptide; and ii) a guide RNA; and c) an endosomal disruptive polymer. The colloidal metal nanoparticle-nucleic acid conjugate and the Type II CRISPR system (comprising: i) a Cas9 polypeptide; and ii) a guide RNA) form a complex, and are encapsulated in the endosomal disruptive polymer. In some cases, the colloidal metal nanoparticle is a gold nanoparticle. In some cases, the complex further comprises a donor polynucleotide (e.g., a DNA donor template). In some cases, the encapsulated complex further comprises a silicate; for example, in some cases, the endosomal disruptive polymer and the silicate encapsulate the Type II CRISPR system. In some cases, the endosomal disruptive polymer is a cationic polymer selected from the group consisting of polyethylene imine, poly(arginine), poly(lysine), poly(histidine), poly-[2-{(2-aminoethyl)amino}-ethyl-aspartamide] (pAsp(DET)), a block co-polymer of poly(ethylene glycol) (PEG) and poly(arginine), a block co-polymer of PEG and poly(lysine), and a block co-polymer of PEG and poly{*N*-[*N*-(2-aminoethyl)-2-aminoethyl]aspartamide} (PEG-pAsp(DET)). In some cases, the endosomal disruptive polymer is poly{*N*-[*N*-(2-aminoethyl)-2-aminoethyl]aspartamide} (PAsp(DET). In some cases, the colloidal metal nanoparticle has a diameter in the range of 10 nm to 1000 nm. In some cases, the colloidal metal nanoparticle has a diameter in the range of 10 nm to 50 nm. In some cases, the Cas9 polypeptide comprises an amino acid sequence having at least 25% (e.g., at least 30%, at least 40%, at least 50%, at least 60%, at least 70%, at least75%, at least 80%, at least 85%, at least 90%, at least 95%, or more than 95%) amino acid sequence identity to an amino acid sequence set forth in Fig. 6A-6J (SEQ ID NOs:5-14). In some cases, the Cas9 polypeptide is enzymatically active. In some cases, the Cas9 polypeptide exhibits reduced enzymatic activity relative to a wild-type Cas9 polypeptide, and wherein the Cas9 polypeptide retains target nucleic acid binding activity. In some cases, the Cas9 polypeptide comprises a nuclear localization signal. In some cases, the guide RNA is a single-molecule guide RNA. In some cases, the guide RNA is a dual-molecule guide RNA.

The present disclosure provides a method of producing the complex of any of claims 1 to 14, the method comprising: contacting a Type II CRISPR system comprising: a ribonucleoprotein (RNP) comprising a Cas9 polypeptide and a guide RNA (gRNA), with a colloidal metal nanoparticle (NP)-nucleic acid conjugate, under conditions sufficient to generate a NP-nucleic acid-RNP complex; and ii) encapsulating the NP-nucleic acid-RNP complex within one or more layers of an endosomal disruptive polymer. In some cases, the Type II CRISPR system comprises a donor polynucleotide.

The present disclosure provides a method of binding a target nucleic acid, comprising:
contacting a cell comprising a target nucleic acid with a complex (e.g., an encapsulated complex) as described above or elsewhere herein, wherein the complex enters the cell, and wherein the guide RNA and Cas9 polypeptide are released from the complex in an endosome in the cell. In some cases, the cell is *in vitro.* In some cases, the cell is *in vivo.* In some cases, the Cas9 fusion polypeptide modulates transcription from the target nucleic acid. In some cases, the Cas9 fusion polypeptide modifies the target nucleic acid. In some cases, the Cas9 fusion polypeptide cleaves the target nucleic acid. In some cases, the complex (e.g., the encapsulated complex) comprises a donor template polynucleotide, and the method comprises contacting the target nucleic acid with the donor template polynucleotide.

The present disclosure provides a method of genetically modifying a target cell, comprising:
contacting a target cell with a complex (e.g., an encapsulated complex) as described above or elsewhere herein. In some cases, the target cell is an *in vivo* target cell. In some cases, the target cell is a plant cell. In some cases, the target cell is an animal cell. In some cases, the target cell is a mammalian cell. In some cases, the target cell is a myoblast, a myofiber, a neuron, a chondrocyte, a lymphocyte, an epithelial cell, an adipocyte, or a keratinocyte. In some cases, the target cell is pluripotent cell.

### BRIEF DESCRIPTION OF THE DRAWINGS

**FIGs. 1A** **and** **1B** are a collection of images and schematic diagrams showing the synthesis of Gold-Cas9 and intracellular release of Cas9 ribonucleoproteins (RNPs), according to embodiments of the present disclosure.
**FIGs. 2A and 2B** are a collection of images showing loading of active Cas9/guide RNA (gRNA) RNP and release of the same from gold nanoparticles (GNPs), according to embodiments of the present disclosure.
**FIGs. 3A-3F** are a collection of images and figures showing delivery of and gene editing by Cas9 using GNPs in cultured cells, according to embodiments of the present disclosure. FIG. 3F: ACCACCGTGACGTACGGC (SEQ ID NO: 1137); and ACCACCCTGACCCATGGC (SEQ ID NO: 1138).
**FIGs. 4A-4F** are a collection of images and figures showing delivery of and gene editing by Cas9 using GNPs in various cell types, according to embodiments of the present disclosure.
**FIGs. 5A-5D** are a collection of images showing *in vivo* delivery of Cas9, according to embodiments of the present disclosure.
**FIGs. 6A-6J** are a collection of figures showing amino acid sequences of *Streptococcus pyogenes* Cas9, and variants thereof.
FIG. 7 depicts a multiple sequence alignment of motifs 1-4 of Cas9 proteins from various diverse species. *S. pyogenes* Motif 1: IGLDIGTNSVGWAVI (SEQ ID NO:1); Motif 2: IVIEMARE (SEQ ID NO:2); Motif 3: DVDHIVPQSFLKDDSIDNKVLTRSDKN (SEQ ID NO:3); and Motif 4: HHAHDAYL (SEQ ID NO:4).
**FIGs. 8A-8C** list examples of suitable fusion partners (or fragments thereof) for a subject Cas9 polypeptide (e.g., wild type Cas9, variant Cas9). Examples include, but are not limited to those listed.
**FIG. 9** provides an amino acid sequence of Cpfl from *Francisella tularensis* subsp. novicida U112 (SEQ ID NO: 1123).
**FIGs. 10A-10B** depict use of CRISPR-gold particles to correct the human Duchenne Muscular Dystrophy (DMD) mutation in dystrophin gene, using the mouse model of human dystrophin mutation (MDX mice) *in vivo:* dystrophin protein (lacking in DMD/MDX) becomes expressed in most/all muscle fibers (myofibers) at the site of CRISPR-gold particles injection after a single application. Negative control particles, which had no Cas9 did nor restore the expression of dystrophin.
**FIGs. 11A-11C** depict use of CRISPR-gold to improve symptoms related to Duchenne Muscular Dystrophy (DMD) in the mouse model of the human disease.
**FIG. 12** demonstrates the effect of gold nanoparticle size on HDR efficiency.
**FIG. 13** depicts synthesis of CRIPSR-Gold using as an example CXCR4 donor DNA (SEQ ID NO: 1114) and Gold nanoparticles conjugated with 5' thiol modified DNA (SEQ ID NO: 1112)
**FIG. 14** depicts the effect of the amount of donor DNA in CRISPR-Gold on the HDR frequency of CRISPR-Gold treatment.
**FIG. 15** depicts the sequence of genomic DNA targeted in the mdx mouse and the sequence of the donor DNA. Targeted DNA: AGTTCTTTGAAAGAGCAATAAAATGGCTTC (SEQ ID NO: 1099); donor DNA: AGTTCTTTAAAGGAGCAGCAGAATGGCTTC (SEQ ID NO: 1100).
**FIG. 16** depicts off-target editing frequency.

### DEFINITIONS

The terms "polynucleotide" and "nucleic acid," used interchangeably herein, refer to a polymeric form of nucleotides of any length, either ribonucleotides or deoxyribonucleotides. Thus, this term includes, but is not limited to, single-, double-, or multi-stranded DNA or RNA, genomic DNA, cDNA, DNA-RNA hybrids, or a polymer comprising purine and pyrimidine bases or other natural, chemically or biochemically modified, non-natural, or derivatized nucleotide bases.

By "hybridizable" or "complementary" or "substantially complementary" it is meant that a nucleic acid (e.g. RNA, DNA) comprises a sequence of nucleotides that enables it to non-covalently bind, i.e. form Watson-Crick base pairs and/or G/U base pairs, "anneal", or "hybridize," to another nucleic acid in a sequence-specific, antiparallel, manner (i.e., a nucleic acid specifically binds to a complementary nucleic acid) under the appropriate in vitro and/or in vivo conditions of temperature and solution ionic strength. Standard Watson-Crick base-pairing includes: adenine (A) pairing with thymidine (T), adenine (A) pairing with uracil (U), and guanine (G) pairing with cytosine (C) [DNA, RNA]. In addition, for hybridization between two RNA molecules (e.g., dsRNA), and for hybridization of a DNA molecule with an RNA molecule: guanine (G) can also base pair with uracil (U). For example, G/U base-pairing is partially responsible for the degeneracy (i.e., redundancy) of the genetic code in the context of tRNA anti-codon base-pairing with codons in mRNA. Thus, in the context of this disclosure, a guanine (G) (e.g., of a protein-binding segment (dsRNA duplex) of a guide nucleic acid molecule; of a target nucleic acid base pairing with a guide nucleic acid, etc.) is considered complementary to both a uracil (U) and to an adenine (A). For example, when a G/U base-pair can be made at a given nucleotide position of a protein-binding segment (e.g., dsRNA duplex) of a subject guide nucleic acid molecule, the position is not considered to be non-complementary, but is instead considered to be complementary.

Hybridization and washing conditions are well known and exemplified in Sambrook, J., Fritsch, E. F. and Maniatis, T. Molecular Cloning: A Laboratory Manual, Second Edition, Cold Spring Harbor Laboratory Press, Cold Spring Harbor (1989), particularly Chapter 11 and Table 11.1 therein; and Sambrook, J. and Russell, W., Molecular Cloning: A Laboratory Manual, Third Edition, Cold Spring Harbor Laboratory Press, Cold Spring Harbor (2001). The conditions of temperature and ionic strength determine the "stringency" of the hybridization.

Hybridization requires that the two nucleic acids contain complementary sequences, although mismatches between bases are possible. The conditions appropriate for hybridization between two nucleic acids depend on the length of the nucleic acids and the degree of complementarity, variables well known in the art. The greater the degree of complementarity between two nucleotide sequences, the greater the value of the melting temperature (Tm) for hybrids of nucleic acids having those sequences. For hybridizations between nucleic acids with short stretches of complementarity (e.g. complementarity over 35 or less, 30 or less, 25 or less, 22 or less, 20 or less, or 18 or less nucleotides) the position of mismatches can become important (see Sambrook et al., supra, 11.7-11.8). Typically, the length for a hybridizable nucleic acid is 8 nucleotides or more (e.g., 10 nucleotides or more, 12 nucleotides or more, 15 nucleotides or more, 20 nucleotides or more, 22 nucleotides or more, 25 nucleotides or more, or 30 nucleotides or more). The temperature and wash solution salt concentration may be adjusted as necessary according to factors such as length of the region of complementation and the degree of complementation.

It is understood that the sequence of a polynucleotide need not be 100% complementary to that of its target nucleic acid to be specifically hybridizable or hybridizable. Moreover, a polynucleotide may hybridize over one or more segments such that intervening or adjacent segments are not involved in the hybridization event (e.g., a loop structure or hairpin structure). A polynucleotide can comprise 60% or more, 65% or more, 70% or more, 75% or more, 80% or more, 85% or more, 90% or more, 95% or more, 98% or more, 99% or more, 99.5% or more, or 100% sequence complementarity to a target region within the target nucleic acid sequence to which it will hybridize. For example, an antisense nucleic acid in which 18 of 20 nucleotides of the antisense compound are complementary to a target region, and would therefore specifically hybridize, would represent 90 percent complementarity. In this example, the remaining noncomplementary nucleotides may be clustered or interspersed with complementary nucleotides and need not be contiguous to each other or to complementary nucleotides. Percent complementarity between particular stretches of nucleic acid sequences within nucleic acids can be determined using any convenient method. Exemplary methods include BLAST programs (basic local alignment search tools) and PowerBLAST programs (Altschul et al., J. Mol. Biol., 1990, 215, 403-410; Zhang and Madden, Genome Res., 1997, 7, 649-656) or by using the Gap program (Wisconsin Sequence Analysis Package, Version 8 for Unix, Genetics Computer Group, University Research Park, Madison Wis.), using default settings, which uses the algorithm of Smith and Waterman (Adv. Appl. Math., 1981, 2, 482-489).

The terms "peptide," "polypeptide," and "protein" are used interchangeably herein, and refer to a polymeric form of amino acids of any length, which can include coded and non-coded amino acids, chemically or biochemically modified or derivatized amino acids, and polypeptides having modified peptide backbones.

"Binding" as used herein (e.g. with reference to an RNA-binding domain of a polypeptide, binding to a target nucleic acid, and the like) refers to a non-covalent interaction between macromolecules (e.g., between a protein and a nucleic acid; between a subject Cas9/guide nucleic acid complex and a target nucleic acid; and the like). While in a state of non-covalent interaction, the macromolecules are said to be "associated" or "interacting" or "binding" (e.g., when a molecule X is said to interact with a molecule Y, it is meant the molecule X binds to molecule Y in a non-covalent manner). Not all components of a binding interaction need be sequence-specific (e.g., contacts with phosphate residues in a DNA backbone), but some portions of a binding interaction may be sequence-specific. Binding interactions are generally characterized by a dissociation constant (K_{d}) of less than 10⁻⁶ M, less than 10⁻⁷ M, less than 10⁻⁸ M, less than 10⁻⁹ M, less than 10⁻¹⁰ M, less than 10⁻¹¹ M, less than 10⁻¹² M, less than 10⁻¹³ M, less than 10⁻¹⁴ M, or less than 10⁻¹⁵ M. "Affinity" refers to the strength of binding, increased binding affinity being correlated with a lower K_{d}.

By "binding domain" it is meant a protein domain that is able to bind non-covalently to another molecule. A binding domain can bind to, for example, a DNA molecule (a DNA-binding domain), an RNA molecule (an RNA-binding domain) and/or a protein molecule (a protein-binding domain). In the case of a protein having a protein-binding domain, it can in some cases bind to itself (to form homodimers, homotrimers, etc.) and/or it can bind to one or more regions of a different protein or proteins.

The term "conservative amino acid substitution" refers to the interchangeability in proteins of amino acid residues having similar side chains. For example, a group of amino acids having aliphatic side chains consists of glycine, alanine, valine, leucine, and isoleucine; a group of amino acids having aliphatic-hydroxyl side chains consists of serine and threonine; a group of amino acids having amide containing side chains consisting of asparagine and glutamine; a group of amino acids having aromatic side chains consists of phenylalanine, tyrosine, and tryptophan; a group of amino acids having basic side chains consists of lysine, arginine, and histidine; a group of amino acids having acidic side chains consists of glutamate and aspartate; and a group of amino acids having sulfur containing side chains consists of cysteine and methionine. Exemplary conservative amino acid substitution groups are: valine-leucine-isoleucine, phenylalanine-tyrosine, lysine-arginine, alanine-valine-glycine, and asparagine-glutamine.

A polynucleotide or polypeptide has a certain percent "sequence identity" to another polynucleotide or polypeptide, meaning that, when aligned, that percentage of bases or amino acids are the same, and in the same relative position, when comparing the two sequences. Sequence identity can be determined in a number of different ways. To determine sequence identity, sequences can be aligned using various methods and computer programs (e.g., BLAST, T-COFFEE, MUSCLE, MAFFT, etc.), available over the world wide web at sites including ncbi.nlm.nili.gov/BLAST, ebi.ac.uk/Tools/msa/tcoffee/, ebi.ac.uk/Tools/msa/muscle/, mafft.cbrc.jp/alignment/software/. See, e.g., Altschul et al. (1990), J. Mol. Bioi. 215:403-10.

A DNA sequence that "encodes" a particular RNA is a DNA nucleic acid sequence that is transcribed into RNA. A DNA polynucleotide may encode an RNA (mRNA) that is translated into protein, or a DNA polynucleotide may encode an RNA that is not translated into protein (e.g. tRNA, rRNA, microRNA (miRNA), a "non-coding" RNA (ncRNA), a guide nucleic acid, etc.).

A "protein coding sequence" or a sequence that encodes a particular protein or polypeptide, is a nucleic acid sequence that is transcribed into mRNA (in the case of DNA) and is translated (in the case of mRNA) into a polypeptide in vitro or in vivo when placed under the control of appropriate regulatory sequences. The boundaries of the coding sequence are determined by a start codon at the 5' terminus (N-terminus) and a translation stop nonsense codon at the 3' terminus (C-terminus). A coding sequence can include, but is not limited to, cDNA from prokaryotic or eukaryotic mRNA, genomic DNA sequences from prokaryotic or eukaryotic DNA, and synthetic nucleic acids. A transcription termination sequence will usually be located 3' to the coding sequence.

The term "naturally-occurring" or "unmodified" or "wild type" as used herein as applied to a nucleic acid, a polypeptide, a cell, or an organism, refers to a nucleic acid, polypeptide, cell, or organism that is found in nature. For example, a polypeptide or polynucleotide sequence that is present in an organism (including viruses) that can be isolated from a source in nature and which has not been intentionally modified by a human in the laboratory is wild type (and naturally occurring).

"Heterologous," as used herein, means a nucleotide or polypeptide sequence that is not found in the native nucleic acid or protein, respectively. For example, in a chimeric Cas9 protein, the RNA-binding domain of a naturally-occurring bacterial Cas9 polypeptide (or a variant thereof) may be fused to a heterologous polypeptide sequence (i.e. a polypeptide sequence from a protein other than Cas9 or a polypeptide sequence from another organism). The heterologous polypeptide sequence may exhibit an activity (e.g., enzymatic activity) that will also be exhibited by the chimeric Cas9 protein (e.g., methyltransferase activity, acetyltransferase activity, kinase activity, ubiquitinating activity, etc.). A heterologous nucleic acid sequence may be linked to a naturally-occurring nucleic acid sequence (or a variant thereof) (e.g., by genetic engineering) to generate a chimeric nucleotide sequence encoding a chimeric polypeptide. As another example, in a fusion variant Cas9 polypeptide, a variant Cas9 polypeptide may be fused to a heterologous polypeptide (i.e. a polypeptide other than Cas9), which exhibits an activity that will also be exhibited by the fusion variant Cas9 polypeptide. A heterologous nucleic acid sequence may be linked to a variant Cas9 polypeptide (e.g., by genetic engineering) to generate a nucleotide sequence encoding a fusion variant polypeptide.

"Recombinant," as used herein, means that a particular nucleic acid (DNA or RNA) is the product of various combinations of cloning, restriction, polymerase chain reaction (PCR) and/or ligation steps resulting in a construct having a structural coding or non-coding sequence distinguishable from endogenous nucleic acids found in natural systems. DNA sequences encoding polypeptides can be assembled from cDNA fragments or from a series of synthetic oligonucleotides, to provide a synthetic nucleic acid which is capable of being expressed from a recombinant transcriptional unit contained in a cell or in a cell-free transcription and translation system. Genomic DNA comprising the relevant sequences can also be used in the formation of a recombinant gene or transcriptional unit. Sequences of non-translated DNA may be present 5' or 3' from the open reading frame, where such sequences do not interfere with manipulation or expression of the coding regions, and may indeed act to modulate production of a desired product by various mechanisms (see "DNA regulatory sequences", below). Alternatively, DNA sequences encoding RNA (e.g., guide nucleic acid) that is not translated may also be considered recombinant. Thus, e.g., the term "recombinant" nucleic acid refers to one which is not naturally occurring, e.g., is made by the artificial combination of two otherwise separated segments of sequence through human intervention. This artificial combination is often accomplished by either chemical synthesis means, or by the artificial manipulation of isolated segments of nucleic acids, e.g., by genetic engineering techniques. Such is usually done to replace a codon with a codon encoding the same amino acid, a conservative amino acid, or a non-conservative amino acid. Alternatively, it is performed to join together nucleic acid segments of desired functions to generate a desired combination of functions. This artificial combination is often accomplished by either chemical synthesis means, or by the artificial manipulation of isolated segments of nucleic acids, e.g., by genetic engineering techniques. When a recombinant polynucleotide encodes a polypeptide, the sequence of the encoded polypeptide can be naturally occurring ("wild type") or can be a variant (e.g., a mutant) of the naturally occurring sequence. Thus, the term "recombinant" polypeptide does not necessarily refer to a polypeptide whose sequence does not naturally occur. Instead, a "recombinant" polypeptide is encoded by a recombinant DNA sequence, but the sequence of the polypeptide can be naturally occurring ("wild type") or non-naturally occurring (e.g., a variant, a mutant, etc.). Thus, a "recombinant" polypeptide is the result of human intervention, but may be a naturally occurring amino acid sequence.

A cell has been "genetically modified" or "transformed" or "transfected" by exogenous DNA, e.g. a recombinant expression vector, when such DNA has been introduced inside the cell. The presence of the exogenous DNA results in permanent or transient genetic change. The transforming DNA may or may not be integrated (covalently linked) into the genome of the cell. In prokaryotes, yeast, and mammalian cells for example, the transforming DNA may be maintained on an episomal element such as a plasmid. With respect to eukaryotic cells, a stably transformed cell is one in which the transforming DNA has become integrated into a chromosome so that it is inherited by daughter cells through chromosome replication. This stability is demonstrated by the ability of the eukaryotic cell to establish cell lines or clones that comprise a population of daughter cells containing the transforming DNA. A "clone" is a population of cells derived from a single cell or common ancestor by mitosis. A "cell line" is a clone of a primary cell that is capable of stable growth in vitro for many generations.

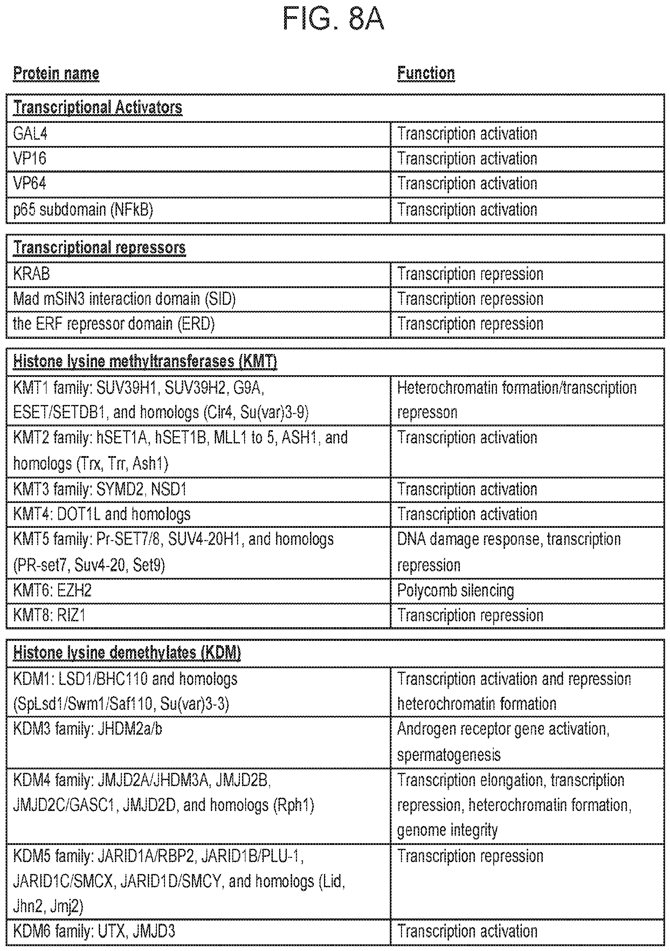

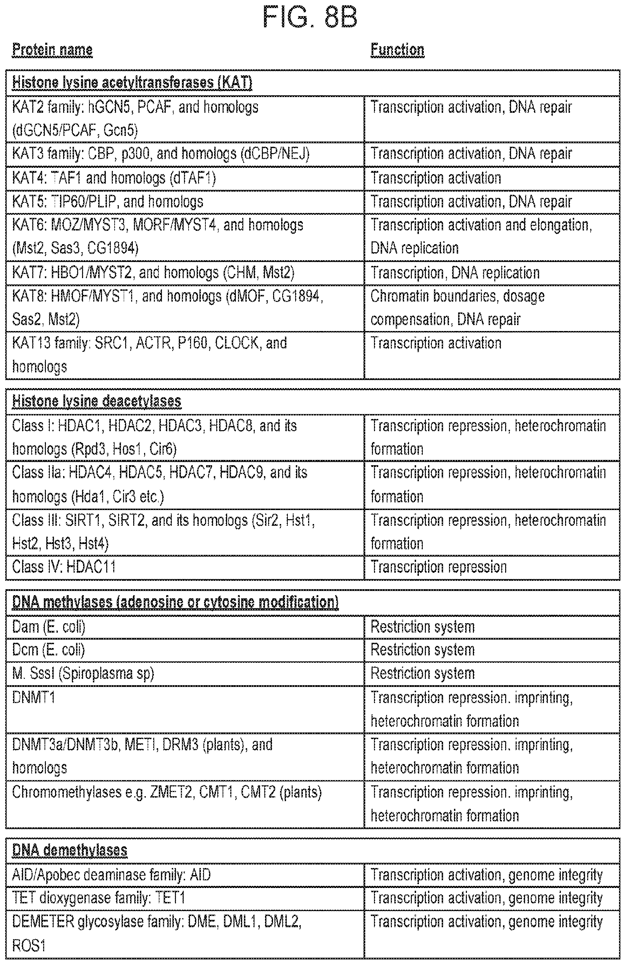

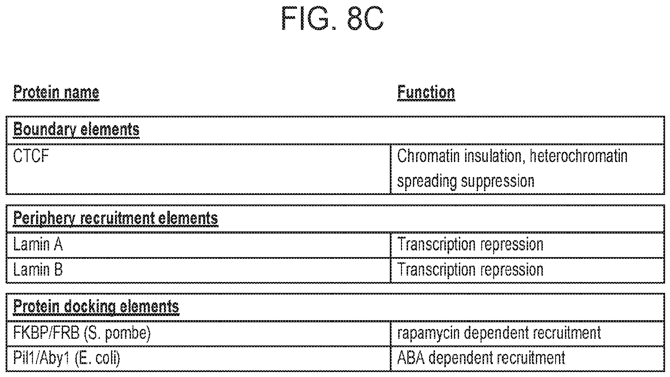

A "target nucleic acid" as used herein is a polynucleotide (e.g., RNA, DNA) that includes a "target site" or "target sequence." The terms "target site" or "target sequence" are used interchangeably herein to refer to a nucleic acid sequence present in a target nucleic acid to which a targeting segment of a subject guide nucleic acid will bind (see FIG. 8), provided sufficient conditions for binding exist. For example, the target site (or target sequence) 5'-GAGCAUAUC-3' within a target nucleic acid is targeted by (or is bound by, or hybridizes with, or is complementary to) the sequence 5'-GAUAUGCUC-3'. Suitable hybridization conditions include physiological conditions normally present in a cell. For a double stranded target nucleic acid, the strand of the target nucleic acid that is complementary to and hybridizes with the guide nucleic acid is referred to as the "complementary strand"; while the strand of the target nucleic acid that is complementary to the "complementary strand" (and is therefore not complementary to the guide nucleic acid) is referred to as the "noncomplementary strand" or "non-complementary strand". In cases where the target nucleic acid is a single stranded target nucleic acid (e.g., single stranded DNA (ssDNA), single stranded RNA (ssRNA)), the guide nucleic acid is complementary to and hybridizes with single stranded target nucleic acid.

By "Cas9 polypeptide" or "site-directed polypeptide" or "site-directed Cas9 polypeptide" it is meant a polypeptide that binds RNA (e.g., the protein binding segment of a guide nucleic acid) and is targeted to a specific sequence (a target site) in a target nucleic acid. A Cas9 polypeptide as described herein is targeted to a target site by the guide nucleic acid to which it is bound. The guide nucleic acid comprises a sequence that is complementary to a target sequence within the target nucleic acid, thus targeting the bound Cas9 polypeptide to a specific location within the target nucleic acid (the target sequence) (e.g., stabilizing the interaction of Cas9 with the target nucleic acid). In some cases, the Cas9 polypeptide is a naturally-occurring polypeptide (e.g, naturally occurs in bacterial and/or archaeal cells). In other cases, the Cas9 polypeptide is not a naturally-occurring polypeptide (e.g., the Cas9 polypeptide is a variant Cas9 polypeptide, a chimeric polypeptide as discussed below, and the like).Exemplary Cas9 polypeptides are set forth in SEQ ID NOs: 5-826 as a non-limiting and non-exhaustive list. Naturally occurring Cas9 polypeptides bind a guide nucleic acid, are thereby directed to a specific sequence within a target nucleic acid (a target site), and cleave the target nucleic acid (e.g., cleave dsDNA to generate a double strand break, cleave ssDNA, cleave ssRNA, etc.). A subject Cas9 polypeptide comprises two portions, an RNA-binding portion and an activity portion. An RNA-binding portion interacts with a subject guide nucleic acid. An activity portion exhibits site-directed enzymatic activity (e.g., nuclease activity, activity for DNA and/or RNA methylation, activity for DNA and/or RNA cleavage, activity for histone acetylation, activity for histone methylation, activity for RNA modification, activity for RNA-binding, activity for RNA splicing etc.). In some cases, the activity portion exhibits reduced nuclease activity relative to the corresponding portion of a wild type Cas9 polypeptide. In some cases, the activity portion is enzymatically inactive.

By "cleavage" it is meant the breakage of the covalent backbone of a target nucleic acid molecule (e.g., RNA, DNA). Cleavage can be initiated by a variety of methods including, but not limited to, enzymatic or chemical hydrolysis of a phosphodiester bond. Both single-stranded cleavage and double-stranded cleavage are possible, and double-stranded cleavage can occur as a result of two distinct single-stranded cleavage events. In certain embodiments, a complex comprising a guide nucleic acid and a Cas9 polypeptide is used for targeted cleavage of a single stranded target nucleic acid (e.g., ssRNA, ssDNA).

"Nuclease" and "endonuclease" are used interchangeably herein to mean an enzyme which possesses catalytic activity for nucleic acid cleavage (e.g., ribonuclease activity (ribonucleic acid cleavage), deoxyribonuclease activity (deoxyribonucleic acid cleavage), etc.).

By "cleavage domain" or "active domain" or "nuclease domain" of a nuclease it is meant the polypeptide sequence or domain within the nuclease which possesses the catalytic activity for nucleic acid cleavage. A cleavage domain can be contained in a single polypeptide chain or cleavage activity can result from the association of two (or more) polypeptides. A single nuclease domain may consist of more than one isolated stretch of amino acids within a given polypeptide.

A nucleic acid molecule that binds to the Cas9 polypeptide and targets the polypeptide to a specific location within the target nucleic acid is referred to herein as a "guide nucleic acid". When the guide nucleic acid is an RNA molecule, it can be referred to as a "guide RNA" or a "gRNA". A guide nucleic acid comprises two segments, a first segment (referred to herein as a "targeting segment"); and a second segment (referred to herein as a "protein-binding segment"). By "segment" it is meant a segment/section/region of a molecule, e.g., a contiguous stretch of nucleotides in a nucleic acid molecule. A segment can also mean a region/section of a complex such that a segment may comprise regions of more than one molecule. For example, in some cases the protein-binding segment (described below) of a guide nucleic acid is one nucleic acid molecule (e.g., one RNA molecule) and the protein-binding segment therefore comprises a region of that one molecule. In other cases, the protein-binding segment (described below) of a guide nucleic acid comprises two separate molecules that are hybridized along a region of complementarity. As an illustrative, non-limiting example, a protein-binding segment of a guide nucleic acid that comprises two separate molecules can comprise (i) base pairs 40-75 of a first molecule (e.g., RNA molecule, DNA/RNA hybrid molecule) that is 100 base pairs in length; and (ii) base pairs 10-25 of a second molecule (e.g., RNA molecule) that is 50 base pairs in length. The definition of "segment," unless otherwise specifically defined in a particular context, is not limited to a specific number of total base pairs, is not limited to any particular number of base pairs from a given nucleic acid molecule, is not limited to a particular number of separate molecules within a complex, and may include regions of nucleic acid molecules that are of any total length and may or may not include regions with complementarity to other molecules.

The first segment (targeting segment) of a guide nucleic acid (e.g., guide RNA) comprises a nucleotide sequence that is complementary to a specific sequence (a target site) within a target nucleic acid (e.g., a target ssRNA, a target ssDNA, the complementary strand of a double stranded target DNA, etc.). The protein-binding segment (or "protein-binding sequence") interacts with a Cas9 polypeptide. Site-specific binding and/or cleavage of the target nucleic acid can occur at locations determined by base-pairing complementarity between the guide nucleic acid (e.g., guide RNA) and the target nucleic acid.

The protein-binding segment of a subject guide nucleic acid comprises two complementary stretches of nucleotides that hybridize to one another to form a double stranded RNA duplex (dsRNA duplex).

In some embodiments, a subject nucleic acid (e.g., a guide nucleic acid, a nucleic acid comprising a nucleotide sequence encoding a guide nucleic acid; a nucleic acid encoding a Cas9 polypeptide; etc.) comprises a modification or sequence (e.g., an additional segment at the 5' and/or 3' end) that provides for an additional desirable feature (e.g., modified or regulated stability; subcellular targeting; tracking, e.g., a fluorescent label; a binding site for a protein or protein complex; etc.). Non-limiting examples include: a 5' cap (e.g., a 7-methylguanylate cap (m7G)); a 3' polyadenylated tail (i.e., a 3' poly(A) tail); a ribozyme sequence (e.g. to allow for self-cleavage and release of a mature molecule in a regulated fashion); a riboswitch sequence (e.g., to allow for regulated stability and/or regulated accessibility by proteins and/or protein complexes); a stability control sequence; a sequence that forms a dsRNA duplex (i.e., a hairpin)); a modification or sequence that targets the nucleic acid to a subcellular location (e.g., nucleus, mitochondria, chloroplasts, and the like); a modification or sequence that provides for tracking (e.g., direct conjugation to a fluorescent molecule, conjugation to a moiety that facilitates fluorescent detection, a sequence that allows for fluorescent detection, etc.); a modification or sequence that provides a binding site for proteins (e.g., proteins that act on DNA and/or RNA, including transcriptional activators, transcriptional repressors, DNA methyltransferases, DNA demethylases, histone acetyltransferases, histone deacetylases, and the like); and combinations thereof.

A subject guide nucleic acid (e.g., guide RNA) and a subject Cas9 polypeptide form a complex (i.e., bind via non-covalent interactions). The guide nucleic acid (e.g., guide RNA) provides target specificity to the complex by comprising a nucleotide sequence that is complementary to a sequence of a target nucleic acid. The Cas9 polypeptide of the complex provides the site-specific activity. In other words, the Cas9 polypeptide is guided to a target nucleic acid sequence (e.g. a target sequence in a chromosomal nucleic acid; a target sequence in an extrachromosomal nucleic acid, e.g. an episomal nucleic acid, a minicircle, an RNA, a DNA, etc.; a target sequence in a mitochondrial nucleic acid; a target sequence in a chloroplast nucleic acid; a target sequence in a plasmid; etc.) by virtue of its association with the protein-binding segment of the guide nucleic acid.

In some embodiments, a subject guide nucleic acid (e.g., guide RNA) comprises two separate nucleic acid molecules: an "activator" and a "targeter" (see below) and is referred to herein as a "dual guide nucleic acid", a "double-molecule guide nucleic acid", or a "two-molecule guide nucleic acid." If both molecules of a dual guide nucleic acid are RNA molecules, the dual guide nucleic acid can be referred to as a "dual guide RNA" or a "dgRNA." In some embodiments, the subject guide nucleic acid is a single nucleic acid molecule (single polynucleotide) and is referred to herein as a "single guide nucleic acid", a "single-molecule guide nucleic acid," or a "one-molecule guide nucleic acid." If a single guide nucleic acid is an RNA molecule, it can be referred to as a "single guide RNA" or an "sgRNA." The term "guide nucleic acid" is inclusive, referring to both dual guide nucleic acids and to single guide nucleic acids (e.g., dgRNAs, sgRNAs, etc.) while the term "guide RNA" is also inclusive, referring to both dual guide RNA (dgRNA) and single guide RNA (sgRNA).

In some cases, a guide nucleic acid is a DNA/RNA hybrid molecule. In such cases, the protein-binding segment of the guide nucleic acid is RNA and forms an RNA duplex. However, the targeting segment of a guide nucleic acid can be DNA. Thus, if a DNA/RNA hybrid guide nucleic acid is a dual guide nucleic acid, the "targeter" molecule and be a hybrid molecule (e.g, the targeting segment can be DNA and the duplex-forming segment can be RNA). In such cases, the duplex-forming segment of the "activator" molecule can be RNA (e.g., in order to form an RNA-duplex with the duplex-forming segment of the targeter molecule), while nucleotides of the "activator" molecule that are outside of the duplex-forming segment can be DNA (in which case the activator molecule is a hybrid DNA/RNA molecule) or can be RNA (in which case the activator molecule is RNA). If a DNA/RNA hybrid guide nucleic acid is a single guide nucleic acid, then the targeting segment can be DNA, the duplex-forming segments (which make up the protein-binding segment) can be RNA, and nucleotides outside of the targeting and duplex-forming segments can be RNA or DNA.

An exemplary dual guide nucleic acid comprises a crRNA-like ("CRISPR RNA" or "targeter" or "crRNA" or "crRNA repeat") molecule and a corresponding tracrRNA-like ("trans-acting CRISPR RNA" or "activator" or "tracrRNA") molecule. A crRNA-like molecule (targeter) comprises both the targeting segment (single stranded) of the guide nucleic acid and a stretch ("duplex-forming segment") of nucleotides that forms one half of the dsRNA duplex of the protein-binding segment of the guide nucleic acid. A corresponding tracrRNA-like molecule (activator) comprises a stretch of nucleotides (duplex-forming segment) that forms the other half of the dsRNA duplex of the protein-binding segment of the guide nucleic acid. In other words, a stretch of nucleotides of a crRNA-like molecule are complementary to and hybridize with a stretch of nucleotides of a tracrRNA-like molecule to form the dsRNA duplex of the protein-binding domain of the guide nucleic acid. As such, each crRNA-like molecule can be said to have a corresponding tracrRNA-like molecule. The crRNA-like molecule additionally provides the single stranded targeting segment. Thus, a crRNA-like and a tracrRNA-like molecule (as a corresponding pair) hybridize to form a dual guide nucleic acid. The exact sequence of a given crRNA or tracrRNA molecule is characteristic of the species in which the RNA molecules are found. A dual guide nucleic acid can include any corresponding activator and targeter pair.

The term "activator" is used herein to refer to a tracrRNA-like molecule of a dual guide nucleic acid (and of a single guide nucleic acid when the "activator" and the "targeter" are linked together by intervening nucleic acids). The term "targeter" is used herein to refer to a crRNA-like molecule of a dual guide nucleic acid (and of a single guide nucleic acid when the "activator" and the "targeter" are linked together by intervening nucleic acids). The term "duplex-forming segment" is used herein to mean the stretch of nucleotides of an activator or a targeter that contributes to the formation of the dsRNA duplex by hybridizing to a stretch of nucleotides of a corresponding activator or targeter molecule. In other words, an activator comprises a duplex-forming segment that is complementary to the duplex-forming segment of the corresponding targeter. As such, an activator comprises a duplex-forming segment while a targeter comprises both a duplex-forming segment and the targeting segment of the guide nucleic acid. A subject single guide nucleic acid can comprise an "activator" and a "targeter" where the "activator" and the "targeter" are covalently linked (e.g., by intervening nucleotides). Therefore, a dual guide nucleic acid can be comprised of any corresponding activator and targeter pair.

A "host cell" or "target cell" as used herein, denotes an in vivo or in vitro eukaryotic cell, or a cell from a multicellular organism (e.g., a cell line) cultured as a unicellular entity, which eukaryotic cells can be, or have been, used as recipients for a nucleic acid, and include the progeny of the original cell which has been transformed by the nucleic acid. It is understood that the progeny of a single cell may not necessarily be completely identical in morphology or in genomic or total DNA complement as the original parent, due to natural, accidental, or deliberate mutation. A "recombinant host cell" (also referred to as a "genetically modified host cell") is a host cell into which has been introduced a heterologous nucleic acid, e.g., an expression vector. For example, a subject eukaryotic host cell is a genetically modified eukaryotic host cell, by virtue of introduction into a suitable eukaryotic host cell of an exogenous nucleic acid.

The term "stem cell" is used herein to refer to a cell (e.g., plant stem cell, vertebrate stem cell) that has the ability both to self-renew and to generate a differentiated cell type (see Morrison et al. (1997) Cell 88:287-298). In the context of cell ontogeny, the adjective "differentiated", or "differentiating" is a relative term. A "differentiated cell" is a cell that has progressed further down the developmental pathway than the cell it is being compared with. Thus, pluripotent stem cells (described below) can differentiate into lineage-restricted progenitor cells (e.g., mesodermal stem cells), which in turn can differentiate into cells that are further restricted (e.g., neuron progenitors), which can differentiate into end-stage cells (i.e., terminally differentiated cells, e.g., neurons, cardiomyocytes, etc.), which play a characteristic role in a certain tissue type, and may or may not retain the capacity to proliferate further. Stem cells may be characterized by both the presence of specific markers (e.g., proteins, RNAs, etc.) and the absence of specific markers. Stem cells may also be identified by functional assays both in vitro and in vivo, particularly assays relating to the ability of stem cells to give rise to multiple differentiated progeny.

Stem cells of interest include pluripotent stem cells (PSCs). The term "pluripotent stem cell" or "PSC" is used herein to mean a stem cell capable of producing all cell types of the organism. Therefore, a PSC can give rise to cells of all germ layers of the organism (e.g., the endoderm, mesoderm, and ectoderm of a vertebrate). Pluripotent cells are capable of forming teratomas and of contributing to ectoderm, mesoderm, or endoderm tissues in a living organism. Pluripotent stem cells of plants are capable of giving rise to all cell types of the plant (e.g., cells of the root, stem, leaves, etc.).

PSCs of animals can be derived in a number of different ways. For example, embryonic stem cells (ESCs) can be derived from the inner cell mass of an embryo (Thomson et. al, Science. 1998 Nov 6;282(5391):1145-7), cited by reference only, whereas induced pluripotent stem cells (iPSCs) are derived from somatic cells (Takahashi et. al, Cell. 2007 Nov 30;131(5):861-72; Takahashi et. al, Nat Protoc. 2007;2(12):3081-9; Yu et. al, Science. 2007 Dec 21;318(5858):1917-20. Epub 2007 Nov 20). Because the term PSC refers to pluripotent stem cells regardless of their derivation, the term PSC encompasses the terms ESC and iPSC, as well as the term embryonic germ stem cells (EGSC), which are another example of a PSC. PSCs may be in the form of an established cell line, they may be obtained directly from primary embryonic tissue, or they may be derived from a somatic cell. PSCs can be target cells of the methods described herein.

By "embryonic stem cell" (ESC) is meant a PSC that can be isolated from an embryo, typically from the inner cell mass of the blastocyst. ESC lines are listed in the NIH Human Embryonic Stem Cell Registry, e.g. hESBGN-01, hESBGN-02, hESBGN-03, hESBGN-04 (BresaGen, Inc.); HES-1, HES-2, HES-3, HES-4, HES-5, HES-6 (ES Cell International); Miz-hES1 (MizMedi Hospital-Seoul National University); HSF-1, HSF-6 (University of California at San Francisco); and H1, H7, H9, H13, H14 (Wisconsin Alumni Research Foundation (WiCell Research Institute)), all are mentioned for reference only. Stem cells of interest also include embryonic stem cells from other primates, such as Rhesus stem cells and marmoset stem cells. The stem cells may be obtained from any mammalian species, e.g. human, equine, bovine, porcine, canine, feline, rodent, e.g. mice, rats, hamster, primate, etc. (Thomson et al. (1998) Science 282:1145; Thomson et al. (1995) Proc. Natl. Acad. Sci USA 92:7844; Thomson et al. (1996) Biol. Reprod. 55:254; Shamblott et al., Proc. Natl. Acad. Sci. USA 95:13726, 1998), cited for reference only. In culture, ESCs typically grow as flat colonies with large nucleo-cytoplasmic ratios, defined borders and prominent nucleoli. In addition, ESCs express SSEA-3, SSEA-4, TRA-1-60, TRA-1-81, and Alkaline Phosphatase, but not SSEA-1. Examples of methods of generating and characterizing ESCs may be found in, for example, US Patent No. 7,029,913, US Patent No. 5,843,780, and US Patent No. 6,200,806. Methods for proliferating hESCs in the undifferentiated form are described in WO 99/20741, WO 01/51616, and WO 03/020920.

By "embryonic germ stem cell" (EGSC) or "embryonic germ cell" or "EG cell" is meant a PSC that is derived from germ cells and/or germ cell progenitors, e.g. primordial germ cells, i.e. those that would become sperm and eggs. Embryonic germ cells (EG cells) are thought to have properties similar to embryonic stem cells as described above. Examples of methods of generating and characterizing EG cells may be found in, for example, US Patent No. 7,153,684; Matsui, Y., et al., (1992) Cell 70:841; Shamblott, M., et al. (2001) Proc. Natl. Acad. Sci. USA 98: 113; Shamblott, M., et al. (1998) Proc. Natl. Acad. Sci. USA, 95:13726; and Koshimizu, U., et al. (1996) Development, 122:1235.

By "induced pluripotent stem cell" or "iPSC" it is meant a PSC that is derived from a cell that is not a PSC (i.e., from a cell this is differentiated relative to a PSC). iPSCs can be derived from multiple different cell types, including terminally differentiated cells. iPSCs have an ES cell-like morphology, growing as flat colonies with large nucleo-cytoplasmic ratios, defined borders and prominent nuclei. In addition, iPSCs express one or more key pluripotency markers known by one of ordinary skill in the art, including but not limited to Alkaline Phosphatase, SSEA3, SSEA4, Sox2, Oct3/4, Nanog, TRA160, TRA181, TDGF 1, Dnmt3b, FoxD3, GDF3, Cyp26a1, TERT, and zfp42. Examples of methods of generating and characterizing iPSCs may be found in, for example, U.S. Patent Publication Nos. US20090047263, US20090068742, US20090191159, US20090227032, US20090246875, and US20090304646. Generally, to generate iPSCs, somatic cells are provided with reprogramming factors (e.g. Oct4, SOX2, KLF4, MYC, Nanog, Lin28, etc.) known in the art to reprogram the somatic cells to become pluripotent stem cells.

By "somatic cell" it is meant any cell in an organism that, in the absence of experimental manipulation, does not ordinarily give rise to all types of cells in an organism. In other words, somatic cells are cells that have differentiated sufficiently that they will not naturally generate cells of all three germ layers of the body, i.e. ectoderm, mesoderm and endoderm. For example, somatic cells would include both neurons and neural progenitors, the latter of which may be able to naturally give rise to all or some cell types of the central nervous system but cannot give rise to cells of the mesoderm or endoderm lineages.

By "mitotic cell" it is meant a cell undergoing mitosis. Mitosis is the process by which a eukaryotic cell separates the chromosomes in its nucleus into two identical sets in two separate nuclei. It is generally followed immediately by cytokinesis, which divides the nuclei, cytoplasm, organelles and cell membrane into two cells containing roughly equal shares of these cellular components.

By "post-mitotic cell" it is meant a cell that has exited from mitosis, i.e., it is "quiescent", i.e. it is no longer undergoing divisions. This quiescent state may be temporary, i.e. reversible, or it may be permanent.

By "meiotic cell" it is meant a cell that is undergoing meiosis. Meiosis is the process by which a cell divides its nuclear material for the purpose of producing gametes or spores. Unlike mitosis, in meiosis, the chromosomes undergo a recombination step which shuffles genetic material between chromosomes. Additionally, the outcome of meiosis is four (genetically unique) haploid cells, as compared with the two (genetically identical) diploid cells produced from mitosis.

The terms "treatment", "treating" and the like are used herein to generally mean obtaining a desired pharmacologic and/or physiologic effect. The effect may be prophylactic in terms of completely or partially preventing a disease or symptom thereof and/or may be therapeutic in terms of a partial or complete cure for a disease and/or adverse effect attributable to the disease. "Treatment" as used herein covers any treatment of a disease or symptom in a mammal, and includes: (a) preventing the disease or symptom from occurring in a subject which may be predisposed to acquiring the disease or symptom but has not yet been diagnosed as having it; (b) inhibiting the disease or symptom, i.e., arresting its development; or (c) relieving the disease, i.e., causing regression of the disease. The therapeutic agent may be administered before, during or after the onset of disease or injury. The treatment of ongoing disease, where the treatment stabilizes or reduces the undesirable clinical symptoms of the patient, is of particular interest. Such treatment is desirably performed prior to complete loss of function in the affected tissues. The subject therapy will desirably be administered during the symptomatic stage of the disease, and in some cases after the symptomatic stage of the disease.

The terms "individual," "subject," "host," and "patient," are used interchangeably herein and refer to any mammalian subject for whom diagnosis, treatment, or therapy is desired, particularly humans.

In some instances, a component (e.g., a nucleic acid component (e.g., a guide nucleic acid, etc.); a protein component (e.g., a Cas9 polypeptide, a variant Cas9 polypeptide); and the like) includes a label moiety. The terms "label", "detectable label", or "label moiety" as used herein refer to any moiety that provides for signal detection and may vary widely depending on the particular nature of the assay. Label moieties of interest include both directly detectable labels (direct labels)(e.g., a fluorescent label) and indirectly detectable labels (indirect labels)(e.g., a binding pair member). A fluorescent label can be any fluorescent label (e.g., a fluorescent dye (e.g., fluorescein, Texas red, rhodamine, ALEXAFLUOR® labels, and the like), a fluorescent protein (e.g., green fluorescent protein (GFP), enhanced GFP (EGFP), yellow fluorescent protein (YFP), red fluorescent protein (RFP), cyan fluorescent protein (CFP), cherry, tomato, tangerine, and any fluorescent derivative thereof), etc.). Suitable detectable (directly or indirectly) label moieties for use in the methods include any moiety that is detectable by spectroscopic, photochemical, biochemical, immunochemical, electrical, optical, chemical, or other means. For example, suitable indirect labels include biotin (a binding pair member), which can be bound by streptavidin (which can itself be directly or indirectly labeled). Labels can also include: a radiolabel (a direct label)(e.g., ³H, ¹²⁵I, ³⁵S, ¹⁴C, or ³²P); an enzyme (an indirect label)(e.g., peroxidase, alkaline phosphatase, galactosidase, luciferase, glucose oxidase, and the like); a fluorescent protein (a direct label)(e.g., green fluorescent protein, red fluorescent protein, yellow fluorescent protein, and any convenient derivatives thereof); a metal label (a direct label); a colorimetric label; a binding pair member; and the like. By "partner of a binding pair" or "binding pair member" is meant one of a first and a second moiety, wherein the first and the second moiety have a specific binding affinity for each other. Suitable binding pairs include, but are not limited to: antigen/antibodies (for example, digoxigenin/anti-digoxigenin, dinitrophenyl (DNP)/anti-DNP, dansyl-X-anti-dansyl, fluorescein/anti-fluorescein, lucifer yellow/anti-lucifer yellow, and rhodamine anti-rhodamine), biotin/avidin (or biotin/streptavidin) and calmodulin binding protein (CBP)/calmodulin. Any binding pair member can be suitable for use as an indirectly detectable label moiety.

Any given component, or combination of components can be unlabeled, or can be detectably labeled with a label moiety. In some cases, when two or more components are labeled, they can be labeled with label moieties that are distinguishable from one another.

General methods in molecular and cellular biochemistry can be found in such standard textbooks as Molecular Cloning: A Laboratory Manual, 3rd Ed. (Sambrook et al., HaRBor Laboratory Press 2001); Short Protocols in Molecular Biology, 4th Ed. (Ausubel et al. eds., John Wiley & Sons 1999); Protein Methods (Bollag et al., John Wiley & Sons 1996); Nonviral Vectors for Gene Therapy (Wagner et al. eds., Academic Press 1999); Viral Vectors (Kaplift & Loewy eds., Academic Press 1995); Immunology Methods Manual (I. Lefkovits ed., Academic Press 1997); and Cell and Tissue Culture: Laboratory Procedures in Biotechnology (Doyle & Griffiths, John Wiley & Sons 1998).

Before the present invention is further described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described. All publications mentioned herein disclose and describe the methods and/or materials in connection with which the publications are cited.

It must be noted that as used herein and in the appended claims, the singular forms "a," "an," and "the" include plural referents unless the context clearly dictates otherwise. Thus, for example, reference to "a complex" includes a plurality of such complexes and reference to "the Cas9 polypeptide" includes reference to one or more Cas9 polypeptides and equivalents thereof known to those skilled in the art, and so forth. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments pertaining to the invention are specifically embraced by the present invention and are disclosed herein just as if each and every combination was individually and explicitly disclosed. In addition, all sub-combinations of the various embodiments and elements thereof are also specifically embraced by the present invention and are disclosed herein just as if each and every such sub-combination was individually and explicitly disclosed herein.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### DETAILED DESCRIPTION

The present invention is defined by the appended claims. The present disclosure provides a complex comprising a nucleic acid-conjugated nanoparticle; a Type II or a Type V CRISPR system comprising a site-directed DNA-modifying polypeptide and a guide RNA; and a polycation-based endosomal escape polymer. The present disclosure provides methods of making and using a complex of the present disclosure.

### COMPLEX

The present disclosure provides a complex comprising: a) a nanoparticle-nucleic acid conjugate; a Type II or a Type V CRISPR system comprising a site-directed DNA-modifying polypeptide and a guide RNA, and optionally also comprising a donor polynucleotide (e.g., a DNA donor template); and b) a polycation-based endosomal escape polymer. A complex of the present disclosure is also referred to herein as "an encapsulated complex," "an encapsulated Type II or Type V CRISPR complex," "an encapsulated nanoparticle complex," or "an encapsulated Type II or Type V CRISPR nanoparticle complex."

The present disclosure provides a complex comprising: a) a nanoparticle-nucleic acid conjugate; a Type II CRISPR system comprising a Cas9 polypeptide and a guide RNA, and optionally also comprising a donor polynucleotide (e.g., a DNA donor template); and b) a polycation-based endosomal escape polymer. A complex of the present disclosure that comprises a Type II CRISPR system is also referred to herein as "an encapsulated Type II CRISPR complex," or "an encapsulated Type II CRISPR nanoparticle complex."

The present disclosure provides a complex comprising: a) a nanoparticle-nucleic acid conjugate; a Type V CRISPR system comprising a Cfp1 polypeptide and a guide RNA, and optionally also comprising a donor polynucleotide (e.g., a DNA donor template); and b) a polycation-based endosomal escape polymer. A complex of the present disclosure that comprises a Type V CRISPR system is also referred to herein as "an encapsulated Type V CRISPR complex," or "an encapsulated Type V CRISPR nanoparticle complex."

A complex of the present disclosure exhibits low toxicity toward a target cell. In some cases, less than 40%, less than 30%, less than 25%, less than 20%, less than 15%, less than 10%, less than 5%, or less than 2%, of the cells of a target cell population are killed following contact with a complex of the present disclosure. In some cases, at least 60%, at least 70%, at least 75%, at least 80%, at least 85%, at least 90%, at least 95%, at least 98%, or more than 98%, of the cells of a target cell population remain viable following contact with a complex of the present disclosure.

In some cases, a complex of the present disclosure has a zeta potential of from -20 mV to 20 mV, e.g., from -20 mV to -15 mV, from -15 mV to -10 mV, from -10 mV to -5 mV, from -5 mV to 0.5 mV, from 0.5 mV to 5 mV, from 5 mV to 10 mV, from 10 mV to 15 mV, or from 15 mV to 20 mV.

As noted above, in some cases, a complex of the present disclosure comprises a donor polynucleotide. For example, in some cases, a complex of the present disclosure comprises: a) a nanoparticle-nucleic acid conjugate; a Type II or a Type V CRISPR system comprising a site-directed DNA-modifying polypeptide and a guide RNA, and a donor polynucleotide (e.g., a DNA donor template); and b) a polycation-based endosomal escape polymer. In some cases, the amount of donor polynucleotide (donor DNA) in a complex of the present disclosure is from 0.1 µg to 10 µg, e.g., from 0.1 µg to 0.5 µg, from 0.5 µg to 1 µg, from 1 µg to 2 µg, from 2 µg to 4 µg, from 4 µg to 6 µg, from 6 µg to 8 µg, or from 8 µg to 10 µg. In some cases, the amount of RNA-guided endoribonuclease (e.g., Cas9, Cpfl, etc.) polypeptide in a complex of the present disclosure is from 1 µg to 20 µg, e.g., from 1 µg to 2 µg, from 2 µg to 4 µg, from 4 µg to 6 µg, from 6 µg to 8 µg, from 8 µg to 10 µg, from 10 µg to 12 µg, from 12 µg to 14 µg, from 14 µg to 16 µg, from 16 µg to 18 µg, or from 18 µg to 20 µg. In some cases, a complex of the present disclosure comprises 4 µg donor DNA and 8 µg Cas9.

### Nanoparticle-Nucleic Acid Conjugates

A complex of the present disclosure comprises a nanoparticle-nucleic acid conjugate. In some cases, a complex of the present disclosure comprises a colloidal metal nanoparticle that comprises a nucleic acid. Thus, aspects of the present disclosure include nanoparticles conjugated with a nucleic acid. In some cases, the nanoparticles comprise any suitable biocompatible polymer. In some cases, the nanoparticles comprise any suitable colloidal metal. A colloidal metal includes any water-insoluble metal particle or metallic compound dispersed in liquid water. A colloid metal can be a suspension of metal particles in aqueous solution. Any metal that can be made in colloidal form can be used, including gold, silver, copper, nickel, aluminum, zinc, calcium, platinum, palladium, and iron. In some cases, gold nanoparticles are used, e.g., prepared from HAuCl₄. In some cases, the nanoparticles are non-gold nanoparticles that are coated with gold to make gold-coated nanoparticles.

### Nanoparticles

Nanoparticles suitable for use in a complex of the present disclosure can be any shape and can range in size from 5 nm to 1000 nm in size, e.g., from 5 nm to 75 nm, 5 to 50 nm, 5 nm to 40 nm, 10 nm to 30, including 20 nm to 30 nm in size. Nanoparticles (e.g., gold nanoparticles) suitable for use in a complex of the present disclosure can have a size in the range of from 5 nm to 50 nm, e.g., from 5 nm to 10 nm, from 10 nm to 15 nm, from 15 nm to 20 nm, from 20 nm to 25 nm, from 25 nm to 30 nm, from 30 nm to 35 nm, from 35 nm to 40 nm, from 40 nm to 45 nm, or from 45 nm to 50 nm. Nanoparticles suitable for use in a complex of the present disclosure can have a size from 50 nm to 55 nm, from 55 nm to 60 nm, from 60 nm to 65 nm, from 65 nm to 70 nm, to 70 nm to 75 nm, from 75 nm to 80 nm, from 80 nm to 85 nm, from 85 nm to 90 nm, from 90 nm to 95 nm, from 95 nm to 100 nm, from 100 nm to 105 nm , from 105 nm to 110 nm, from 110 nm to 115 nm, from 115 nm to 120 nm, from 120 nm to 125 nm, from 125nm to 130 nm, from 130nm to 135 nm, from 135 nm to 140 nm, from 140 nm to 145 nm, from 145 nm to 150 nm. Nanoparticles suitable for use in a complex of the present disclosure can have a size of from 100 nm to 500 nm, e.g., from about 100 nm to 150 nm, from 150 nm to 200 nm, from 200 nm to 250 nm, from 250 nm to 300 nm, from 300 nm to 350 nm, from 350 nm to 400 nm, from 400 nm to 450 nm, or from 450 nm to 500 nm.

Nanoparticles suitable for use in a complex of the present disclosure can have a size of from 500 nm to 10 µm, e.g., from 500 nm to 750 nm, from 750 nm to 1 µm, from 1 µm to 2 µm, from 2 µm to 5 µm, from 5 µm to 7 µm, or from 7 µm to 10 µm. Nanoparticles suitable for use in a complex of the present disclosure can have a size of from 10 µm to 100 µm, e.g., from 10 µm to 20 µm, from 20 µm to 30 µm, from 30 µm to 40 µm, from 40 µm to 50 µm, from 50 µm to 60 µm, from 60 µm to 70 µm, from 70 µm to 80 µm, from 80 µm to 90 µm, or from 90 µm to 100 µm.

A nanoparticle can comprise any suitable material, e.g., a biocompatible material. The biocompatible material can be a polymer. Suitable nanoparticle polymers include polystyrene, silicone rubber, polycarbonate, polyurethanes, polypropylenes, polymethylmethacrylate, polyvinyl chloride, polyesters, polyethers, and polyethylene. Non-limiting examples of specific polymers include poly(caprolactone) (PCL), ethylene vinyl acetate polymer (EVA), poly(lactic acid) (PLA), poly(L-lactic acid) (PLLA), poly(glycolic acid) (PGA), poly(lactic acid-co-glycolic acid) (PLGA), poly(L-lactic acid-co-glycolic acid) (PLLGA), poly(D,L-lactide) (PDLA), poly(L-lactide) (PLLA), poly(D,L-lactide-co-caprolactone), poly(D,L-lactide-co-caprolactone-co-glycolide), poly(D,L-lactide-co-PEO-co-D,L-lactide), poly(D,L-lactide-co-PPO-co-D,L-lactide), polyalkyl cyanoacralate, polyurethane, poly-L-lysine (PLL), hydroxypropyl methacrylate (HPMA), polyethyleneglycol, poly-L-glutamic acid, poly(hydroxy acids), polyanhydrides, polyorthoesters, poly(ester amides), polyamides, poly(ester ethers), polycarbonates, polyalkylenes such as polyethylene and polypropylene, polyalkylene glycols such as poly(ethylene glycol) (PEG), polyethylenimine (PEI), polyalkylene oxides (PEO), polyalkylene terephthalates such as poly(ethylene terephthalate), polyvinyl alcohols (PVA), polyvinyl ethers, polyvinyl esters such as poly(vinyl acetate), polyvinyl halides such as poly(vinyl chloride) (PVC), polyvinylpyrrolidone, polysiloxanes, polystyrene (PS), polyurethanes, derivatized celluloses such as alkyl celluloses, hydroxyalkyl celluloses, cellulose ethers, cellulose esters, nitro celluloses, hydroxypropylcellulose, carboxymethylcellulose, polymers of acrylic acids, such as poly(methyl(meth)acrylate) (PMMA), poly(ethyl(meth)acrylate), poly(butyl(meth)acrylate), poly(isobutyl(meth)acrylate), poly(hexyl(meth)acrylate), poly(isodecyl(meth)acrylate), poly(lauryl(meth)acrylate), poly(phenyl(meth)acrylate), poly(methyl acrylate), poly(isopropyl acrylate), poly(isobutyl acrylate), poly(octadecyl acrylate) and copolymers and mixtures thereof, polydioxanone and its copolymers, polyhydroxyalkanoates, polypropylene fumarate, polyoxymethylene, poloxamers, poly(ortho)esters, poly(butyric acid), poly(valeric acid), poly(lactide-co-caprolactone), and trimethylene carbonate, polyvinylpyrrolidone.

In some cases, the nanoparticle is a lipid nanoparticle. A lipid nanoparticle can include one or more lipids, and one or more of the polymers listed above.

In some cases, the nanoparticle in a complex of the present disclosure is a colloidal metal nanoparticle. A colloidal metal includes any water-insoluble metal particle or metallic compound dispersed in liquid water. A colloid metal can be a suspension of metal particles in aqueous solution. Any metal that can be made in colloidal form can be used, including gold, silver, copper, nickel, aluminum, zinc, calcium, platinum, palladium, and iron. In some cases, gold nanoparticles are used, e.g., prepared from HAuCl₄. In some cases, the nanoparticles are non-gold nanoparticles that are coated with gold to make gold-coated nanoparticles.

In some cases, the nanoparticle is selected from the group consisting of a gold nanoparticle, a silver nanoparticle, a platinum nanoparticle, an aluminum nanoparticle, a palladium nanoparticle, a copper nanoparticle, a cobalt nanoparticle, an indium nanoparticle, and a nickel nanoparticle.

Methods for making colloidal metal nanoparticles, including gold colloidal nanoparticles from HAuCl₄, are known to those having ordinary skill in the art. For example, the methods described herein as well as those described elsewhere (e.g., US 2001/005581; 2003/0118657; and 2003/0053983) can be used to make nanoparticles.

Further aspects of the present disclosure include a nanoparticle, e.g., gold nanoparticle, conjugated to a nucleic acid. The nucleic acid can be conjugated covalently or noncovalently to the surface of the nanoparticle. For example, a nucleic acid may be covalently bonded at one end of the nucleic acid to the surface of the nanoparticle.

### Nucleic acid linked to a nanoparticle

The nucleic acid that is conjugated to the nanoparticle may be single stranded, double stranded, or may have mix of single stranded and double stranded regions.

A nucleic acid can be conjugated directly or indirectly to a nanoparticle surface. For example, a nucleic acid can be conjugated directly to the surface of a nanoparticle or indirectly through an intervening linker. Any type of molecule can be used as a linker. For example, a linker can be an aliphatic chain including at least two carbon atoms (e.g., 3, 4, 5, 6, 7, 8, 9, 10 or more carbon atoms), and can be substituted with one or more functional groups including ketone, ether, ester, amide, alcohol, amine, urea, thiourea, sulfoxide, sulfone, sulfonamide, and disulfide functionalities. In cases where the nanoparticle includes gold, a linker can be any thiol-containing molecule. Reaction of a thiol group with the gold results in a covalent sulfide (-S-) bond. Linker design and synthesis are well known in the art.

The nucleic acid conjugated to the nanoparticle can have a length of from 10 nucleotides (nt) to 1000 nt, e.g., from 1 nt to 25 nt, from 25 nt to 50 nt, from 50 nt to 100 nt, from 100 nt to 250 nt, from 250 nt to 500 nt, or from 500 nt to 1000 nt. The nucleic acid conjugated to the nanoparticle can have a length of greater than 1000 nt. In some cases, the nucleic acid conjugated to the nanoparticle does not encode any protein or any other gene product. Instead, in some cases, the nucleic acid conjugated to the nanoparticle serves to non-covalently bind the Type II or Type V CRISPR system (where the Type II CRISPR system comprises a Cas9 polypeptide and a guide RNA; or where the Type II CRISPR system comprises a Cas9 polypeptide, a guide RNA, and a donor polynucleotide; where the Type V CRISPR system comprises a Cpfl polypeptide and a guide RNA; or where the Type V CRISPR system comprises a Cpfl polypeptide, a guide RNA and a donor DNA template) to the nanoparticle-nucleic acid conjugate. In some cases, the nucleic acid conjugated to the nanoparticle comprises one or more protospacer adjacent motif (PAM) sequences, e.g., a GG sequence or any other PAM sequence known in the art.

The nucleic acid conjugated to the nanoparticle (e.g., a colloidal metal (e.g., gold) nanoparticle; a nanoparticle comprising a biocompatible polymer) can have a length of from 10 nucleotides (nt) to 1000 nt, e.g., from 1 nt to 25 nt, from 25 nt to 50 nt, from 50 nt to 100 nt, from 100 nt to 250 nt, from 250 nt to 500 nt, or from 500 nt to 1000 nt. The nucleic acid conjugated to the nanoparticle (e.g., a colloidal metal (e.g., gold) nanoparticle; a nanoparticle comprising a biocompatible polymer) nanoparticle can have a length of greater than 1000 nt. In some cases, the nucleic acid conjugated to the nanoparticle (e.g., a colloidal metal (e.g., gold) nanoparticle) does not encode any protein or any other gene product. Instead, the nucleic acid conjugated to the nanoparticle (e.g., a colloidal metal (e.g., gold) nanoparticle) serves to non-covalently bind the Type II CRISPR system or Type V CRISPR system (where the Type II CRISPR system comprises a Cas9 polypeptide and a guide RNA; or where the Type II CRISPR system comprises a Cas9 polypeptide, a guide RNA, and a donor polynucleotide; where the Type V CRISPR system comprises a Cpfl polypeptide and a guide RNA; or where the Type V CRISPR system comprises a Cpfl polypeptide, a guide RNA, and a donor polynucleotide) to the nanoparticle-nucleic acid conjugate. In some cases, the nucleic acid conjugated to the nanoparticle (e.g., a colloidal metal (e.g., gold) nanoparticle; a nanoparticle comprising a biocompatible polymer) comprises one or more protospacer adjacent motif (PAM) sequences, e.g., a GG sequence or any other PAM sequence known in the art.

In some cases, a nucleic acid linked (e.g., covalently linked; non-covalently linked) to a nanoparticle comprises a nucleotide sequence that hybridizes to at least a portion of the guide RNA present in a complex of the present disclosure. In some cases, a nucleic acid linked to a nanoparticle in a complex of the present disclosure has at least 50%, at least 60%, at least 70%, at least 80%, at least 90%, at least 95%, at least 98%, at least 99%, or 100%, nucleotide sequence identity to a complement of from 10 to 50 nucleotides (e.g., from 10 nucleotides (nt) to 15 nt, from 15 nt to 20 nt, from 20 nt to 25 nt, from 25 nt to 30 nt, from 30 nt to 40 nt, or from 40 nt to 50 nt) of a guide RNA present in the complex.

In some cases, a nucleic acid linked (e.g., covalently linked; non-covalently linked) to a nanoparticle is a donor DNA template, or has the same or substantially the same nucleotide sequence as a donor DNA template. The term "donor DNA template" is also referred to herein as "donor sequence" or "donor polynucleotide" or "donor nucleic acid."

In some cases, a nucleic acid linked (e.g., covalently linked; non-covalently linked) to a nanoparticle comprises a nucleotide sequence that is complementary to a donor DNA template.

By a "donor sequence" or "donor polynucleotide" it is meant a nucleic acid sequence to be inserted at the cleavage site induced by a site-directed modifying polypeptide (e.g., a Cas9 polypeptide; a Cpfl polypeptide). The donor polynucleotide will contain sufficient homology to a genomic sequence at the cleavage site, e.g. 70%, 80%, 85%, 90%, 95%, or 100% homology with the nucleotide sequences flanking the cleavage site, e.g. within about 50 bases or less of the cleavage site, e.g. within about 30 bases, within about 15 bases, within about 10 bases, within about 5 bases, or immediately flanking the cleavage site, to support homology-directed repair between it and the genomic sequence to which it bears homology. Approximately 25, 50, 100, or 200 nucleotides, or more than 200 nucleotides, of sequence homology between a donor and a genomic sequence (or any integral value between 10 and 200 nucleotides, or more) will support homology-directed repair. Donor sequences can be of any length, e.g. 10 nucleotides or more, 50 nucleotides or more, 100 nucleotides or more, 250 nucleotides or more, 500 nucleotides or more, 1000 nucleotides or more, 5000 nucleotides or more, etc.

The donor sequence is typically not identical to a target genomic sequence. Rather, the donor sequence may contain one, or more than one, single base changes, insertions, deletions, inversions or rearrangements with respect to the genomic sequence, so long as sufficient homology is present to support homology-directed repair. In some embodiments, the donor sequence comprises a non-homologous sequence flanked by two regions of homology, such that homology-directed repair between the target DNA region and the two flanking sequences results in insertion of the non-homologous sequence at the target region. Donor sequences may also comprise a vector backbone containing sequences that are not homologous to the DNA region of interest and that are not intended for insertion into the DNA region of interest. Generally, the homologous region(s) of a donor sequence will have at least 50% sequence identity to a genomic sequence with which recombination is desired. In certain embodiments, 60%, 70%, 80%, 90%, 95%, 98%, 99%, or 99.9% sequence identity is present. Any value between 1% and 100% sequence identity can be present, depending upon the length of the donor polynucleotide.

The donor sequence may be a single-stranded DNA, a single-stranded RNA, a double-stranded DNA, or a double-stranded RNA. In some cases, the end of the donor sequence not attached to the nanoparticle may be protected (e.g., from exonucleolytic degradation) by methods known to those of skill in the art. For example, one or more dideoxynucleotide residues are added to the 3' terminus of a linear molecule and/or self-complementary oligonucleotides are ligated to one or both ends. See, for example, Chang et al. (1987) Proc. Natl. Acad Sci USA 84:4959-4963; Nehls et al. (1996) Science 272:886-889. Additional methods for protecting exogenous polynucleotides from degradation include, but are not limited to, addition of terminal amino group(s) and the use of modified internucleotide linkages such as, for example, phosphorothioates, phosphoramidates, and O-methyl ribose or deoxyribose residues.

### Cas9 polypeptides

A Cas9 polypeptide that is suitable for inclusion in a complex (e.g., an encapsulated complex) of the present disclosure can be an enzymatically active Cas9 polypeptide, e.g., can make single- or double-stranded breaks in a target nucleic acid. A Cas9 polypeptide that is suitable for inclusion in a complex of the present disclosure can have reduced enzymatic activity compared to a wild-type Cas9 polypeptide, e.g., compared to a Cas9 polypeptide comprises an amino acid sequence set forth in SEQ ID NO:5.

Suitable Cas9 polypeptides for inclusion in a complex of the present disclosure include a naturally-occurring Cas9 polypeptide (e.g., naturally occurs in bacterial and/or archaeal cells), or a non- naturally-occurring Cas9 polypeptide (e.g., the Cas9 polypeptide is a variant Cas9 polypeptide, a chimeric polypeptide as discussed below, and the like), as described below.

Exemplary Cas9 polypeptides are set forth in SEQ ID NOs: 5-826 as a non-limiting and non-exhaustive list. Naturally occurring Cas9 polypeptides bind a guide nucleic acid, are thereby directed to a specific sequence within a target nucleic acid (a target site), and cleave the target nucleic acid (e.g., cleave dsDNA to generate a double strand break, cleave ssDNA, cleave ssRNA, etc.). A subject Cas9 polypeptide comprises two portions, an RNA-binding portion and an activity portion. An RNA-binding portion interacts with a subject guide nucleic acid. An activity portion exhibits site-directed enzymatic activity (e.g., nuclease activity, activity for DNA and/or RNA methylation, activity for DNA and/or RNA cleavage, activity for histone acetylation, activity for histone methylation, activity for RNA modification, activity for RNA-binding, activity for RNA splicing etc.). In some cases the activity portion exhibits reduced nuclease activity relative to the corresponding portion of a wild type Cas9 polypeptide. In some cases, the activity portion is enzymatically inactive.

Assays to determine whether a protein has an RNA-binding portion interacts with a subject guide nucleic acid can be any convenient binding assay that tests for binding between a protein and a nucleic acid. Exemplary binding assays include binding assays (e.g., gel shift assays) that involve adding a guide nucleic acid and a Cas9 polypeptide to a target nucleic acid.

Assays to determine whether a protein has an activity portion (e.g., to determine if the polypeptide has nuclease activity that cleave a target nucleic acid) can be any convenient nucleic acid cleavage assay that tests for nucleic acid cleavage. Exemplary cleavage assays that include adding a guide nucleic acid and a Cas9 polypeptide to a target nucleic acid.

In some cases, a suitable Cas9 polypeptide for inclusion in a complex of the present disclosure has enzymatic activity that modifies target nucleic acid (e.g., nuclease activity, methyltransferase activity, demethylase activity, DNA repair activity, DNA damage activity, deamination activity, dismutase activity, alkylation activity, depurination activity, oxidation activity, pyrimidine dimer forming activity, integrase activity, transposase activity, recombinase activity, polymerase activity, ligase activity, helicase activity, photolyase activity or glycosylase activity).

In other cases, a suitable Cas9 polypeptide for inclusion in a complex of the present disclosure has enzymatic activity that modifies a polypeptide (e.g., a histone) associated with target nucleic acid (e.g., methyltransferase activity, demethylase activity, acetyltransferase activity, deacetylase activity, kinase activity, phosphatase activity, ubiquitin ligase activity, deubiquitinating activity, adenylation activity, deadenylation activity, SUMOylating activity, deSUMOylating activity, ribosylation activity, deribosylation activity, myristoylation activity or demyristoylation activity).

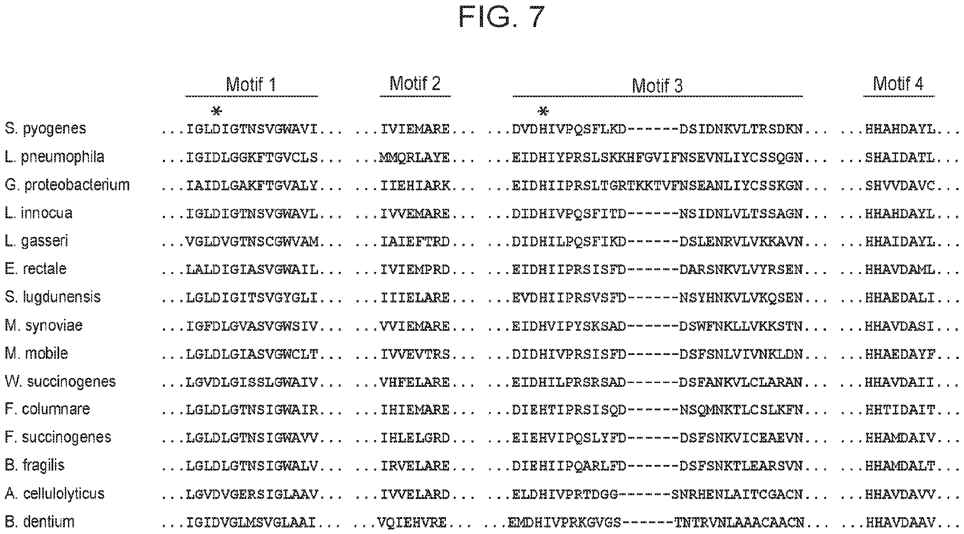

Many Cas9 orthologues from a wide variety of species have been identified and in some cases, the proteins share only a few identical amino acids. All identified Cas9 orthologues have the same domain architecture with a central HNH endonuclease domain and a split RuvC/RNaseH domain. Cas9 proteins share 4 key motifs with a conserved architecture. Motifs 1, 2, and 4 are RuvC like motifs while motif 3 is an HNH-motif. In some cases, a suitable Cas9 polypeptide comprises an amino acid sequence having 4 motifs, each of motifs 1-4 having 60% or more, 70% or more, 75% or more, 80% or more, 85% or more, 90% or more, 95% or more, 99% or more or 100% amino acid sequence identity to motifs 1-4 of the Cas9 amino acid sequence depicted in FIG. 6A (SEQ ID NO:5) (motifs 1-4 of SEQ ID NO:5 are SEQ ID NOs:1-4, respectively, as depicted in Table 1), or to the corresponding portions in any of the amino acid sequences set forth in SEQ ID NOs:6-826 (see FIG. 7 for an alignment of motifs 1-4 from divergent Cas9 sequences).

In some cases, a suitable Cas9 polypeptide comprises an amino acid sequence having 60% or more, 70% or more, 75% or more, 80% or more, 85% or more, 90% or more, 95% or more, 99% or more or 100% amino acid sequence identity to one of the following amino acid sequences: (*Streptococcus pyogenes* (SEQ ID NO:5), *Legionella pneumophila* (SEQ ID NO:32), *Gamma proteobacterium* (SEQ ID NO:122), *Listeria innocua* (SEQ ID NO:19), *Lactobacillus gasseri* (SEQ ID NO:167), *Eubacterium rectale* (SEQ ID NO:114), *Staphylococcus lugdunensis* (SEQ ID NO:200), *Mycoplasma synoviae* (SEQ ID NO:37), *Mycoplasma mobile* (SEQ ID NO:31), *Wolinella succinogenes* (SEQ ID NO:25), *Flavobacterium columnare* (SEQ ID NO:250), *Fibrobacter succinogenes* (SEQ ID NO:136), *Bacteroides fragilis* (SEQ ID NO:36), *Acidothermus cellulolyticus* (SEQ ID NO:57), and *Bifidobacterium dentium* (SEQ ID NO:146).

In some cases, a suitable Cas9 polypeptide comprises an amino acid sequence having 4 motifs, each of motifs 1-4 having 60% or more amino acid sequence identity to motifs 1-4 of the Cas9 amino acid sequence depicted in FIG. 6A (SEQ ID NO:5) (motifs 1-4 of SEQ ID NO:5 are SEQ ID NOs:1-4, respectively, as depicted in Table 1), or to the corresponding portions in any of the amino acid sequences set forth in SEQ ID NOs:6-826 (see FIG. 7 for an alignment of motifs 1-4 from divergent Cas9 sequences).

In some cases, a suitable Cas9 polypeptide comprises an amino acid sequence having 4 motifs, each of motifs 1-4 having 70% or more amino acid sequence identity to motifs 1-4 of the Cas9 amino acid sequence depicted in FIG. 6A (SEQ ID NO:5) (motifs 1-4 of SEQ ID NO:5 are SEQ ID NOs:1-4, respectively, as depicted in Table 1), or to the corresponding portions in any of the amino acid sequences set forth in SEQ ID NOs:6-826 (see FIG. 7 for an alignment of motifs 1-4 from divergent Cas9 sequences).

In some cases, a suitable Cas9 polypeptide comprises an amino acid sequence having 4 motifs, each of motifs 1-4 having 75% or more amino acid sequence identity to motifs 1-4 of the Cas9 amino acid sequence depicted in FIG. 6A (SEQ ID NO:5) (motifs 1-4 of SEQ ID NO:5 are SEQ ID NOs:1-4, respectively, as depicted in Table 1), or to the corresponding portions in any of the amino acid sequences set forth in SEQ ID NOs:6-826 (see FIG. 7 for an alignment of motifs 1-4 from divergent Cas9 sequences).

In some cases, a suitable Cas9 polypeptide comprises an amino acid sequence having 4 motifs, each of motifs 1-4 having 80% or more amino acid sequence identity to motifs 1-4 of the Cas9 amino acid sequence depicted in FIG. 6A (SEQ ID NO:5) (motifs 1-4 of SEQ ID NO:5 are SEQ ID NOs:1-4, respectively, as depicted in Table 1), or to the corresponding portions in any of the amino acid sequences set forth in SEQ ID NOs:6-826 (see FIG. 7 for an alignment of motifs 1-4 from divergent Cas9 sequences).

In some cases, a suitable Cas9 polypeptide comprises an amino acid sequence having 4 motifs, each of motifs 1-4 having 85% or more amino acid sequence identity to motifs 1-4 of the Cas9 amino acid sequence depicted in FIG. 6A (SEQ ID NO:5) (motifs 1-4 of SEQ ID NO:5 are SEQ ID NOs:1-4, respectively, as depicted in Table 1), or to the corresponding portions in any of the amino acid sequences set forth in SEQ ID NOs:6-826 (see FIG. 7 for an alignment of motifs 1-4 from divergent Cas9 sequences).

In some cases, a suitable Cas9 polypeptide comprises an amino acid sequence having 4 motifs, each of motifs 1-4 having 90% or more amino acid sequence identity to motifs 1-4 of the Cas9 amino acid sequence depicted in FIG. 6A (SEQ ID NO:5) (motifs 1-4 of SEQ ID NO:5 are SEQ ID NOs:1-4, respectively, as depicted in Table 1), or to the corresponding portions in any of the amino acid sequences set forth in SEQ ID NOs:6-826 (see FIG. 7 for an alignment of motifs 1-4 from divergent Cas9 sequences).

In some cases, a suitable Cas9 polypeptide comprises an amino acid sequence having 4 motifs, each of motifs 1-4 having 95% or more amino acid sequence identity to motifs 1-4 of the Cas9 amino acid sequence depicted in FIG. 6A (SEQ ID NO:5) (motifs 1-4 of SEQ ID NO:5 are SEQ ID NOs:1-4, respectively, as depicted in Table 1), or to the corresponding portions in any of the amino acid sequences set forth in SEQ ID NOs:6-826 (see FIG. 7 for an alignment of motifs 1-4 from divergent Cas9 sequences).

In some cases, a suitable Cas9 polypeptide comprises an amino acid sequence having 4 motifs, each of motifs 1-4 having 99% or more amino acid sequence identity to motifs 1-4 of the Cas9 amino acid sequence depicted in FIG. 6A (SEQ ID NO:5) (motifs 1-4 of SEQ ID NO:5 are SEQ ID NOs:1-4, respectively, as depicted in Table 1), or to the corresponding portions in any of the amino acid sequences set forth in SEQ ID NOs:6-826 (see FIG. 7 for an alignment of motifs 1-4 from divergent Cas9 sequences).

In some cases, a suitable Cas9 polypeptide comprises an amino acid sequence having 4 motifs, each of motifs 1-4 having 100% amino acid sequence identity to motifs 1-4 of the Cas9 amino acid sequence depicted in FIG. 6A (SEQ ID NO:5) (motifs 1-4 of SEQ ID NO:5 are SEQ ID NOs:1-4, respectively, as depicted in Table 1), or to the corresponding portions in any of the amino acid sequences set forth in SEQ ID NOs:6-826 (see FIG. 7 for an alignment of motifs 1-4 from divergent Cas9 sequences).

In some cases, a suitable Cas9 polypeptide comprises an amino acid sequence having 60% or more, 70% or more, 75% or more, 80% or more, 85% or more, 90% or more, 95% or more, 99% or more or 100% amino acid sequence identity to amino acids 7-166 or 731-1003 of the Cas9 amino acid sequence depicted in FIG. 6A (SEQ ID NO:5), or to the corresponding portions in any of the amino acid sequences set forth as SEQ ID NOs:6-826. Any Cas9 protein as defined above can be used as a Cas9 polypeptide, or as part of a chimeric Cas9 polypeptide, in a complex of the present disclosure.

In some cases, a suitable Cas9 polypeptide comprises an amino acid sequence having 60% or more amino acid sequence identity to amino acids 7-166 or 731-1003 of the Cas9 amino acid sequence depicted in FIG. 6A (SEQ ID NO:5), or to the corresponding portions in any of the amino acid sequences set forth as SEQ ID NOs:6-826. Any Cas9 protein as defined above can be used as a Cas9 polypeptide, or as part of a chimeric Cas9 polypeptide, in a complex of the present disclosure.

In some cases, a suitable Cas9 polypeptide comprises an amino acid sequence having 70% or more amino acid sequence identity to amino acids 7-166 or 731-1003 of the Cas9 amino acid sequence depicted in FIG. 6A (SEQ ID NO:5), or to the corresponding portions in any of the amino acid sequences set forth as SEQ ID NOs:6-826. Any Cas9 protein as defined above can be used as a Cas9 polypeptide, or as part of a chimeric Cas9 polypeptide, in a complex of the present disclosure.

In some cases, a suitable Cas9 polypeptide comprises an amino acid sequence having 75% or more amino acid sequence identity to amino acids 7-166 or 731-1003 of the Cas9 amino acid sequence depicted in FIG. 6A (SEQ ID NO:5), or to the corresponding portions in any of the amino acid sequences set forth as SEQ ID NOs:6-826. Any Cas9 protein as defined above can be used as a Cas9 polypeptide, or as part of a chimeric Cas9 polypeptide, in a complex of the present disclosure.

In some cases, a suitable Cas9 polypeptide comprises an amino acid sequence having 80% or more amino acid sequence identity to amino acids 7-166 or 731-1003 of the Cas9 amino acid sequence depicted in FIG. 6A (SEQ ID NO:5), or to the corresponding portions in any of the amino acid sequences set forth as SEQ ID NOs:6-826. Any Cas9 protein as defined above can be used as a Cas9 polypeptide, or as part of a chimeric Cas9 polypeptide, in a complex of the present disclosure.

In some cases, a suitable Cas9 polypeptide comprises an amino acid sequence having 85% or more amino acid sequence identity to amino acids 7-166 or 731-1003 of the Cas9 amino acid sequence depicted in FIG. 6A (SEQ ID NO:5), or to the corresponding portions in any of the amino acid sequences set forth as SEQ ID NOs:6-826. Any Cas9 protein as defined above can be used as a Cas9 polypeptide, or as part of a chimeric Cas9 polypeptide, in a complex of the present disclosure.

In some cases, a suitable Cas9 polypeptide comprises an amino acid sequence having 90% or more amino acid sequence identity to amino acids 7-166 or 731-1003 of the Cas9 amino acid sequence depicted in FIG. 6A (SEQ ID NO:5), or to the corresponding portions in any of the amino acid sequences set forth as SEQ ID NOs:6-826. Any Cas9 protein as defined above can be used as a Cas9 polypeptide, or as part of a chimeric Cas9 polypeptide, in a complex of the present disclosure.

In some cases, a suitable Cas9 polypeptide comprises an amino acid sequence having 95% or more amino acid sequence identity to amino acids 7-166 or 731-1003 of the Cas9 amino acid sequence depicted in FIG. 6A (SEQ ID NO:5), or to the corresponding portions in any of the amino acid sequences set forth as SEQ ID NOs:6-826. Any Cas9 protein as defined above can be used as a Cas9 polypeptide, or as part of a chimeric Cas9 polypeptide, in a complex of the present disclosure.

In some cases, a suitable Cas9 polypeptide comprises an amino acid sequence having 99% or more amino acid sequence identity to amino acids 7-166 or 731-1003 of the Cas9 amino acid sequence depicted in FIG. 6A (SEQ ID NO:5), or to the corresponding portions in any of the amino acid sequences set forth as SEQ ID NOs:6-826. Any Cas9 protein as defined above can be used as a Cas9 polypeptide, or as part of a chimeric Cas9 polypeptide, in a complex of the present disclosure.

In some cases, a suitable Cas9 polypeptide comprises an amino acid sequence having 100% amino acid sequence identity to amino acids 7-166 or 731-1003 of the Cas9 amino acid sequence depicted in FIG. 6A (SEQ ID NO:5), or to the corresponding portions in any of the amino acid sequences set forth as SEQ ID NOs:6-826. Any Cas9 protein as defined above can be used as a Cas9 polypeptide, or as part of a chimeric Cas9 polypeptide, in a complex of the present disclosure.

In some cases, a suitable Cas9 polypeptide comprises an amino acid sequence having 60% or more, 70% or more, 75% or more, 80% or more, 85% or more, 90% or more, 95% or more, 99% or more or 100% amino acid sequence identity to the Cas9 amino acid sequence depicted in FIG. 6A (SEQ ID NO:5), or to any of the amino acid sequences set forth as SEQ ID NOs:6-826. Any Cas9 protein as defined above can be used as a Cas9 polypeptide, or as part of a chimeric Cas9 polypeptide, in a complex of the present disclosure.

In some cases, a suitable Cas9 polypeptide comprises an amino acid sequence having 60% or more amino acid sequence identity to the Cas9 amino acid sequence depicted in FIG. 6A (SEQ ID NO:5), or to any of the amino acid sequences set forth as SEQ ID NOs:6-826. Any Cas9 protein as defined above can be used as a Cas9 polypeptide, or as part of a chimeric Cas9 polypeptide, in a complex of the present disclosure.

In some cases, a suitable Cas9 polypeptide comprises an amino acid sequence having 70% or more amino acid sequence identity to the Cas9 amino acid sequence depicted in FIG. 6A (SEQ ID NO:5), or to any of the amino acid sequences set forth as SEQ ID NOs:6-826. Any Cas9 protein as defined above can be used as a Cas9 polypeptide, or as part of a chimeric Cas9 polypeptide, in a complex of the present disclosure.

In some cases, a suitable Cas9 polypeptide comprises an amino acid sequence having 75% or more amino acid sequence identity to the Cas9 amino acid sequence depicted in FIG. 6A (SEQ ID NO:5), or to any of the amino acid sequences set forth as SEQ ID NOs:6-826. Any Cas9 protein as defined above can be used as a Cas9 polypeptide, or as part of a chimeric Cas9 polypeptide, in a complex of the present disclosure.

In some cases, a suitable Cas9 polypeptide comprises an amino acid sequence having 80% or more amino acid sequence identity to the Cas9 amino acid sequence depicted in FIG. 6A (SEQ ID NO:5), or to any of the amino acid sequences set forth as SEQ ID NOs:6-826. Any Cas9 protein as defined above can be used as a Cas9 polypeptide, or as part of a chimeric Cas9 polypeptide, in a complex of the present disclosure..

In some cases, a suitable Cas9 polypeptide comprises an amino acid sequence having 85% or more amino acid sequence identity to the Cas9 amino acid sequence depicted in FIG. 6A (SEQ ID NO:5), or to any of the amino acid sequences set forth as SEQ ID NOs:6-826. Any Cas9 protein as defined above can be used as a Cas9 polypeptide, or as part of a chimeric Cas9 polypeptide, in a complex of the present disclosure.

In some cases, a suitable Cas9 polypeptide comprises an amino acid sequence having 90% or more amino acid sequence identity to the Cas9 amino acid sequence depicted in FIG. 6A (SEQ ID NO:5), or to any of the amino acid sequences set forth as SEQ ID NOs:6-826. Any Cas9 protein as defined above can be used as a Cas9 polypeptide, or as part of a chimeric Cas9 polypeptide, in a complex of the present disclosure.

In some cases, a suitable Cas9 polypeptide comprises an amino acid sequence having 95% or more amino acid sequence identity to the Cas9 amino acid sequence depicted in FIG. 6A (SEQ ID NO:5), or to any of the amino acid sequences set forth as SEQ ID NOs:6-826. Any Cas9 protein as defined above can be used as a Cas9 polypeptide, or as part of a chimeric Cas9 polypeptide, in a complex of the present disclosure.

In some cases, a suitable Cas9 polypeptide comprises an amino acid sequence having 99% or more amino acid sequence identity to the Cas9 amino acid sequence depicted in FIG. 6A (SEQ ID NO:5), or to any of the amino acid sequences set forth as SEQ ID NOs:6-826. Any Cas9 protein as defined above can be used as a Cas9 polypeptide, or as part of a chimeric Cas9 polypeptide, in a complex of the present disclosure.

In some cases, a suitable Cas9 polypeptide comprises an amino acid sequence having 100% amino acid sequence identity to the Cas9 amino acid sequence depicted in FIG. 6A (SEQ ID NO:5), or to any of the amino acid sequences set forth as SEQ ID NOs:6-826. Any Cas9 protein as defined above can be used as a Cas9 polypeptide, or as part of a chimeric Cas9 polypeptide, in a complex of the present disclosure.

In some cases, a Cas9 polypeptide comprises 4 motifs (as listed in Table 1 and depicted in FIG. 6A and FIG. 7), at least one with (or each with) amino acid sequences having 75% or more, 80% or more, 85% or more, 90% or more, 95% or more, 99% or more or 100% amino acid sequence identity to each of the 4 motifs listed in Table 1(SEQ ID NOs:1-4), or to the corresponding portions in any of the amino acid sequences set forth as SEQ ID NOs:6-826.

In some cases, a Cas9 polypeptide comprises an amino acid sequence having 75% or more, 80% or more, 85% or more, 90% or more, 95% or more, or 98%, amino acid sequence identity to the amino acid sequence depicted in FIG. 6A and set forth in SEQ ID NO:5; and has amino acid substitutions of N497, R661, Q695, and Q926 relative to the amino acid sequence set forth in SEQ ID NO:5. In some cases, a Cas9 polypeptide comprises an amino acid sequence having 75% or more, 80% or more, 85% or more, 90% or more, 95% or more, or 98%, amino acid sequence identity to the amino acid sequence depicted in FIG. 6A and set forth in SEQ ID NO:5; and has amino acid substitutions N497A, R661A, Q695A, and Q926A relative to the amino acid sequence set forth in SEQ ID NO:5.

In some cases, a Cas9 polypeptide comprises an amino acid sequence having 75% or more, 80% or more, 85% or more, 90% or more, 95% or more, or 98%, amino acid sequence identity to the amino acid sequence depicted in FIG. 6A and set forth in SEQ ID NO:5; and has an amino acid substitution of K855 relative to the amino acid sequence set forth in SEQ ID NO:5. In some cases, a Cas9 polypeptide comprises an amino acid sequence having 75% or more, 80% or more, 85% or more, 90% or more, 95% or more, or 98%, amino acid sequence identity to the amino acid sequence depicted in FIG. 6A and set forth in SEQ ID NO:5; and has the amino acid substitution K855A relative to the amino acid sequence set forth in SEQ ID NO:5.

In some cases, a Cas9 polypeptide comprises an amino acid sequence having 75% or more, 80% or more, 85% or more, 90% or more, 95% or more, or 98%, amino acid sequence identity to the amino acid sequence depicted in FIG. 6A and set forth in SEQ ID NO:5; and has amino acid substitutions of K810, K1003, and R1060 relative to the amino acid sequence set forth in SEQ ID NO:5. In some cases, a Cas9 polypeptide comprises an amino acid sequence having 75% or more, 80% or more, 85% or more, 90% or more, 95% or more, or 98%, amino acid sequence identity to the amino acid sequence depicted in FIG. 6A and set forth in SEQ ID NO:5; and has amino acid substitutions K810A, K1003A, and R1060A relative to the amino acid sequence set forth in SEQ ID NO:5.

In some cases, a Cas9 polypeptide comprises an amino acid sequence having 75% or more, 80% or more, 85% or more, 90% or more, 95% or more, or 98%, amino acid sequence identity to the amino acid sequence depicted in FIG. 6A and set forth in SEQ ID NO:5; and has amino acid substitutions of K848, K1003, and R1060 relative to the amino acid sequence set forth in SEQ ID NO:5. In some cases, a Cas9 polypeptide comprises an amino acid sequence having 75% or more, 80% or more, 85% or more, 90% or more, 95% or more, or 98%, amino acid sequence identity to the amino acid sequence depicted in FIG. 6A and set forth in SEQ ID NO:5; and has amino acid substitutions K848A, K1003A, and R1060A relative to the amino acid sequence set forth in SEQ ID NO:5.

As used herein, the term "Cas9 polypeptide" encompasses the term "variant Cas9 polypeptide"; and the term "variant Cas9 polypeptide" encompasses the term "chimeric Cas9 polypeptide."

### Variant Cas9 polypeptides

A suitable Cas9 polypeptide for inclusion in a complex of the present disclosure includes a variant Cas9 polypeptide. A variant Cas9 polypeptide has an amino acid sequence that is different by one amino acid (e.g., has a deletion, insertion, substitution, fusion) (i.e., different by at least one amino acid) when compared to the amino acid sequence of a wild type Cas9 polypeptide (e.g., a naturally occurring Cas9 polypeptide, as described above). In some instances, the variant Cas9 polypeptide has an amino acid change (e.g., deletion, insertion, or substitution) that reduces the nuclease activity of the Cas9 polypeptide. For example, in some instances, the variant Cas9 polypeptide has less than 50%, less than 40%, less than 30%, less than 20%, less than 10%, less than 5%, or less than 1% of the nuclease activity of the corresponding wild-type Cas9 polypeptide. In some cases, the variant Cas9 polypeptide has no substantial nuclease activity. When a Cas9 polypeptide is a variant Cas9 polypeptide that has no substantial nuclease activity, it can be referred to as "dCas9."

In some cases, a variant Cas9 polypeptide has reduced nuclease activity. For example, a variant Cas9 polypeptide suitable for use in a binding method of the present disclosure exhibits less than about 20%, less than about 15%, less than about 10%, less than about 5%, less than about 1%, or less than about 0.1%, of the endonuclease activity of a wild-type Cas9 polypeptide, e.g., a wild-type Cas9 polypeptide comprising an amino acid sequence as depicted in FIG. 6A (SEQ ID NO:5).

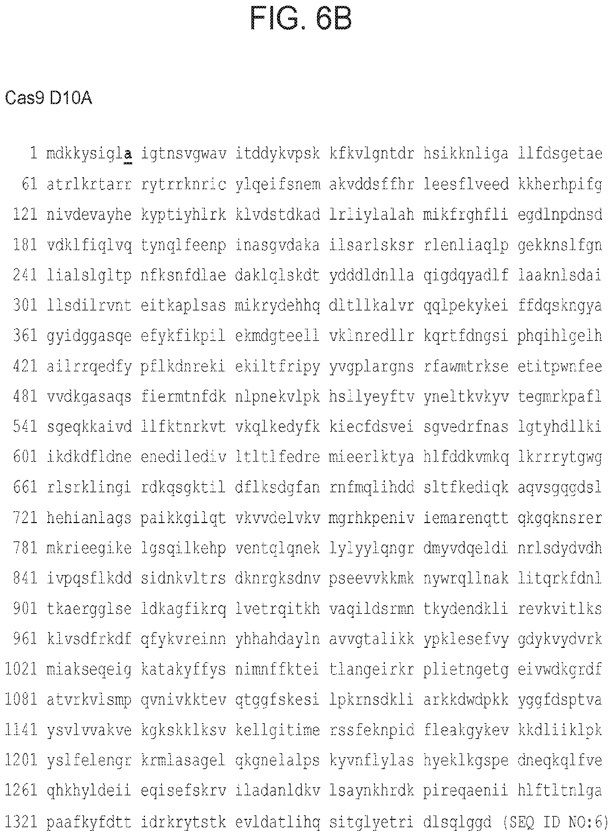

In some cases, a variant Cas9 polypeptide can cleave the complementary strand of a target nucleic acid but has reduced ability to cleave the non-complementary strand of a double stranded target nucleic acid. For example, the variant Cas9 polypeptide can have a mutation (amino acid substitution) that reduces the function of the RuvC domain (e.g., "domain 1" of FIG. 6A). As a non-limiting example, in some embodiments, a variant Cas9 polypeptide has a D10A mutation (e.g., aspartate to alanine at an amino acid position corresponding to position 10 of SEQ ID NO:5) (FIG. 6B) (e.g., or the corresponding mutation of any of the proteins presented in SEQ ID NOs:6-826) and can therefore cleave the complementary strand of a double stranded target nucleic acid but has reduced ability to cleave the non-complementary strand of a double stranded target nucleic acid (thus resulting in a single strand break (SSB) instead of a double strand break (DSB) when the variant Cas9 polypeptide cleaves a double stranded target nucleic acid) (see, for example, Jinek et al., Science. 2012 Aug 17;337(6096):816-21).

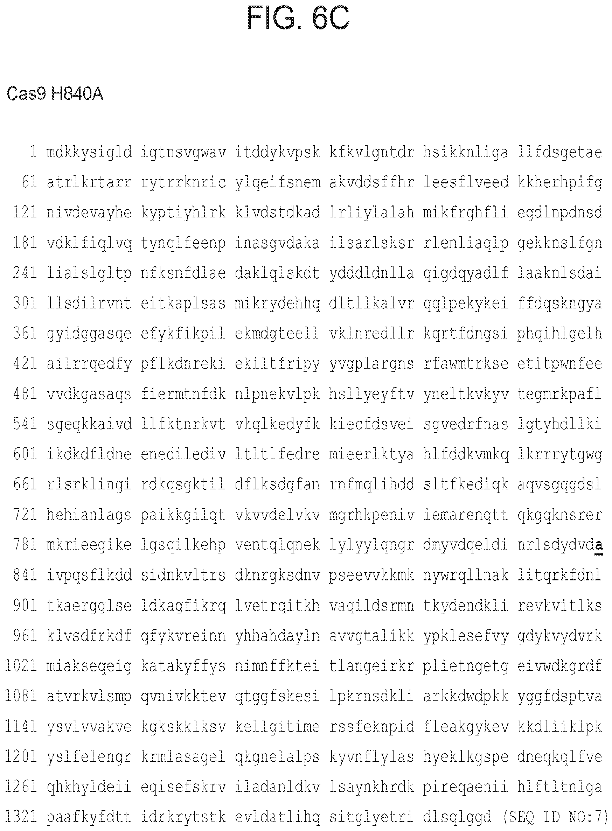

In some cases, a variant Cas9 polypeptide can cleave the non-complementary strand of a double stranded target nucleic acid but has reduced ability to cleave the complementary strand of the target nucleic acid. For example, the variant Cas9 polypeptide can have a mutation (amino acid substitution) that reduces the function of the HNH domain (RuvC/HNH/RuvC domain motifs, "domain 2" of FIG. 6A). As a non-limiting example, in some embodiments, the variant Cas9 polypeptide can have an H840A mutation (e.g., histidine to alanine at an amino acid position corresponding to position 840 of SEQ ID NO:5) (FIG. 6C) (or the corresponding mutation of any of the proteins set forth as SEQ ID NOs:6-826) and can therefore cleave the non-complementary strand of the target nucleic acid but has reduced ability to cleave the complementary strand of the target nucleic acid (thus resulting in a SSB instead of a DSB when the variant Cas9 polypeptide cleaves a double stranded target nucleic acid). Such a Cas9 polypeptide has a reduced ability to cleave a target nucleic acid (e.g., a single stranded target nucleic acid) but retains the ability to bind a target nucleic acid (e.g., a single-stranded or a double-stranded target nucleic acid).

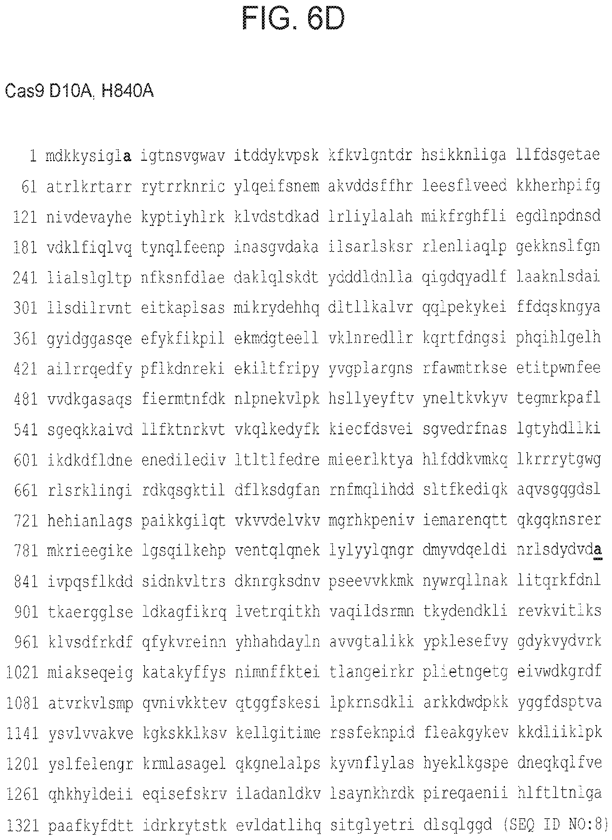

In some cases, a variant Cas9 polypeptide has a reduced ability to cleave both the complementary and the non-complementary strands of a double stranded target nucleic acid. As a non-limiting example, in some cases, the variant Cas9 polypeptide harbors both the D10A and the H840A mutations (e.g., mutations in both the RuvC domain and the HNH domain) (FIG. 6D) (or the corresponding mutations of any of the proteins set forth as SEQ ID NOs:6-826) such that the polypeptide has a reduced ability to cleave both the complementary and the non-complementary strands of a double stranded target nucleic acid. Such a Cas9 polypeptide has a reduced ability to cleave a target nucleic acid (e.g., a single-stranded target nucleic acid or a double-stranded target nucleic acid) but retains the ability to bind a target nucleic acid (e.g., a single stranded target nucleic acid or a double-stranded target nucleic acid).

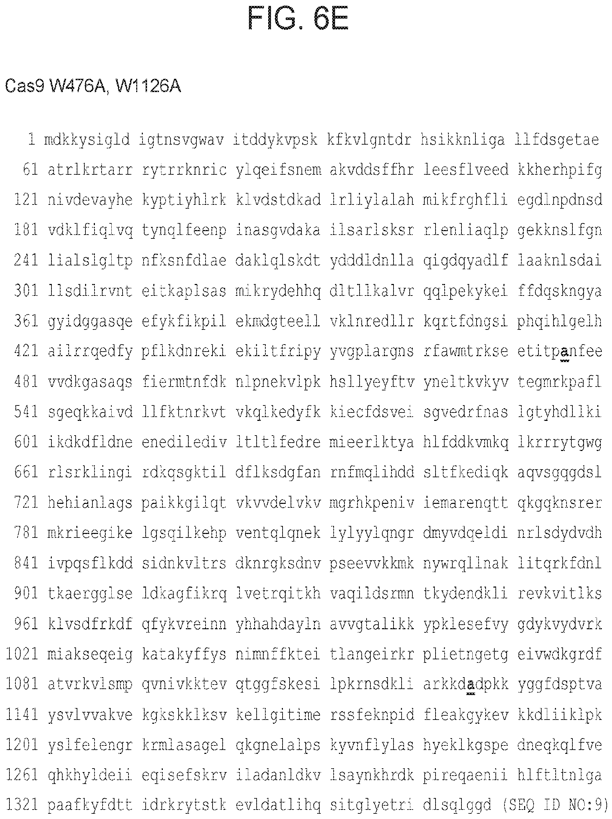

As another non-limiting example, in some cases, the variant Cas9 polypeptide harbors W476A and W1126A mutations (FIG. 6E) (or the corresponding mutations of any of the proteins set forth as SEQ ID NOs:5-826) such that the polypeptide has a reduced ability to cleave a target nucleic acid. Such a Cas9 polypeptide has a reduced ability to cleave a target nucleic acid but retains the ability to bind a target nucleic acid.

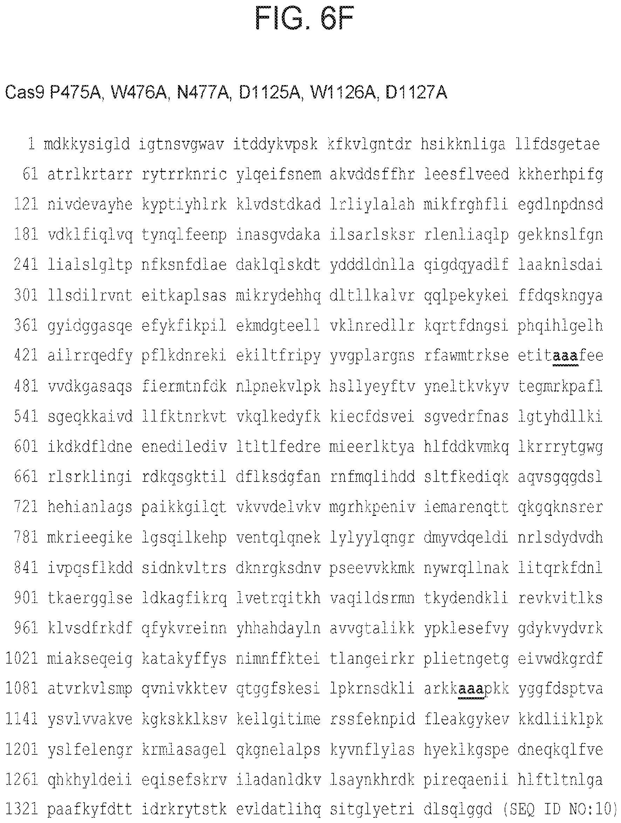

As another non-limiting example, in some cases, the variant Cas9 polypeptide harbors P475A, W476A, N477A, D1125A, W1126A, and D1127A mutations (FIG. 6F) (or the corresponding mutations of any of the proteins set forth as SEQ ID NOs:5-826) such that the polypeptide has a reduced ability to cleave a target nucleic acid. Such a Cas9 polypeptide has a reduced ability to cleave a target nucleic acid but retains the ability to bind a target nucleic acid.

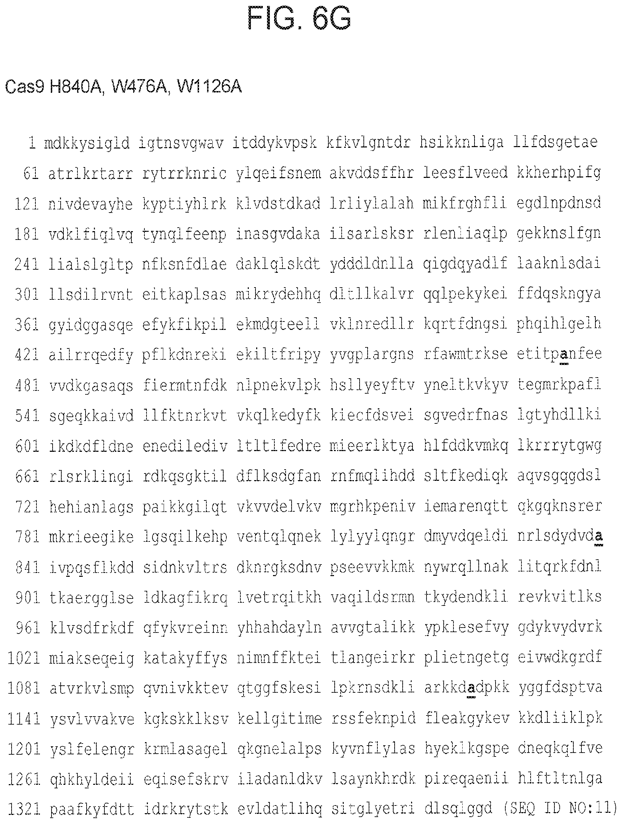

As another non-limiting example, in some cases, the variant Cas9 polypeptide harbors H840A, W476A, and W1126A, mutations (FIG. 6G) (or the corresponding mutations of any of the proteins set forth as SEQ ID NOs:5-826) such that the polypeptide has a reduced ability to cleave a target nucleic acid. Such a Cas9 polypeptide has a reduced ability to cleave a target nucleic acid but retains the ability to bind a target nucleic acid.

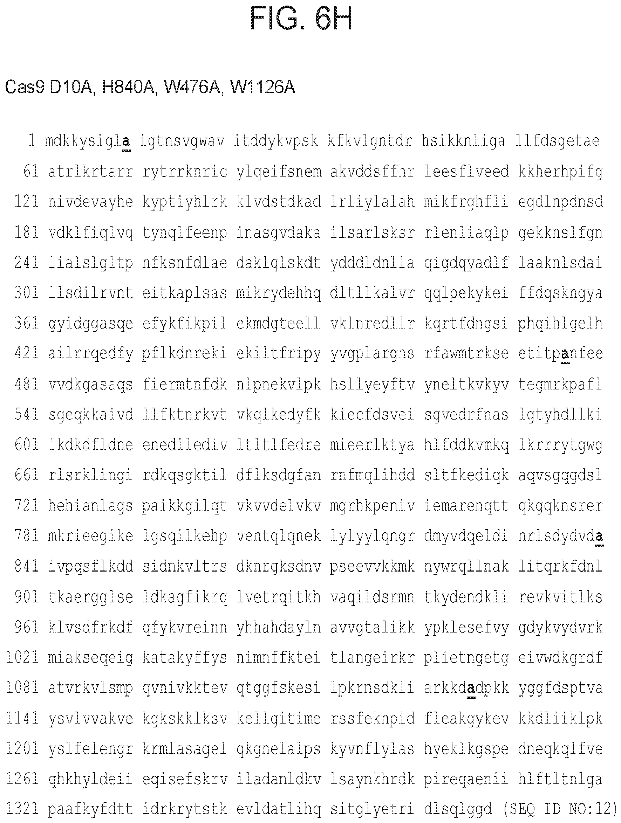

As another non-limiting example, in some cases, the variant Cas9 polypeptide harbors H840A, D10A, W476A, and W1126A, mutations (FIG. 6H) (or the corresponding mutations of any of the proteins set forth as SEQ ID NOs:5-826) such that the polypeptide has a reduced ability to cleave a target nucleic acid. Such a Cas9 polypeptide has a reduced ability to cleave a target nucleic acid but retains the ability to bind a target nucleic acid.

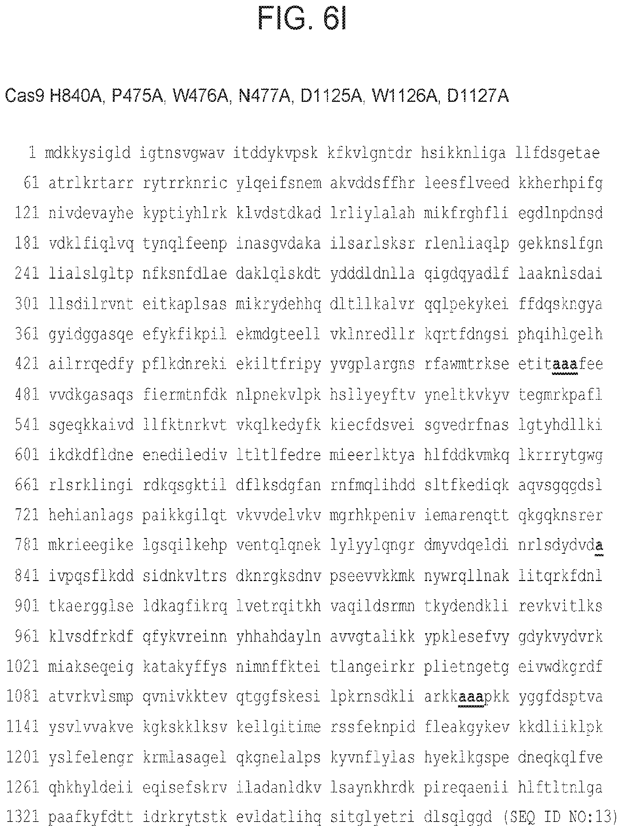

As another non-limiting example, in some cases, the variant Cas9 polypeptide harbors, H840A, P475A, W476A, N477A, D1125A, W1126A, and D1127A mutations (FIG. 6I) (or the corresponding mutations of any of the proteins set forth as SEQ ID NOs:5-826) such that the polypeptide has a reduced ability to cleave a target nucleic acid. Such a Cas9 polypeptide has a reduced ability to cleave a target nucleic acid but retains the ability to bind a target nucleic acid.

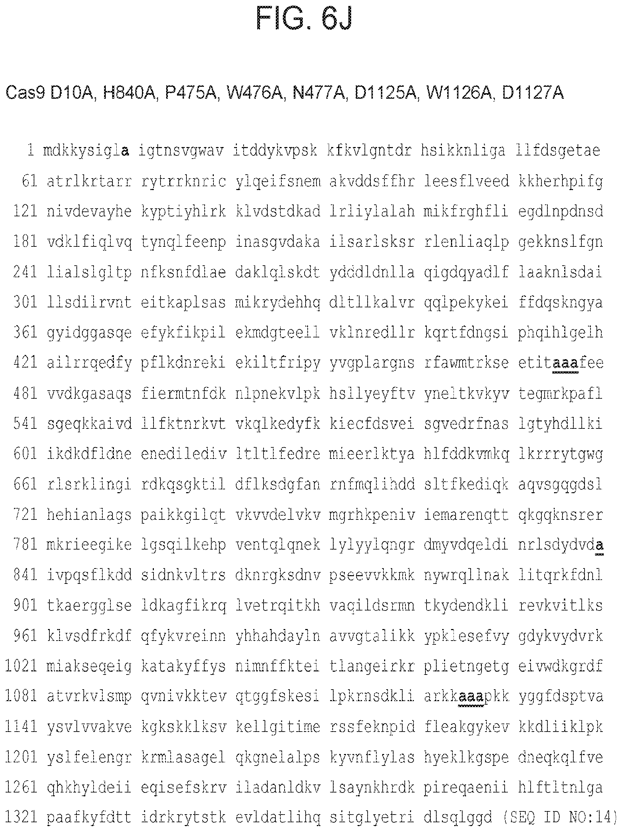

As another non-limiting example, in some cases, the variant Cas9 polypeptide harbors D10A, H840A, P475A, W476A, N477A, D1125A, W1126A, and D1127A mutations (FIG. 6J) (or the corresponding mutations of any of the proteins set forth as SEQ ID NOs:5-826) such that the polypeptide has a reduced ability to cleave a target nucleic acid. Such a Cas9 polypeptide has a reduced ability to cleave a target nucleic acid but retains the ability to bind a target nucleic acid.

Other residues can be mutated to achieve the above effects (i.e. inactivate one or the other nuclease portions). As non-limiting examples, residues D10, G12, G17, E762, H840, N854, N863, H982, H983, A984, D986, and/or A987 (or the corresponding mutations of any of the proteins set forth as SEQ ID NOs:5-826) can be altered (i.e., substituted) (see **Table 1** for more information regarding the conservation of Cas9 amino acid residues). Also, mutations other than alanine substitutions are suitable.

In some embodiments, a variant Cas9 polypeptide that has reduced catalytic activity (e.g., when a Cas9 protein has a D10, G12, G17, E762, H840, N854, N863, H982, H983, A984, D986, and/or a A987 mutation, e.g., D10A, G12A, G17A, E762A, H840A, N854A, N863A, H982A, H983A, A984A, and/or D986A), the variant Cas9 polypeptide can still bind to target nucleic acid in a site-specific manner (because it is still guided to a target nucleic acid sequence by a guide nucleic acid) as long as it retains the ability to interact with the guide nucleic acid.

**Table 1. Table 1 lists 4 motifs that are present in Cas9 sequences from various species (e.g., see alignments of Fig. 7). The amino acids listed here are from the Cas9 from S. pyogenes (SEQ ID NO:5).**

| **Motif** | **Motif** | **Amino acids (residue #s)** | **Highly conserved** |
|---|---|---|---|
| 1 | RuvC | IGLDIGTNSVGWAVI (7-21) | D10, G12, G17 |
| | | (SEQ ID NO:1) | |
| 2 | RuvC | IVIEMARE (759-766) | E762 |
| | | (SEQ ID NO:2) | |
| 3 | HNH-motif | DVDHIVPQSFLKDDSIDNKVLTRSDKN | H840, N854, N863 |
| | | (837-863) (SEQ ID NO:3) | |
| 4 | RuvC | HHAHDAYL (982-989) | H982, H983, A984, D986, A987 |
| | | (SEQ ID NO:4) | |

In addition to the above, a variant Cas9 protein can have the same parameters for sequence identity as described above for Cas9 polypeptides. Thus, in some cases, a suitable variant Cas9 polypeptide comprises an amino acid sequence having 4 motifs, each of motifs 1-4 having 60% or more, 70% or more, 75% or more, 80% or more, 85% or more, 90% or more, 95% or more, 99% or more or 100% amino acid sequence identity to motifs 1-4 of the Cas9 amino acid sequence depicted in FIG. 6A (SEQ ID NO:5) (motifs 1-4 of SEQ ID NO:5 are SEQ ID NOs:1-4, respectively, as depicted in Table 1), or to the corresponding portions in any of the amino acid sequences set forth in SEQ ID NOs:6-826 (see FIG. 7 for an alignment of motifs 1-4 from divergent Cas9 sequences).

In some cases, a suitable variant Cas9 polypeptide comprises an amino acid sequence having 4 motifs, each of motifs 1-4 having 60% or more amino acid sequence identity to motifs 1-4 of the Cas9 amino acid sequence depicted in FIG. 6A (SEQ ID NO:5) (motifs 1-4 of SEQ ID NO:5 are SEQ ID NOs:1-4, respectively, as depicted in Table 1), or to the corresponding portions in any of the amino acid sequences set forth in SEQ ID NOs:6-826 (see FIG. 7 for an alignment of motifs 1-4 from divergent Cas9 sequences).

In some cases, a suitable variant Cas9 polypeptide comprises an amino acid sequence having 4 motifs, each of motifs 1-4 having 70% or more amino acid sequence identity to motifs 1-4 of the Cas9 amino acid sequence depicted in FIG. 6A (SEQ ID NO:5) (motifs 1-4 of SEQ ID NO:5 are SEQ ID NOs:1-4, respectively, as depicted in Table 1), or to the corresponding portions in any of the amino acid sequences set forth in SEQ ID NOs:6-826 (see FIG. 7 for an alignment of motifs 1-4 from divergent Cas9 sequences).

In some cases, a suitable variant Cas9 polypeptide comprises an amino acid sequence having 4 motifs, each of motifs 1-4 having 75% or more amino acid sequence identity to motifs 1-4 of the Cas9 amino acid sequence depicted in FIG. 6A (SEQ ID NO:5) (motifs 1-4 of SEQ ID NO:5 are SEQ ID NOs:1-4, respectively, as depicted in Table 1), or to the corresponding portions in any of the amino acid sequences set forth in SEQ ID NOs:6-826 (see FIG. 7 for an alignment of motifs 1-4 from divergent Cas9 sequences).

In some cases, a suitable variant Cas9 polypeptide comprises an amino acid sequence having 4 motifs, each of motifs 1-4 having 80% or more amino acid sequence identity to motifs 1-4 of the Cas9 amino acid sequence depicted in FIG. 6A (SEQ ID NO:5) (motifs 1-4 of SEQ ID NO:5 are SEQ ID NOs:1-4, respectively, as depicted in Table 1), or to the corresponding portions in any of the amino acid sequences set forth in SEQ ID NOs:6-826 (see FIG. 7 for an alignment of motifs 1-4 from divergent Cas9 sequences).

In some cases, a suitable variant Cas9 polypeptide comprises an amino acid sequence having 4 motifs, each of motifs 1-4 having 85% or more amino acid sequence identity to motifs 1-4 of the Cas9 amino acid sequence depicted in FIG. 6A (SEQ ID NO:5) (motifs 1-4 of SEQ ID NO:5 are SEQ ID NOs:1-4, respectively, as depicted in Table 1), or to the corresponding portions in any of the amino acid sequences set forth in SEQ ID NOs:6-826 (see FIG. 7 for an alignment of motifs 1-4 from divergent Cas9 sequences).

In some cases, a suitable variant Cas9 polypeptide comprises an amino acid sequence having 4 motifs, each of motifs 1-4 having 90% or more amino acid sequence identity to motifs 1-4 of the Cas9 amino acid sequence depicted in FIG. 6A (SEQ ID NO:5) (motifs 1-4 of SEQ ID NO:5 are SEQ ID NOs:1-4, respectively, as depicted in Table 1), or to the corresponding portions in any of the amino acid sequences set forth in SEQ ID NOs:6-826 (see FIG. 7 for an alignment of motifs 1-4 from divergent Cas9 sequences).

In some cases, a suitable variant Cas9 polypeptide comprises an amino acid sequence having 4 motifs, each of motifs 1-4 having 95% or more amino acid sequence identity to motifs 1-4 of the Cas9 amino acid sequence depicted in FIG. 6A (SEQ ID NO:5) (motifs 1-4 of SEQ ID NO:5 are SEQ ID NOs:1-4, respectively, as depicted in Table 1), or to the corresponding portions in any of the amino acid sequences set forth in SEQ ID NOs:6-826 (see FIG. 7 for an alignment of motifs 1-4 from divergent Cas9 sequences).

In some cases, a suitable variant Cas9 polypeptide comprises an amino acid sequence having 4 motifs, each of motifs 1-4 having 99% or more amino acid sequence identity to motifs 1-4 of the Cas9 amino acid sequence depicted in FIG. 6A (SEQ ID NO:5) (motifs 1-4 of SEQ ID NO:5 are SEQ ID NOs:1-4, respectively, as depicted in Table 1), or to the corresponding portions in any of the amino acid sequences set forth in SEQ ID NOs:6-826 (see FIG. 7 for an alignment of motifs 1-4 from divergent Cas9 sequences).

In some cases, a suitable variant Cas9 polypeptide comprises an amino acid sequence having 4 motifs, each of motifs 1-4 having 100% amino acid sequence identity to motifs 1-4 of the Cas9 amino acid sequence depicted in FIG. 6A (SEQ ID NO:5) (motifs 1-4 of SEQ ID NO:5 are SEQ ID NOs:1-4, respectively, as depicted in Table 1), or to the corresponding portions in any of the amino acid sequences set forth in SEQ ID NOs:6-826 (see FIG. 7 for an alignment of motifs 1-4 from divergent Cas9 sequences).

In some cases, a suitable variant Cas9 polypeptide comprises an amino acid sequence having 60% or more, 70% or more, 75% or more, 80% or more, 85% or more, 90% or more, 95% or more, 99% or more, or 100% amino acid sequence identity to amino acids 7-166 or 731-1003 of the Cas9 amino acid sequence depicted in FIG. 6A (SEQ ID NO:5), or to the corresponding portions in any of the amino acid sequences set forth as SEQ ID NOs:6-826. Any Cas9 protein as defined above can be used as a Cas9 polypeptide, or as part of a chimeric Cas9 polypeptide, in a complex of the present disclosure.

In some cases, a suitable variant Cas9 polypeptide comprises an amino acid sequence having 60% or more amino acid sequence identity to amino acids 7-166 or 731-1003 of the Cas9 amino acid sequence depicted in FIG. 6A (SEQ ID NO:5), or to the corresponding portions in any of the amino acid sequences set forth as SEQ ID NOs:6-826. Any Cas9 protein as defined above can be used as a Cas9 polypeptide, or as part of a chimeric Cas9 polypeptide, in a complex of the present disclosure.

In some cases, a suitable variant Cas9 polypeptide comprises an amino acid sequence having 70% or more amino acid sequence identity to amino acids 7-166 or 731-1003 of the Cas9 amino acid sequence depicted in FIG. 6A (SEQ ID NO:5), or to the corresponding portions in any of the amino acid sequences set forth as SEQ ID NOs:6-826. Any Cas9 protein as defined above can be used as a Cas9 polypeptide, or as part of a chimeric Cas9 polypeptide, in a complex of the present disclosure.

In some cases, a suitable variant Cas9 polypeptide comprises an amino acid sequence having 75% or more amino acid sequence identity to amino acids 7-166 or 731-1003 of the Cas9 amino acid sequence depicted in FIG. 6A (SEQ ID NO:5), or to the corresponding portions in any of the amino acid sequences set forth as SEQ ID NOs:6-826. Any Cas9 protein as defined above can be used as a Cas9 polypeptide, or as part of a chimeric Cas9 polypeptide, in a complex of the present disclosure.

In some cases, a suitable variant Cas9 polypeptide comprises an amino acid sequence having 80% or more amino acid sequence identity to amino acids 7-166 or 731-1003 of the Cas9 amino acid sequence depicted in FIG. 6A (SEQ ID NO:5), or to the corresponding portions in any of the amino acid sequences set forth as SEQ ID NOs:6-826. Any Cas9 protein as defined above can be used as a Cas9 polypeptide, or as part of a chimeric Cas9 polypeptide, in a complex of the present disclosure.

In some cases, a suitable variant Cas9 polypeptide comprises an amino acid sequence having 85% or more amino acid sequence identity to amino acids 7-166 or 731-1003 of the Cas9 amino acid sequence depicted in FIG. 6A (SEQ ID NO:5), or to the corresponding portions in any of the amino acid sequences set forth as SEQ ID NOs:6-826. Any Cas9 protein as defined above can be used as a Cas9 polypeptide, or as part of a chimeric Cas9 polypeptide, in a complex of the present disclosure.

In some cases, a suitable variant Cas9 polypeptide comprises an amino acid sequence having 90% or more amino acid sequence identity to amino acids 7-166 or 731-1003 of the Cas9 amino acid sequence depicted in FIG. 6A (SEQ ID NO:5), or to the corresponding portions in any of the amino acid sequences set forth as SEQ ID NOs:6-826. Any Cas9 protein as defined above can be used as a Cas9 polypeptide, or as part of a chimeric Cas9 polypeptide, in a complex of the present disclosure.

In some cases, a suitable variant Cas9 polypeptide comprises an amino acid sequence having 95% or more amino acid sequence identity to amino acids 7-166 or 731-1003 of the Cas9 amino acid sequence depicted in FIG. 6A (SEQ ID NO:5), or to the corresponding portions in any of the amino acid sequences set forth as SEQ ID NOs:6-826. Any Cas9 protein as defined above can be used as a Cas9 polypeptide, or as part of a chimeric Cas9 polypeptide, in a complex of the present disclosure.

In some cases, a suitable variant Cas9 polypeptide comprises an amino acid sequence having 99% or more amino acid sequence identity to amino acids 7-166 or 731-1003 of the Cas9 amino acid sequence depicted in FIG. 6A (SEQ ID NO:5), or to the corresponding portions in any of the amino acid sequences set forth as SEQ ID NOs:6-826. Any Cas9 protein as defined above can be used as a Cas9 polypeptide, or as part of a chimeric Cas9 polypeptide, in a complex of the present disclosure.

In some cases, a suitable variant Cas9 polypeptide comprises an amino acid sequence having 100% amino acid sequence identity to amino acids 7-166 or 731-1003 of the Cas9 amino acid sequence depicted in FIG. 6A (SEQ ID NO:5), or to the corresponding portions in any of the amino acid sequences set forth as SEQ ID NOs:6-826. Any Cas9 protein as defined above can be used as a Cas9 polypeptide, or as part of a chimeric Cas9 polypeptide, in a complex of the present disclosure.

In some cases, a suitable variant Cas9 polypeptide comprises an amino acid sequence having 60% or more, 70% or more, 75% or more, 80% or more, 85% or more, 90% or more, 95% or more, 99% or more, or 100% amino acid sequence identity to the Cas9 amino acid sequence depicted in FIG. 6A (SEQ ID NO:5), or to any of the amino acid sequences set forth as SEQ ID NOs:6-826. Any Cas9 protein as defined above can be used as a variant Cas9 polypeptide or as part of a chimeric variant Cas9 polypeptide in a complex of the present disclosure.

In some cases, a suitable variant Cas9 polypeptide comprises an amino acid sequence having 60% or more amino acid sequence identity to the Cas9 amino acid sequence depicted in FIG. 6A (SEQ ID NO:5), or to any of the amino acid sequences set forth as SEQ ID NOs:6-826. Any Cas9 protein as defined above can be used as a variant Cas9 polypeptide or as part of a chimeric variant Cas9 polypeptide in a complex of the present disclosure.

In some cases, a suitable variant Cas9 polypeptide comprises an amino acid sequence having 70% or more amino acid sequence identity to the Cas9 amino acid sequence depicted in FIG. 6A (SEQ ID NO:5), or to any of the amino acid sequences set forth as SEQ ID NOs:6-826. Any Cas9 protein as defined above can be used as a variant Cas9 polypeptide or as part of a chimeric variant Cas9 polypeptide in a complex of the present disclosure.

In some cases, a suitable variant Cas9 polypeptide comprises an amino acid sequence having 75% or more amino acid sequence identity to the Cas9 amino acid sequence depicted in FIG. 6A (SEQ ID NO:5), or to any of the amino acid sequences set forth as SEQ ID NOs:6-826. Any Cas9 protein as defined above can be used as a variant Cas9 polypeptide or as part of a chimeric variant Cas9 polypeptide in a complex of the present disclosure.

In some cases, a suitable variant Cas9 polypeptide comprises an amino acid sequence having 80% or more amino acid sequence identity to the Cas9 amino acid sequence depicted in FIG. 6A (SEQ ID NO:5), or to any of the amino acid sequences set forth as SEQ ID NOs:6-826. Any Cas9 protein as defined above can be used as a variant Cas9 polypeptide or as part of a chimeric variant Cas9 polypeptide in a complex of the present disclosure.

In some cases, a suitable variant Cas9 polypeptide comprises an amino acid sequence having 85% or more amino acid sequence identity to the Cas9 amino acid sequence depicted in FIG. 6A (SEQ ID NO:5), or to any of the amino acid sequences set forth as SEQ ID NOs:6-826. Any Cas9 protein as defined above can be used as a variant Cas9 polypeptide or as part of a chimeric variant Cas9 polypeptide in a complex of the present disclosure.

In some cases, a suitable variant Cas9 polypeptide comprises an amino acid sequence having 90% or more amino acid sequence identity to the Cas9 amino acid sequence depicted in FIG. 6A (SEQ ID NO:5), or to any of the amino acid sequences set forth as SEQ ID NOs:6-826. Any Cas9 protein as defined above can be used as a variant Cas9 polypeptide or as part of a chimeric variant Cas9 polypeptide in a complex of the present disclosure.

In some cases, a suitable variant Cas9 polypeptide comprises an amino acid sequence having 95% or more amino acid sequence identity to the Cas9 amino acid sequence depicted in FIG. 6A (SEQ ID NO:5), or to any of the amino acid sequences set forth as SEQ ID NOs:6-826. Any Cas9 protein as defined above can be used as a variant Cas9 polypeptide or as part of a chimeric variant Cas9 polypeptide in a complex of the present disclosure.

In some cases, a suitable variant Cas9 polypeptide comprises an amino acid sequence having 99% or more amino acid sequence identity to the Cas9 amino acid sequence depicted in FIG. 6A (SEQ ID NO:5), or to any of the amino acid sequences set forth as SEQ ID NOs:6-826. Any Cas9 protein as defined above can be used as a variant Cas9 polypeptide or as part of a chimeric variant Cas9 polypeptide in a complex of the present disclosure.

In some cases, a suitable variant Cas9 polypeptide comprises an amino acid sequence having 100% amino acid sequence identity to the Cas9 amino acid sequence depicted in FIG. 6A (SEQ ID NO:5), or to any of the amino acid sequences set forth as SEQ ID NOs:6-826. Any Cas9 protein as defined above can be used as a variant Cas9 polypeptide or as part of a chimeric variant Cas9 polypeptide in a complex of the present disclosure.

### Chimeric polypeptides (fusion polypeptides)

In some embodiments, a variant Cas9 polypeptide is a chimeric Cas9 polypeptide (also referred to herein as a fusion polypeptide, e.g., a "Cas9 fusion polypeptide"). A Cas9 fusion polypeptide can bind and/or modify a target nucleic acid (e.g., cleave, methylate, demethylate, etc.) and/or a polypeptide associated with target nucleic acid (e.g., methylation, acetylation, etc., of, for example, a histone tail).

A Cas9 fusion polypeptide is a variant Cas9 polypeptide by virtue of differing in sequence from a wild type Cas9 polypeptide (e.g., a naturally occurring Cas9 polypeptide). A Cas9 fusion polypeptide is a Cas9 polypeptide (e.g., a wild type Cas9 polypeptide, a variant Cas9 polypeptide, a variant Cas9 polypeptide with reduced nuclease activity (as described above), and the like) fused to a covalently linked heterologous polypeptide (also referred to as a "fusion partner"). In some cases, a Cas9 fusion polypeptide is a variant Cas9 polypeptide with reduced nuclease activity (e.g., dCas9) fused to a covalently linked heterologous polypeptide. In some cases, the heterologous polypeptide exhibits (and therefore provides for) an activity (e.g., an enzymatic activity) that will also be exhibited by the Cas9 fusion polypeptide (e.g., methyltransferase activity, acetyltransferase activity, kinase activity, ubiquitinating activity, etc.). In some such cases, a method of binding, e.g., where the Cas9 polypeptide is a variant Cas9 polypeptide having a fusion partner (i.e., having a heterologous polypeptide) with an activity (e.g., an enzymatic activity) that modifies the target nucleic acid, the method can also be considered to be a method of modifying the target nucleic acid. In some cases, a method of binding a target nucleic acid (e.g., a single stranded target nucleic acid) can result in modification of the target nucleic acid. Thus, in some cases, a method of binding a target nucleic acid (e.g., a single stranded target nucleic acid) can be a method of modifying the target nucleic acid.

In some cases, the heterologous sequence provides for subcellular localization, i.e., the heterologous sequence is a subcellular localization sequence (e.g., a nuclear localization signal (NLS) for targeting to the nucleus, a sequence to keep the fusion protein out of the nucleus, e.g., a nuclear export sequence (NES), a sequence to keep the fusion protein retained in the cytoplasm, a mitochondrial localization signal for targeting to the mitochondria, a chloroplast localization signal for targeting to a chloroplast, an endoplasmic reticulum (ER) retention signal, and the like). In some embodiments, a variant Cas9 does not include a NLS so that the protein is not targeted to the nucleus (which can be advantageous, e.g., when the target nucleic acid is an RNA that is present in the cytosol). In some embodiments, the heterologous sequence can provide a tag (i.e., the heterologous sequence is a detectable label) for ease of tracking and/or purification (e.g., a fluorescent protein, e.g., green fluorescent protein (GFP), YFP, RFP, CFP, mCherry, tdTomato, and the like; a histidine tag, e.g., a 6XHis tag; a hemagglutinin (HA) tag; a FLAG tag; a Myc tag; and the like). In some embodiments, the heterologous sequence can provide for increased or decreased stability (i.e., the heterologous sequence is a stability control peptide, e.g., a degron, which in some cases is controllable (e.g., a temperature sensitive or drug controllable degron sequence, see below). In some embodiments, the heterologous sequence can provide for increased or decreased transcription from the target nucleic acid (i.e., the heterologous sequence is a transcription modulation sequence, e.g., a transcription factor/activator or a fragment thereof, a protein or fragment thereof that recruits a transcription factor/activator, a transcription repressor or a fragment thereof, a protein or fragment thereof that recruits a transcription repressor, a small molecule/drug-responsive transcription regulator, etc.). In some embodiments, the heterologous sequence can provide a binding domain (i.e., the heterologous sequence is a protein binding sequence, e.g., to provide the ability of a Cas9 fusion polypeptide to bind to another protein of interest, e.g., a DNA or histone modifying protein, a transcription factor or transcription repressor, a recruiting protein, an RNA modification enzyme, an RNA-binding protein, a translation initiation factor, an RNA splicing factor, etc.). A heterologous nucleic acid sequence may be linked to another nucleic acid sequence (e.g., by genetic engineering) to generate a chimeric nucleotide sequence encoding a chimeric polypeptide.

A subject Cas9 fusion polypeptide (Cas9 fusion protein) can have multiple (1 or more, 2 or more, 3 or more, etc.) fusion partners in any combination of the above. As an illustrative example, a Cas9 fusion protein can have a heterologous sequence that provides an activity (e.g., for transcription modulation, target modification, modification of a protein associated with a target nucleic acid, etc.) and can also have a subcellular localization sequence. In some cases, such a Cas9 fusion protein might also have a tag for ease of tracking and/or purification (e.g., green fluorescent protein (GFP), YFP, RFP, CFP, mCherry, tdTomato, and the like; a histidine tag, e.g., a 6XHis tag; a hemagglutinin (HA) tag; a FLAG tag; a Myc tag; and the like). As another illustrative example, a Cas9 protein can have one or more NLSs (e.g., two or more, three or more, four or more, five or more, 1, 2, 3, 4, or 5 NLSs). In some cases a fusion partner (or multiple fusion partners) (e.g., an NLS, a tag, a fusion partner providing an activity, etc.) is located at or near the C-terminus of Cas9. In some cases a fusion partner (or multiple fusion partners) (e.g., an NLS, a tag, a fusion partner providing an activity, etc.) is located at the N-terminus of Cas9. In some cases a Cas9 has a fusion partner (or multiple fusion partners)(e.g., an NLS, a tag, a fusion partner providing an activity, etc.) at both the N-terminus and C-terminus.

Suitable fusion partners that provide for increased or decreased stability include, but are not limited to degron sequences. Degrons are readily understood by one of ordinary skill in the art to be amino acid sequences that control the stability of the protein of which they are part. For example, the stability of a protein comprising a degron sequence is controlled in part by the degron sequence. In some cases, a suitable degron is constitutive such that the degron exerts its influence on protein stability independent of experimental control (i.e., the degron is not drug inducible, temperature inducible, etc.) In some cases, the degron provides the variant Cas9 polypeptide with controllable stability such that the variant Cas9 polypeptide can be turned "on" (i.e., stable) or "off' (i.e., unstable, degraded) depending on the desired conditions. For example, if the degron is a temperature sensitive degron, the variant Cas9 polypeptide may be functional (i.e., "on", stable) below a threshold temperature (e.g., 42°C, 41°C, 40°C, 39°C, 38°C, 37°C, 36°C, 35°C, 34°C, 33°C, 32°C, 31°C, 30°C, etc.) but non-functional (i.e., "off', degraded) above the threshold temperature. As another example, if the degron is a drug inducible degron, the presence or absence of drug can switch the protein from an "off' (i.e., unstable) state to an "on" (i.e., stable) state or vice versa. An exemplary drug inducible degron is derived from the FKBP12 protein. The stability of the degron is controlled by the presence or absence of a small molecule that binds to the degron.

Examples of suitable degrons include, but are not limited to those degrons controlled by Shield-1, DHFR, auxins, and/or temperature. Non-limiting examples of suitable degrons are known in the art (e.g., Dohmen et al., Science, 1994. 263(5151): p. 1273-1276: Heat-inducible degron: a method for constructing temperature-sensitive mutants; Schoeber et al., Am J Physiol Renal Physiol. 2009 Jan;296(1):F204-11 : Conditional fast expression and function of multimeric TRPV5 channels using Shield-1 ; Chu et al., Bioorg Med Chem Lett. 2008 Nov 15;18(22):5941-4: Recent progress with FKBP-derived destabilizing domains ; Kanemaki, Pflugers Arch. 2012 Dec 28: Frontiers of protein expression control with conditional degrons; Yang et al., Mol Cell. 2012 Nov 30;48(4):487-8: Titivated for destruction: the methyl degron; Barbour et al., Biosci Rep. 2013 Jan 18;33(1).: Characterization of the bipartite degron that regulates ubiquitin-independent degradation of thymidylate synthase; and Greussing et al., J Vis Exp. 2012 Nov 10;(69): Monitoring of ubiquitin-proteasome activity in living cells using a Degron (dgn)-destabilized green fluorescent protein (GFP)-based reporter protein).

Exemplary degron sequences have been well-characterized and tested in both cells and animals. Thus, fusing Cas9 (e.g., wild type Cas9; variant Cas9; variant Cas9 with reduced nuclease activity, e.g., dCas9; and the like) to a degron sequence produces a "tunable" and "inducible" Cas9 polypeptide. Any of the fusion partners described herein can be used in any desirable combination. As one non-limiting example to illustrate this point, a Cas9 fusion protein (i.e., a chimeric Cas9 polypeptide) can comprise a YFP sequence for detection, a degron sequence for stability, and transcription activator sequence to increase transcription of the target nucleic acid. A suitable reporter protein for use as a fusion partner for a Cas9 polypeptide (e.g., wild type Cas9, variant Cas9, variant Cas9 with reduced nuclease function, etc.), includes, but is not limited to, the following exemplary proteins (or functional fragment thereof): his3, β-galactosidase, a fluorescent protein (e.g., GFP, RFP, YFP, cherry, tomato, etc., and various derivatives thereof), luciferase, β- glucuronidase, and alkaline phosphatase. Furthermore, the number of fusion partners that can be used in a Cas9 fusion protein is unlimited. In some cases, a Cas9 fusion protein comprises one or more (e.g. two or more, three or more, four or more, or five or more) heterologous sequences.

Suitable fusion partners include, but are not limited to, a polypeptide that provides for methyltransferase activity, demethylase activity, acetyltransferase activity, deacetylase activity, kinase activity, phosphatase activity, ubiquitin ligase activity, deubiquitinating activity, adenylation activity, deadenylation activity, SUMOylating activity, deSUMOylating activity, ribosylation activity, deribosylation activity, myristoylation activity, or demyristoylation activity, any of which can be directed at modifying nucleic acid directly (e.g., methylation of DNA or RNA) or at modifying a nucleic acid-associated polypeptide (e.g., a histone, a DNA binding protein, and RNA binding protein, and the like). Further suitable fusion partners include, but are not limited to boundary elements (e.g., CTCF), proteins and fragments thereof that provide periphery recruitment (e.g., Lamin A, Lamin B, etc.), and protein docking elements (e.g., FKBP/FRB, Pil1/Aby1, etc.).

Examples of various additional suitable fusion partners (or fragments thereof) for a subject variant Cas9 polypeptide include, but are not limited to those listed in **FIGs. 8A-C** and are also described in the PCT patent applications: WO2010/075303, WO2012/068627, and WO2013/155555.

Suitable fusion partners include, but are not limited to, a polypeptide that provides an activity that indirectly increases transcription by acting directly on the target nucleic acid or on a polypeptide (e.g., a histone, a DNA-binding protein, an RNA-binding protein, an RNA editing protein, etc.) associated with the target nucleic acid. Suitable fusion partners include, but are not limited to, a polypeptide that provides for methyltransferase activity, demethylase activity, acetyltransferase activity, deacetylase activity, kinase activity, phosphatase activity, ubiquitin ligase activity, deubiquitinating activity, adenylation activity, deadenylation activity, SUMOylating activity, deSUMOylating activity, ribosylation activity, deribosylation activity, myristoylation activity, or demyristoylation activity.

Additional suitable fusion partners include, but are not limited to, a polypeptide that directly provides for increased transcription and/or translation of a target nucleic acid (e.g., a transcription activator or a fragment thereof, a protein or fragment thereof that recruits a transcription activator, a small molecule/drug-responsive transcription and/or translation regulator, a translation-regulating protein, etc.).

Non-limiting examples of fusion partners to accomplish increased or decreased transcription are listed in Fig. 8A-8C and include transcription activator and transcription repressor domains (e.g., the Krüppel associated box (KRAB or SKD); the Mad mSIN3 interaction domain (SID); the ERF repressor domain (ERD), etc.). In some such cases, a Cas9 fusion protein is targeted by the guide nucleic acid to a specific location (i.e., sequence) in the target nucleic acid and exerts locus-specific regulation such as blocking RNA polymerase binding to a promoter (which selectively inhibits transcription activator function), and/or modifying the local chromatin status (e.g., when a fusion sequence is used that modifies the target nucleic acid or modifies a polypeptide associated with the target nucleic acid). In some cases, the changes are transient (e.g., transcription repression or activation). In some cases, the changes are inheritable (e.g., when epigenetic modifications are made to the target nucleic acid or to proteins associated with the target nucleic acid, e.g., nucleosomal histones).

Non-limiting examples of fusion partners for use when targeting ssRNA target nucleic acids are listed in **FIG. 8A** and include (but are not limited to): splicing factors (e.g., RS domains); protein translation components (e.g., translation initiation, elongation, and/or release factors; e.g., eIF4G); RNA methylases; RNA editing enzymes (e.g., RNA deaminases, e.g., adenosine deaminase acting on RNA (ADAR), including A to I and/or C to U editing enzymes); helicases; RNA-binding proteins; and the like. It is understood that a fusion partner can include the entire protein or in some cases can include a fragment of the protein (e.g., a functional domain).

In some embodiments, the heterologous sequence can be fused to the C-terminus of the Cas9 polypeptide. In some embodiments, the heterologous sequence can be fused to the N-terminus of the Cas9 polypeptide. In some embodiments, the heterologous sequence can be fused to an internal portion (i.e., a portion other than the N- or C-terminus) of the Cas9 polypeptide.

In addition to the fusion partners listed in **FIGs. 8A-8C** the fusion partner of a chimeric Cas9 polypeptide can be any domain capable of interacting with ssRNA (which, for the purposes of this disclosure, includes intramolecular and/or intermolecular secondary structures, e.g., double-stranded RNA duplexes such as hairpins, stem-loops, etc.), whether transiently or irreversibly, directly or indirectly, including but not limited to an effector domain selected from the group comprising; Endonucleases (for example RNase III, the CRR22 DYW domain, Dicer, and PIN (PilT N-terminus) domains from proteins such as SMG5 and SMG6); proteins and protein domains responsible for stimulating RNA cleavage (for example CPSF, CstF, CFIm and CFIIm); Exonucleases (for example XRN-1 or Exonuclease T) ; Deadenylases (for example HNT3); proteins and protein domains responsible for nonsense mediated RNA decay (for example UPF1, UPF2, UPF3, UPF3b, RNP S1, Y14, DEK, REF2, and SRm160); proteins and protein domains responsible for stabilizing RNA (for example PABP) ; proteins and protein domains responsible for repressing translation (for example Ago2 and Ago4); proteins and protein domains responsible for stimulating translation (for example Staufen); proteins and protein domains responsible for (e.g., capable of) modulating translation (e.g., translation factors such as initiation factors, elongation factors, release factors, etc., e.g., eIF4G); proteins and protein domains responsible for polyadenylation of RNA (for example PAP1, GLD-2, and Star- PAP) ; proteins and protein domains responsible for polyuridinylation of RNA (for example CI D1 and terminal uridylate transferase) ; proteins and protein domains responsible for RNA localization (for example from IMP1, ZBP1, She2p, She3p, and Bicaudal-D); proteins and protein domains responsible for nuclear retention of RNA (for example Rrp6); proteins and protein domains responsible for nuclear export of RNA (for example TAP, NXF1, THO, TREX, REF, and Aly); proteins and protein domains responsible for repression of RNA splicing (for example PTB, Sam68, and hnRNP A1) ; proteins and protein domains responsible for stimulation of RNA splicing (for example Serine/Arginine-rich (SR) domains) ; proteins and protein domains responsible for reducing the efficiency of transcription (for example FUS (TLS)); and proteins and protein domains responsible for stimulating transcription (for example CDK7 and HIV Tat). Alternatively, the effector domain may be selected from the group comprising Endonucleases; proteins and protein domains capable of stimulating RNA cleavage; Exonucleases; Deadenylases; proteins and protein domains having nonsense mediated RNA decay activity; proteins and protein domains capable of stabilizing RNA; proteins and protein domains capable of repressing translation; proteins and protein domains capable of stimulating translation; proteins and protein domains capable of modulating translation (e.g., translation factors such as initiation factors, elongation factors, release factors, etc., e.g., eIF4G); proteins and protein domains capable of polyadenylation of RNA; proteins and protein domains capable of polyuridinylation of RNA; proteins and protein domains having RNA localization activity; proteins and protein domains capable of nuclear retention of RNA; proteins and protein domains having RNA nuclear export activity; proteins and protein domains capable of repression of RNA splicing; proteins and protein domains capable of stimulation of RNA splicing; proteins and protein domains capable of reducing the efficiency of transcription ; and proteins and protein domains capable of stimulating transcription. Another suitable fusion partner is a PUF RNA-binding domain, which is described in more detail in WO2012068627.

Some RNA splicing factors that can be used (in whole or as fragments thereof) as fusion partners for a Cas9 polypeptide have modular organization, with separate sequence-specific RNA binding modules and splicing effector domains. For example, members of the Serine/ Arginine-rich (SR) protein family contain N-terminal RNA recognition motifs (RRMs) that bind to exonic splicing enhancers (ESEs) in pre-mRNAs and C-terminal RS domains that promote exon inclusion. As another example, the hnRNP protein hnRNP Al binds to exonic splicing silencers (ESSs) through its RRM domains and inhibits exon inclusion through a C-terminal Glycine-rich domain. Some splicing factors can regulate alternative use of splice site (ss) by binding to regulatory sequences between the two alternative sites. For example, ASF/SF2 can recognize ESEs and promote the use of intron proximal sites, whereas hnRNP Al can bind to ESSs and shift splicing towards the use of intron distal sites. One application for such factors is to generate ESFs that modulate alternative splicing of endogenous genes, particularly disease associated genes. For example, Bcl-x pre-mRNA produces two splicing isoforms with two alternative 5' splice sites to encode proteins of opposite functions. The long splicing isoform Bcl-xL is a potent apoptosis inhibitor expressed in long-lived postmitotic cells and is up-regulated in many cancer cells, protecting cells against apoptotic signals. The short isoform Bcl-xS is a pro-apoptotic isoform and expressed at high levels in cells with a high turnover rate (e.g., developing lymphocytes). The ratio of the two Bcl-x splicing isoforms is regulated by multiple cώ-elements that are located in either the core exon region or the exon extension region (i.e., between the two alternative 5' splice sites). For more examples, see WO2010075303.

In some embodiments, a Cas9 polypeptide (e.g., a wild type Cas9, a variant Cas9, a variant Cas9 with reduced nuclease activity, etc.) can be linked to a fusion partner via a peptide spacer.

In some cases, a Cas9 polypeptide comprises a "Protein Transduction Domain" or PTD (also known as a CPP - cell penetrating peptide), which may refer to a polypeptide, polynucleotide, carbohydrate, or organic or inorganic compound that facilitates traversing a lipid bilayer, micelle, cell membrane, organelle membrane, or vesicle membrane. A PTD attached to another molecule, which can range from a small polar molecule to a large macromolecule and/or a nanoparticle, facilitates the molecule traversing a membrane, for example going from extracellular space to intracellular space, or cytosol to within an organelle. In some cases, a PTD attached to another molecule facilitates entry of the molecule into the nucleus (e.g., in some cases, a PTD includes a nuclear localization signal (NLS)). In some cases, a Cas9 polypeptide comprises two or more NLSs, e.g., two or more NLSs in tandem. In some cases, a PTD is covalently linked to the amino terminus of a Cas9 polypeptide. In some cases, a PTD is covalently linked to the carboxyl terminus of a Cas9 polypeptide. In some cases, a PTD is covalently linked to the amino terminus and to the carboxyl terminus of a Cas9 polypeptide. In some cases, a PTD is covalently linked to a nucleic acid (e.g., a guide nucleic acid, a polynucleotide encoding a guide nucleic acid, a polynucleotide encoding a Cas9 polypeptide, etc.). Exemplary PTDs include but are not limited to a minimal undecapeptide protein transduction domain (corresponding to residues 47-57 of HIV-1 TAT comprising YGRKKRRQRRR; SEQ ID NO:1086); a polyarginine sequence comprising a number of arginines sufficient to direct entry into a cell (e.g., 3, 4, 5, 6, 7, 8, 9, 10, or 10-50 arginines); a VP22 domain (Zender et al. (2002) Cancer Gene Ther. 9(6):489-96); an Drosophila Antennapedia protein transduction domain (Noguchi et al. (2003) Diabetes 52(7):1732-1737); a truncated human calcitonin peptide (Trehin et al. (2004) Pharm. Research 21:1248-1256); polylysine (Wender et al. (2000) Proc. Natl. Acad. Sci. USA 97:13003-13008); RRQRRTSKLMKR (SEQ ID NO:1087); Transportan GWTLNSAGYLLGKINLKALAALAKKIL (SEQ ID NO:1088); KALAWEAKLAKALAKALAKHLAKALAKALKCEA (SEQ ID NO:1089); and RQIKIWFQNRRMKWKK (SEQ ID NO:1090). Exemplary PTDs include but are not limited to, YGRKKRRQRRR (SEQ ID NO:1091), RKKRRQRRR (SEQ ID NO:1092); an arginine homopolymer of from 3 arginine residues to 50 arginine residues; Exemplary PTD domain amino acid sequences include, but are not limited to, any of the following: YGRKKRRQRRR (SEQ ID NO:1093); RKKRRQRR (SEQ ID NO:1094); YARAAARQARA (SEQ ID NO:1095); THRLPRRRRRR (SEQ ID NO:1096); and GGRRARRRRRR (SEQ ID NO:1097). In some embodiments, the PTD is an activatable CPP (ACPP) (Aguilera et al. (2009) Integr Biol (Camb) June; 1(5-6): 371-381). ACPPs comprise a polycationic CPP (e.g., Arg9 or "R9") connected via a cleavable linker to a matching polyanion (e.g., Glu9 or "E9"), which reduces the net charge to nearly zero and thereby inhibits adhesion and uptake into cells. Upon cleavage of the linker, the polyanion is released, locally unmasking the polyarginine and its inherent adhesiveness, thus "activating" the ACPP to traverse the membrane.

### Type V CRISPR site-directed modifying polypeptides

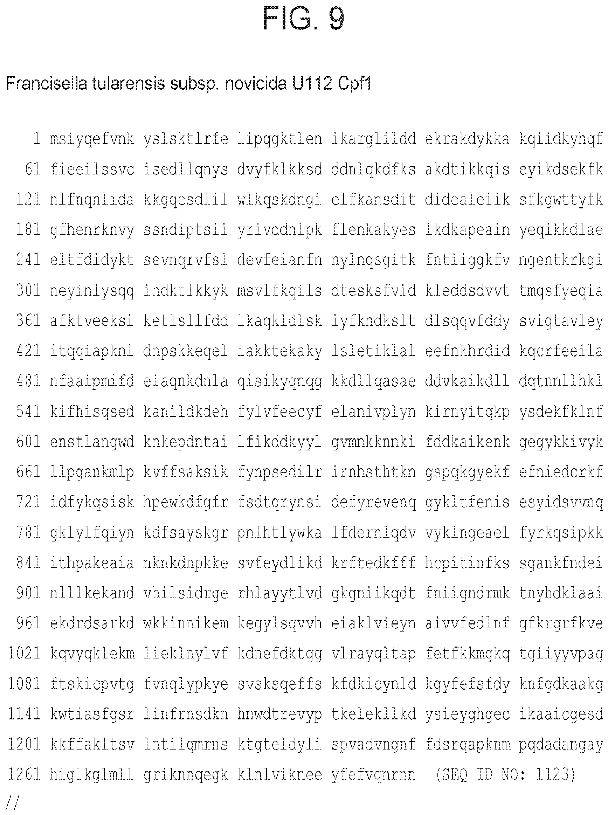

In some cases, a complex of the present disclosure comprises a Type V CRISPR site-directed modifying polypeptide. A Type V CRISPR site-directed modifying polypeptide is also referred to herein as a "Cpfl polypeptide." In some cases, the Cpfl polypeptide is enzymatically active, e.g., the Cpfl polypeptide, when bound to a guide RNA, cleaves a target nucleic acid. In some cases, the Cpfl polypeptide exhibits reduced enzymatic activity relative to a wild-type Cpfl polypeptide (e.g., relative to a Cpfl polypeptide comprising the amino acid sequence depicted in FIG. 9), and retains DNA binding activity.

In some cases, a Cpfl polypeptide comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the amino acid sequence depicted in FIG. 9. In some cases, a Cpfl polypeptide comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to a contiguous stretch of from 100 amino acids to 200 amino acids (aa), from 200 aa to 400 aa, from 400 aa to 600 aa, from 600 aa to 800 aa, from 800 aa to 1000 aa, from 1000 aa to 1100 aa, from 1100 aa to 1200 aa, or from 1200 aa to 1300 aa, of the amino acid sequence depicted in FIG. 9.

In some cases, a Cpfl polypeptide comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the RuvCI domain of a Cpfl polypeptide of the amino acid sequence depicted in FIG. 9. In some cases, a Cpfl polypeptide comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the RuvCII domain of a Cpfl polypeptide of the amino acid sequence depicted in FIG. 9. In some cases, a Cpfl polypeptide comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the RuvCIII domain of a Cpfl polypeptide of the amino acid sequence depicted in FIG. 9.

In some cases, the Cpfl polypeptide exhibits reduced enzymatic activity relative to a wild-type Cpfl polypeptide (e.g., relative to a Cpfl polypeptide comprising the amino acid sequence depicted in FIG. 9), and retains DNA binding activity. In some cases, a Cpfl polypeptide comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the amino acid sequence depicted in FIG. 9; and comprises an amino acid substitution (e.g., a D→A substitution) at an amino acid residue corresponding to amino acid 917 of the amino acid sequence depicted in FIG. 9. In some cases, a Cpfl polypeptide comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the amino acid sequence depicted in FIG. 9; and comprises an amino acid substitution (e.g., an E→A substitution) at an amino acid residue corresponding to amino acid 1006 of the amino acid sequence depicted in FIG. 9. In some cases, a Cpfl polypeptide comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the amino acid sequence depicted in FIG. 9; and comprises an amino acid substitution (e.g., a D→A substitution) at an amino acid residue corresponding to amino acid 1255 of the amino acid sequence depicted in FIG. 9.

In some cases, the Cpfl polypeptide is a fusion polypeptide, e.g., where a Cpfl fusion polypeptide comprises: a) a Cpfl polypeptide; and b) a heterologous fusion partner. In some cases, the heterologous fusion partner is fused to the N-terminus of the Cpfl polypeptide. In some cases, the heterologous fusion partner is fused to the C-terminus of the Cpfl polypeptide. In some cases, the heterologous fusion partner is fused to both the N-terminus and the C-terminus of the Cpfl polypeptide. In some cases, the heterologous fusion partner is inserted internally within the Cpfl polypeptide.

Suitable heterologous fusion partners include NLS, epitope tags, fluorescent polypeptides, and the like.

### Guide RNA

Guide RNAs suitable for inclusion in a complex of the present disclosure include single-molecule guide RNAs ("single-guide RNA" / "sgRNA") and dual-molecule guide RNAs ("dual-guide RNA" / "dgRNA").

A guide nucleic acid (e.g., guide RNA) suitable for inclusion in a complex of the present disclosure directs the activities of a polypeptide (e.g., a Cas9 polypeptide) to a specific target sequence within a target nucleic acid. A guide nucleic acid (e.g., guide RNA) comprises: a first segment (also referred to herein as a "nucleic acid targeting segment", or simply a "targeting segment"); and a second segment (also referred to herein as a "protein-binding segment").

### First segment: targeting segment

The first segment of a guide nucleic acid (e.g., guide RNA) includes a nucleotide sequence that is complementary to a sequence (a target site) in a target nucleic acid. In other words, the targeting segment of a guide nucleic acid (e.g., guide RNA) can interact with a target nucleic acid (e.g., an RNA, a DNA, a double-stranded DNA) in a sequence-specific manner via hybridization (i.e., base pairing). As such, the nucleotide sequence of the targeting segment may vary and can determine the location within the target nucleic acid that the guide nucleic acid (e.g., guide RNA) and the target nucleic acid will interact. The targeting segment of a guide nucleic acid (e.g., guide RNA) can be modified (e.g., by genetic engineering) to hybridize to any desired sequence (target site) within a target nucleic acid.

The targeting segment can have a length of from 12 nucleotides to 100 nucleotides. For example, the targeting segment can have a length of from 12 nucleotides (nt) to 80 nt, from 12 nt to 50nt, from 12 nt to 40 nt, from 12 nt to 30 nt, from 12 nt to 25 nt, from 12 nt to 20 nt, or from 12 nt to 19 nt. For example, the targeting segment can have a length of from 17 nt to 20 nt, from 17 nt to 25 nt, from 17 nt to 30 nt, from 17 nt to 35 nt, from 17 nt to 40 nt, from 17 nt to 45 nt, from 17 nt to 50 nt, from 17 nt to 60 nt, from 17 nt to 70 nt, from 17 nt to 80 nt, from 17 nt to 90 nt, 18 nt to 20 nt, from 18 nt to 25 nt, from 18 nt to 30 nt, from 18 nt to 35 nt, from 18 nt to 40 nt, from 18 nt to 45 nt, from 18 nt to 50 nt, from 18 nt to 60 nt, from 18 nt to 70 nt, from 18 nt to 80 nt, from 18 nt to 90 nt, 19 nt to 20 nt, from 19 nt to 25 nt, from 19 nt to 30 nt, from 19 nt to 35 nt, from 19 nt to 40 nt, from 19 nt to 45 nt, from 19 nt to 50 nt, from 19 nt to 60 nt, from 19 nt to 70 nt, from 19 nt to 80 nt, from 19 nt to 90 nt, from 19 nt to 100 nt, from 20 nt to 25 nt, from 20 nt to 30 nt, from 20 nt to 35 nt, from 20 nt to 40 nt, from 20 nt to 45 nt, from 20 nt to 50 nt, from 20 nt to 60 nt, from 20 nt to 70 nt, from 20 nt to 80 nt, from 20 nt to 90 nt, or from 20 nt to 100 nt.

The nucleotide sequence (the targeting sequence, also referred to as a guide sequence) of the targeting segment that is complementary to a nucleotide sequence (target site) of the target nucleic acid can have a length of 12 nt or more. For example, the targeting sequence of the targeting segment that is complementary to a target site of the target nucleic acid can have a length of 12 nt or more, 15 nt or more, 17 nt or more, 18 nt or more, 19 nt or more, 20 nt or more, 25 nt or more, 30 nt or more, 35 nt or more or 40 nt. For example, the targeting sequence of the targeting segment that is complementary to a target sequence of the target nucleic acid can have a length of from 12 nucleotides (nt) to 80 nt, from 12 nt to 50nt, from 12 nt to 45 nt, from 12 nt to 40 nt, from 12 nt to 35 nt, from 12 nt to 30 nt, from 12 nt to 25 nt, from 12 nt to 20 nt, from 12 nt to 19 nt, from 17 nt to 20 nt, from 17 nt to 25 nt, from 17 nt to 30 nt, from 17 nt to 35 nt, from 17 nt to 40 nt, from 17 nt to 45 nt, from 17 nt to 50 nt, from 17 nt to 60 nt, from 18 nt to 20 nt, from 18 nt to 25 nt, from 18 nt to 30 nt, from 18 nt to 35 nt, from 18 nt to 40 nt, from 18 nt to 45 nt, from 18 nt to 50 nt, from 18 nt to 60 nt, from 19 nt to 20 nt, from 19 nt to 25 nt, from 19 nt to 30 nt, from 19 nt to 35 nt, from 19 nt to 40 nt, from 19 nt to 45 nt, from 19 nt to 50 nt, from 19 nt to 60 nt, from 20 nt to 25 nt, from 20 nt to 30 nt, from 20 nt to 35 nt, from 20 nt to 40 nt, from 20 nt to 45 nt, from 20 nt to 50 nt, or from 20 nt to 60 nt. The nucleotide sequence (the targeting sequence) of the targeting segment that is complementary to a nucleotide sequence (target site) of the target nucleic acid can have a length of 12 nt or more.

In some cases, the targeting sequence of the targeting segment that is complementary to a target site of the target nucleic acid is 20 nucleotides in length. In some cases, the targeting sequence of the targeting segment that is complementary to a target site of the target nucleic acid is 19 nucleotides in length. In some cases, the targeting sequence of the targeting segment that is complementary to a target site of the target nucleic acid is 18 nucleotides in length. In some cases, the targeting sequence of the targeting segment that is complementary to a target site of the target nucleic acid is 17 nucleotides in length.

The percent complementarity between the targeting sequence (i.e., guide sequence) of the targeting segment and the target site of the target nucleic acid can be 60% or more (e.g., 65% or more, 70% or more, 75% or more, 80% or more, 85% or more, 90% or more, 95% or more, 97% or more, 98% or more, 99% or more, or 100%). In some cases, the percent complementarity between the targeting sequence of the targeting segment and the target site of the target nucleic acid is 100% over the seven contiguous 5'-most nucleotides of the target site of the target nucleic acid. In some cases, the percent complementarity between the targeting sequence of the targeting segment and the target site of the target nucleic acid is 60% or more over 20 contiguous nucleotides. In some cases, the percent complementarity between the targeting sequence of the targeting segment and the target site of the target nucleic acid is 100% over the seventeen contiguous 5'-most nucleotides of the target site of the target nucleic acid and as low as 0% or more over the remainder. In such a case, the targeting sequence can be considered to be 17 nucleotides in length. In some cases, the percent complementarity between the targeting sequence of the targeting segment and the target site of the target nucleic acid is 100% over the eighteen contiguous 5'-most nucleotides of the target site of the target nucleic acid and as low as 0% or more over the remainder. In such a case, the targeting sequence can be considered to be 18 nucleotides in length. In some cases, the percent complementarity between the targeting sequence of the targeting segment and the target site of the target nucleic acid is 100% over the nineteen contiguous 5'-most nucleotides of the target site of the target nucleic acid and as low as 0% or more over the remainder. In such a case, the targeting sequence can be considered to be 19 nucleotides in length. In some cases, the percent complementarity between the targeting sequence of the targeting segment and the target site of the target nucleic acid is 100% over the twenty contiguous 5'-most nucleotides of the target site of the target nucleic acid and as low as 0% or more over the remainder. In such a case, the targeting sequence can be considered to be 20 nucleotides in length.

### Second segment: protein-binding segment

The protein-binding segment of a subject guide nucleic acid (e.g., guide RNA) interacts with (binds) a Cas9 polypeptide. The subject guide nucleic acid (e.g., guide RNA) guides the bound polypeptide (Cas9) to a specific nucleotide sequence within target nucleic acid (the target site) via the above mentioned targeting segment/targeting sequence/guide sequence. The protein-binding segment of a subject guide nucleic acid (e.g., guide RNA) comprises two stretches of nucleotides that are complementary to one another. The complementary nucleotides of the protein-binding segment hybridize to form a double stranded RNA duplex (dsRNA).

A subject dual guide nucleic acid (e.g., guide RNA) comprises two separate nucleic acid molecules. Each of the two molecules of a subject dual guide nucleic acid (e.g., guide RNA) comprises a stretch of nucleotides that are complementary to one another such that the complementary nucleotides of the two molecules hybridize to form the double stranded RNA duplex of the protein-binding segment.

In some embodiments, the duplex-forming segment of the activator is 60% or more identical to one of the activator (tracrRNA) molecules set forth in SEQ ID NOs:837-967, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides). For example, the duplex-forming segment of the activator (or the DNA encoding the duplex-forming segment of the activator) can be 65% or more identical to one of the tracrRNA sequences set forth in SEQ ID NOs: 837-967, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides).

The duplex-forming segment of the activator (or the DNA encoding the duplex-forming segment of the activator) can be 70% or more identical to one of the tracrRNA sequences set forth in SEQ ID NOs: 837-967, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides).

The duplex-forming segment of the activator (or the DNA encoding the duplex-forming segment of the activator) can be 75% or more identical to one of the tracrRNA sequences set forth in SEQ ID NOs: 837-967, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides).

The duplex-forming segment of the activator (or the DNA encoding the duplex-forming segment of the activator) can be 80% or more identical to one of the tracrRNA sequences set forth in SEQ ID NOs: 837-967, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides).

The duplex-forming segment of the activator (or the DNA encoding the duplex-forming segment of the activator) can be 85% or more identical to one of the tracrRNA sequences set forth in SEQ ID NOs: 837-967, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides).

The duplex-forming segment of the activator (or the DNA encoding the duplex-forming segment of the activator) can be 90% or more identical to one of the tracrRNA sequences set forth in SEQ ID NOs: 837-967, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides).

The duplex-forming segment of the activator (or the DNA encoding the duplex-forming segment of the activator) can be 95% or more identical to one of the tracrRNA sequences set forth in SEQ ID NOs: 837-967, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides).

The duplex-forming segment of the activator (or the DNA encoding the duplex-forming segment of the activator) can be 98% or more identical to one of the tracrRNA sequences set forth in SEQ ID NOs: 837-967, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides).

The duplex-forming segment of the activator (or the DNA encoding the duplex-forming segment of the activator) can be 99% or more identical to one of the tracrRNA sequences set forth in SEQ ID NOs: 837-967, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides).

The duplex-forming segment of the activator (or the DNA encoding the duplex-forming segment of the activator) can be 100% identical to one of the tracrRNA sequences set forth in SEQ ID NOs: 837-967, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides).

In some embodiments, the duplex-forming segment of the targeter is 60% or more identical to one of the targeter (crRNA) sequences set forth in SEQ ID NOs: 974-1085, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides). For example, the duplex-forming segment of the targeter (or the DNA encoding the duplex-forming segment of the targeter) can be 65% or more identical to one of the crRNA sequences set forth in SEQ ID NOs: 974-1085, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides).

The duplex-forming segment of the targeter (or the DNA encoding the duplex-forming segment of the targeter) can be 70% or more identical to one of the crRNA sequences set forth in SEQ ID NOs: 974-1085, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides).

The duplex-forming segment of the targeter (or the DNA encoding the duplex-forming segment of the targeter) can be 75% or more identical to one of the crRNA sequences set forth in SEQ ID NOs: 974-1085, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides).

The duplex-forming segment of the targeter (or the DNA encoding the duplex-forming segment of the targeter) can be 80% or more identical to one of the crRNA sequences set forth in SEQ ID NOs: 974-1085, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides).

The duplex-forming segment of the targeter (or the DNA encoding the duplex-forming segment of the targeter) can be 85% or more identical to one of the crRNA sequences set forth in SEQ ID NOs: 974-1085, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides).

The duplex-forming segment of the targeter (or the DNA encoding the duplex-forming segment of the targeter) can be 90% or more identical to one of the crRNA sequences set forth in SEQ ID NOs: 974-1085, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides).

The duplex-forming segment of the targeter (or the DNA encoding the duplex-forming segment of the targeter) can be 95% or more identical to one of the crRNA sequences set forth in SEQ ID NOs: 974-1085, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides).

The duplex-forming segment of the targeter (or the DNA encoding the duplex-forming segment of the targeter) can be 98% or more identical to one of the crRNA sequences set forth in SEQ ID NOs: 974-1085, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides).

The duplex-forming segment of the targeter (or the DNA encoding the duplex-forming segment of the targeter) can be 99% or more identical to one of the crRNA sequences set forth in SEQ ID NOs: 974-1085, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides).

The duplex-forming segment of the targeter (or the DNA encoding the duplex-forming segment of the targeter) can be 100% identical to one of the crRNA sequences set forth in SEQ ID NOs: 974-1085, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides).

A dual guide nucleic acid (e.g., guide RNA) can be designed to allow for controlled (i.e., conditional) binding of a targeter with an activator. Because a dual guide nucleic acid (e.g., guide RNA) is not functional unless both the activator and the targeter are bound in a functional complex with Cas9, a dual guide nucleic acid (e.g., guide RNA)can be inducible (e.g., drug inducible) by rendering the binding between the activator and the targeter to be inducible. As one non-limiting example, RNA aptamers can be used to regulate (i.e., control) the binding of the activator with the targeter. Accordingly, the activator and/or the targeter can include an RNA aptamer sequence.

Aptamers (e.g., RNA aptamers) are known in the art and are generally a synthetic version of a riboswitch. The terms "RNA aptamer" and "riboswitch" are used interchangeably herein to encompass both synthetic and natural nucleic acid sequences that provide for inducible regulation of the structure (and therefore the availability of specific sequences) of the nucleic acid molecule (e.g., RNA, DNA/RNA hybrid, etc.) of which they are part. RNA aptamers usually comprise a sequence that folds into a particular structure (e.g., a hairpin), which specifically binds a particular drug (e.g., a small molecule). Binding of the drug causes a structural change in the folding of the RNA, which changes a feature of the nucleic acid of which the aptamer is a part. As non-limiting examples: (i) an activator with an aptamer may not be able to bind to the cognate targeter unless the aptamer is bound by the appropriate drug; (ii) a targeter with an aptamer may not be able to bind to the cognate activator unless the aptamer is bound by the appropriate drug; and (iii) a targeter and an activator, each comprising a different aptamer that binds a different drug, may not be able to bind to each other unless both drugs are present. As illustrated by these examples, a dual guide nucleic acid (e.g., guide RNA)can be designed to be inducible.

Examples of aptamers and riboswitches can be found, for example, in: Nakamura et al., Genes Cells. 2012 May; 17(5):344-64; Vavalle et al., Future Cardiol. 2012 May;8(3):371-82; Citartan et al., Biosens Bioelectron. 2012 Apr 15;34(1):1-11; and Liberman et al., Wiley Interdiscip Rev RNA. 2012 May-Jun;3(3):369-84.

Non-limiting examples of nucleotide sequences that can be included in a dual guide nucleic acid (e.g., guide RNA) include either of the sequences set forth in SEQ ID NOs:837-967, or complements thereof pairing with any sequences set forth in SEQ ID NOs: 974-1085, or complements thereof that can hybridize to form a protein binding segment.

A subject single guide nucleic acid (e.g., guide RNA) comprises two stretches of nucleotides (much like a "targeter" and an "activator" of a dual guide nucleic acid) that are complementary to one another, hybridize to form the double stranded RNA duplex (dsRNA duplex) of the protein-binding segment (thus resulting in a stem-loop structure), and are covalently linked by intervening nucleotides ("linkers" or "linker nucleotides"). Thus, a subject single guide nucleic acid (e.g., a single guide RNA) can comprise a targeter and an activator, each having a duplex-forming segment, where the duplex-forming segments of the targeter and the activator hybridize with one another to form a dsRNA duplex. The targeter and the activator can be covalently linked via the 3' end of the targeter and the 5' end of the activator. Alternatively, targeter and the activator can be covalently linked via the 5' end of the targeter and the 3' end of the activator.

The linker of a single guide nucleic acid can have a length of from 3 nucleotides to 100 nucleotides. For example, the linker can have a length of from 3 nucleotides (nt) to 90 nt, from 3 nucleotides (nt) to 80 nt, from 3 nucleotides (nt) to 70 nt, from 3 nucleotides (nt) to 60 nt, from 3 nucleotides (nt) to 50 nt, from 3 nucleotides (nt) to 40 nt, from 3 nucleotides (nt) to 30 nt, from 3 nucleotides (nt) to 20 nt or from 3 nucleotides (nt) to 10 nt. For example, the linker can have a length of from 3 nt to 5 nt, from 5 nt to 10 nt, from 10 nt to 15 nt, from 15 nt to 20 nt, from 20 nt to 25 nt, from 25 nt to 30 nt, from 30 nt to 35 nt, from 35 nt to 40 nt, from 40 nt to 50 nt, from 50 nt to 60 nt, from 60 nt to 70 nt, from 70 nt to 80 nt, from 80 nt to 90 nt, or from 90 nt to 100 nt. In some embodiments, the linker of a single guide nucleic acid (e.g., guide RNA) is 4 nt.

An exemplary single guide nucleic acid (e.g., guide RNA) comprises two complementary stretches of nucleotides that hybridize to form a dsRNA duplex. In some embodiments, one of the two complementary stretches of nucleotides of the single guide nucleic acid (e.g., guide RNA) (or the DNA encoding the stretch) is 60% or more identical to one of the activator (tracrRNA) molecules set forth in SEQ ID NOs:837-967, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides). For example, one of the two complementary stretches of nucleotides of the single guide nucleic acid (e.g., guide RNA) (or the DNA encoding the stretch) is 65% or more identical, 70% or more identical, 75% or more identical, 80% or more identical, 85% or more identical, 90% or more identical, 95% or more identical, 98% or more identical, 99% or more identical or 100% identical to one of the tracrRNA sequences set forth in SEQ ID NOs: 837-967, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides).

In some embodiments, one of the two complementary stretches of nucleotides of the single guide nucleic acid (e.g., guide RNA) (or the DNA encoding the stretch) is 60% or more identical to one of the targeter (crRNA) sequences set forth in SEQ ID NOs:974-1085, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides). For example, one of the two complementary stretches of nucleotides of the single guide nucleic acid (e.g., guide RNA) (or the DNA encoding the stretch) is 65% or more identical, 70% or more identical, 75% or more identical, 80% or more identical, 85% or more identical, 90% or more identical, 95% or more identical, 98% or more identical, 99% or more identical or 100% identical to one of the crRNA sequences set forth in SEQ ID NOs: 974-1085, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides).

In some embodiments, one of the two complementary stretches of nucleotides of the single guide nucleic acid (e.g., guide RNA) (or the DNA encoding the stretch) is 60% or more identical to one of the targeter (crRNA) sequences or activator (tracrRNA) sequences set forth in SEQ ID NOs:837-967 and 974-1085, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides). For example, one of the two complementary stretches of nucleotides of the single guide nucleic acid (e.g., guide RNA) (or the DNA encoding the stretch) can be 65% or more identical to one of the sequences set forth in SEQ ID NOs: 837-967 and 974-1085, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides).

One of the two complementary stretches of nucleotides of the single guide nucleic acid (e.g., guide RNA) (or the DNA encoding the stretch) can be 70% or more identical to one of the sequences set forth in SEQ ID NOs: 837-967 and 974-1085, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides).

One of the two complementary stretches of nucleotides of the single guide nucleic acid (e.g., guide RNA) (or the DNA encoding the stretch) can be 75% or more identical to one of the sequences set forth in SEQ ID NOs: 837-967 and 974-1085, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides).

One of the two complementary stretches of nucleotides of the single guide nucleic acid (e.g., guide RNA) (or the DNA encoding the stretch) can be 80% or more identical to one of the sequences set forth in SEQ ID NOs: 837-967 and 974-1085, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides).

One of the two complementary stretches of nucleotides of the single guide nucleic acid (e.g., guide RNA) (or the DNA encoding the stretch) can be 85% or more identical to one of the sequences set forth in SEQ ID NOs: 837-967 and 974-1085, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides).

One of the two complementary stretches of nucleotides of the single guide nucleic acid (e.g., guide RNA) (or the DNA encoding the stretch) can be 90% or more identical to one of the sequences set forth in SEQ ID NOs: 837-967 and 974-1085, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides).

One of the two complementary stretches of nucleotides of the single guide nucleic acid (e.g., guide RNA) (or the DNA encoding the stretch) can be 95% or more identical to one of the sequences set forth in SEQ ID NOs: 837-967 and 974-1085, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides).

One of the two complementary stretches of nucleotides of the single guide nucleic acid (e.g., guide RNA) (or the DNA encoding the stretch) can be 98% or more identical to one of the sequences set forth in SEQ ID NOs: 837-967 and 974-1085, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides).

One of the two complementary stretches of nucleotides of the single guide nucleic acid (e.g., guide RNA) (or the DNA encoding the stretch) can be 99% or more identical to one of the sequences set forth in SEQ ID NOs: 837-967 and 974-1085, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides).

One of the two complementary stretches of nucleotides of the single guide nucleic acid (e.g., guide RNA) (or the DNA encoding the stretch) can be 100% identical to one of the sequences set forth in SEQ ID NOs: 837-967 and 974-1085, or a complement thereof, over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides).

Appropriate cognate pairs of targeters and activators can be routinely determined for SEQ ID NOs: 837-967 and 974-1085 by taking into account the species name and base-pairing (for the dsRNA duplex of the protein-binding domain) Any activator/targeter pair can be used as part of dual guide nucleic acid (e.g., guide RNA) or as part of a single guide nucleic acid (e.g., guide RNA).

In some cases, an activator (e.g., a trRNA, trRNA-like molecule, etc.) of a dual guide nucleic acid (e.g., guide RNA) (e.g., a dual guide RNA) or a single guide nucleic acid (e.g., guide RNA) (e.g., a single guide RNA) includes a stretch of nucleotides with 60% or more sequence identity (e.g., 65% or more, 70% or more, 75% or more, 80% or more, 85% or more, 90% or more, 95% or more, 98% or more, or 100% sequence identity) with an activator (tracrRNA) molecule set forth in any one of SEQ ID NOs:837-967, or a complement thereof. In some cases, an activator (e.g., a trRNA, trRNA-like molecule, etc.) of a dual guide nucleic acid (e.g., guide RNA) (e.g., a dual guide RNA) or a single guide nucleic acid (e.g., a single guide RNA) includes a stretch of nucleotides with 70% or more sequence identity with an activator (tracrRNA) molecule set forth in any one of SEQ ID NOs: 837-967, or a complement thereof. In some cases, an activator (e.g., a trRNA, trRNA-like molecule, etc.) of a dual guide nucleic acid (e.g., a dual guide RNA) or a single guide nucleic acid (e.g., a single guide RNA) includes a stretch of nucleotides with 75% or more sequence identity with an activator (tracrRNA) molecule set forth in any one of SEQ ID NOs:837-967, or a complement thereof. In some cases, an activator (e.g., a trRNA, trRNA-like molecule, etc.) of a dual guide nucleic acid (e.g., a dual guide RNA) or a single guide nucleic acid (e.g., a single guide RNA) includes a stretch of nucleotides with 80% or more sequence identity with an activator (tracrRNA) molecule set forth in any one of SEQ ID NOs: 837-967, or a complement thereof. In some cases, an activator (e.g., a trRNA, trRNA-like molecule, etc.) of a dual guide nucleic acid (e.g., a dual guide RNA) or a single guide nucleic acid (e.g., a single guide RNA) includes a stretch of nucleotides with 85% or more sequence identity with an activator (tracrRNA) molecule set forth in any one of SEQ ID NOs: 837-967, or a complement thereof. In some cases, an activator (e.g., a trRNA, trRNA-like molecule, etc.) of a dual guide nucleic acid (e.g., a dual guide RNA) or a single guide nucleic acid (e.g., a single guide RNA) includes a stretch of nucleotides with 90% or more sequence identity with an activator (tracrRNA) molecule set forth in any one of SEQ ID NOs: 837-967, or a complement thereof. In some cases, an activator (e.g., a trRNA, trRNA-like molecule, etc.) of a dual guide nucleic acid (e.g., a dual guide RNA) or a single guide nucleic acid (e.g., a single guide RNA) includes a stretch of nucleotides with 95% or more sequence identity with an activator (tracrRNA) molecule set forth in any one of SEQ ID NOs: 837-967, or a complement thereof. In some cases, an activator (e.g., a trRNA, trRNA-like molecule, etc.) of a dual guide nucleic acid (e.g., a dual guide RNA) or a single guide nucleic acid (e.g., a single guide RNA) includes a stretch of nucleotides with 98% or more sequence identity with an activator (tracrRNA) molecule set forth in any one of SEQ ID NOs: 837-967, or a complement thereof. In some cases, an activator (e.g., a trRNA, trRNA-like molecule, etc.) of a dual guide nucleic acid (e.g., a dual guide RNA) or a single guide nucleic acid (e.g., a single guide RNA) includes a stretch of nucleotides with 100% sequence identity with an activator (tracrRNA) molecule set forth in any one of SEQ ID NOs: 837-967, or a complement thereof.

In some cases, an activator (e.g., a trRNA, trRNA-like molecule, etc.) of a dual guide nucleic acid (e.g., a dual guide RNA) or a single guide nucleic acid (e.g., a single guide RNA) includes 30 or more nucleotides (nt) (e.g., 40 or more, 50 or more, 60 or more, 70 or more, 75 or more nt). In some cases, an activator (e.g., a trRNA, trRNA-like molecule, etc.) of a dual guide nucleic acid (e.g., a dual guide RNA) or a single guide nucleic acid (e.g., a single guide RNA) has a length in a range of from 30 to 200 nucleotides (nt) (e.g., 40 to 200 nucleotides, 50 to 200 nucleotides, 60 to 200 nucleotides, 65 to 200 nucleotides, 70 to 200 nucleotides, 75 to 200 nucleotides, 40 to 150 nucleotides, 50 to 150 nucleotides, 60 to 150 nucleotides, 65 to 150 nucleotides, 70 to 150 nucleotides, 75 to 150 nucleotides, 40 to 100 nucleotides, 50 to 100 nucleotides, 60 to 100 nucleotides, 65 to 100 nucleotides, 70 to 100 nucleotides, or 75 to 100 nucleotides).

The protein-binding segment can have a length of from 10 nucleotides to 100 nucleotides. For example, the protein-binding segment can have a length of from 15 nucleotides (nt) to 80 nt, from 15 nt to 50 nt, from 15 nt to 40 nt, from 15 nt to 30 nt or from 15 nt to 25 nt.

Also with regard to both a subject single guide nucleic acid (e.g., single guide RNA) and to a subject dual guide nucleic acid (e.g., dual guide RNA), the dsRNA duplex of the protein-binding segment can have a length from 6 base pairs (bp) to 50bp. For example, the dsRNA duplex of the protein-binding segment can have a length from 6 bp to 40 bp, from 6 bp to 30bp, from 6 bp to 25 bp, from 6 bp to 20 bp, from 6 bp to 15 bp, from 8 bp to 40 bp, from 8 bp to 30bp, from 8 bp to 25 bp, from 8 bp to 20 bp or from 8 bp to 15 bp. For example, the dsRNA duplex of the protein-binding segment can have a length from from 8 bp to 10 bp, from 10 bp to 15 bp, from 15 bp to 18 bp, from 18 bp to 20 bp, from 20 bp to 25 bp, from 25 bp to 30 bp, from 30 bp to 35 bp, from 35 bp to 40 bp, or from 40 bp to 50 bp. In some embodiments, the dsRNA duplex of the protein-binding segment has a length of 36 base pairs. In some embodiments, the dsRNA duplex of the protein-binding segment has a length of 12 base pairs. In some embodiments, the dsRNA duplex of the protein-binding segment has a length of 16 base pairs. In some embodiments, the dsRNA duplex of the protein-binding segment has a length of 17 base pairs. The percent complementarity between the nucleotide sequences that hybridize to form the dsRNA duplex of the protein-binding segment can be 60% or more. For example, the percent complementarity between the nucleotide sequences that hybridize to form the dsRNA duplex of the protein-binding segment can be 65% or more, 70% or more, 75% or more, 80% or more, 85% or more, 90% or more, 95% or more, 98% or more, or 99% or more (e.g., in some cases, there are a some nucleotides that do not hybridize and therefore create a bulge within the dsRNA duplex. In some cases, the percent complementarity between the nucleotide sequences that hybridize to form the dsRNA duplex of the protein-binding segment is 100%.

### Hybrid guide nucleic acids

In some cases, a guide nucleic acid is two RNA molecules (dual guide RNA). In some cases, a guide nucleic acid is one RNA molecule (single guide RNA). In some cases, a guide nucleic acid is a DNA/RNA hybrid molecule. In such cases, the protein-binding segment of the guide nucleic acid is RNA and forms an RNA duplex. Thus, the duplex-forming segments of the activator and the targeter is RNA. However, the targeting segment of a guide nucleic acid can be DNA. Thus, if a DNA/RNA hybrid guide nucleic acid is a dual guide nucleic acid, the "targeter" molecule and be a hybrid molecule (e.g., the targeting segment can be DNA and the duplex-forming segment can be RNA). In such cases, the duplex-forming segment of the "activator" molecule can be RNA (e.g., in order to form an RNA-duplex with the duplex-forming segment of the targeter molecule), while nucleotides of the "activator" molecule that are outside of the duplex-forming segment can be DNA (in which case the activator molecule is a hybrid DNA/RNA molecule) or can be RNA (in which case the activator molecule is RNA). If a DNA/RNA hybrid guide nucleic acid is a single guide nucleic acid, then the targeting segment can be DNA, the duplex-forming segments (which make up the protein-binding segment of the single guide nucleic acid) can be RNA, and nucleotides outside of the targeting and duplex-forming segments can be RNA or DNA.

A DNA/RNA hybrid guide nucleic can be useful in some cases, for example, when a target nucleic acid is an RNA. Cas9 normally associates with a guide RNA that hybridizes with a target DNA, thus forming a DNA-RNA duplex at the target site. Therefore, when the target nucleic acid is an RNA, it is sometimes advantageous to recapitulate a DNA-RNA duplex at the target site by using a targeting segment (of the guide nucleic acid) that is DNA instead of RNA. However, because the protein-binding segment of a guide nucleic acid is an RNA-duplex, the targeter molecule is DNA in the targeting segment and RNA in the duplex-forming segment. Hybrid guide nucleic acids can bias Cas9 binding to single stranded target nucleic acids relative to double stranded target nucleic acids.

### Exemplary guide nucleic acids

In some embodiments, a suitable guide nucleic acid includes two separate RNA polynucleotide molecules. In some cases, the first of the two separate RNA polynucleotide molecules (the activator) comprises a nucleotide sequence having 60% or more (e.g., 65% or more, 70% or more, 75% or more, 80% or more, 85% or more, 90% or more, 95% or more, 98% or more, 99% or more, or 100%) nucleotide sequence identity over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides) to any one of the nucleotide sequences set forth in SEQ ID NOs:837-967 and 974-1085, or a complement thereof. In some cases, the second of the two separate RNA polynucleotide molecules (the targeter) comprises a nucleotide sequence having 60% or more (e.g., 65% or more, 70% or more, 75% or more, 80% or more, 85% or more, 90% or more, 95% or more, 98% or more, 99% or more, or 100%) nucleotide sequence identity over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides) to any one of the nucleotide sequences set forth in SEQ ID NOs: 837-967 and 974-1085, or a complement thereof.

In some embodiments, a suitable guide nucleic acid is a single RNA polynucleotide and comprises a first nucleotide sequence having 60% or more (e.g., 65% or more, 70% or more, 75% or more, 80% or more, 85% or more, 90% or more, 95% or more, 98% or more, 99% or more, or 100%) nucleotide sequence identity over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides) to any one of the nucleotide sequences set forth in SEQ ID NOs: 837-967 and 974-1085 and a second nucleotide sequence having 60% or more (e.g., 65% or more, 70% or more, 75% or more, 80% or more, 85% or more, 90% or more, 95% or more, 98% or more, 99% or more, or 100%) nucleotide sequence identity over a stretch of 8 or more contiguous nucleotides (e.g., 8 or more contiguous nucleotides, 10 or more contiguous nucleotides, 12 or more contiguous nucleotides, 15 or more contiguous nucleotides, or 20 or more contiguous nucleotides) to any one of the nucleotide sequences set forth in SEQ ID NOs: 837-967 and 974-1085.

### Additional sequences

In some embodiments, a guide nucleic acid (e.g., guide RNA) includes an additional segment or segments (in some cases at the 5' end, in some cases the 3' end, in some cases at either the 5' or 3' end, in some cases embedded within the sequence (i.e., not at the 5' and/or 3' end), in some cases at both the 5' end and the 3' end, in some cases embedded and at the 5' end and/or the 3' end, etc). For example, a suitable additional segment can include a 5' cap (e.g., a 7-methylguanylate cap (m⁷G)); a 3' polyadenylated tail (i.e., a 3' poly(A) tail); a ribozyme sequence (e.g. to allow for self-cleavage of a guide nucleic acid (or component of a guide nucleic acid, e.g., a targeter, an activator, etc.); a riboswitch sequence (e.g., to allow for regulated stability and/or regulated accessibility by proteins and protein complexes); a sequence that forms a dsRNA duplex (i.e., a hairpin)); a sequence that targets an RNA to a subcellular location (e.g., nucleus, mitochondria, chloroplasts, and the like); a modification or sequence that provides for tracking (e.g., a direct label (e.g., direct conjugation to a fluorescent molecule (i.e., fluorescent dye)), conjugation to a moiety that facilitates fluorescent detection, a sequence that allows for fluorescent detection; a modification or sequence that provides a binding site for proteins (e.g., proteins that act on DNA, including transcriptional activators, transcriptional repressors, DNA methyltransferases, DNA demethylases, histone acetyltransferases, histone deacetylases, proteins that bind RNA (e.g., RNA aptamers), labeled proteins, fluorescently labeled proteins, and the like); a modification or sequence that provides for increased, decreased, and/or controllable stability; and combinations thereof.

### Cpfl guide RNA

A Cpfl guide RNA can have a total length of from 30 nucleotides (nt) to 100 nt, e.g., from 30 nt to 40 nt, from 40 nt to 45 nt, from 45 nt to 50 nt, from 50 nt to 60 nt, from 60 nt to 70 nt, from 70 nt to 80 nt, from 80 nt to 90 nt, or from 90 nt to 100 nt. In some cases, a Cpfl guide RNA has a total length of 35 nt, 36 nt, 37 nt, 38 nt, 39 nt, 40 nt, 41 nt, 42 nt, 43 nt, 44 nt, 45 nt, 46 nt, 47 nt, 48 nt, 49 nt, or 50 nt.

A Cpfl guide RNA can include a target nucleic acid-binding segment and a duplex-forming segment.

The target nucleic acid-binding segment of a Cpfl guide RNA can have a length of from 15 nt to 30 nt, e.g., 15 nt, 16 nt, 17 nt, 18 nt, 19 nt, 20 nt, 21 nt, 22 nt, 23 nt, 24 nt, 25 nt, 26 nt, 27 nt, 28 nt, 29 nt,, or 30 nt. In some cases, the target nucleic acid-binding segment has a length of 23 nt. In some cases, the target nucleic acid-binding segment has a length of 24 nt. In some cases, the target nucleic acid-binding segment has a length of 25 nt.

The target nucleic acid-binding segment of a Cpfl guide RNA can have 100% complementarity with a corresponding length of target nucleic acid sequence. The targeting segment can have less than 100% complementarity with a corresponding length of target nucleic acid sequence. For example, the target nucleic acid binding segment of a Cpfl guide RNA can have 1, 2, 3, 4, or 5 nucleotides that are not complementary to the target nucleic acid sequence. For example, in some cases, where a target nucleic acid-binding segment has a length of 25 nucleotides, and the target nucleic acid sequence has a length of 25 nucleotides, in some cases, the target nucleic acid-binding segment has 100% complementarity to the target nucleic acid sequence. As another example, in some cases, where a target nucleic acid-binding segment has a length of 25 nucleotides, and the target nucleic acid sequence has a length of 25 nucleotides, in some cases, the target nucleic acid-binding segment has 1 non-complementary nucleotide and 24 complementary nucleotides with the target nucleic acid sequence. As another example, in some cases, where a target nucleic acid-binding segment has a length of 25 nucleotides, and the target nucleic acid sequence has a length of 25 nucleotides, in some cases, the target nucleic acid-binding segment has 2 non-complementary nucleotide and 23 complementary nucleotides with the target nucleic acid sequence.

The duplex-forming segment of a Cpfl guide RNA can have a length of from 15 nt to 25 nt, e.g., 15 nt, 16 nt, 17 nt, 18 nt, 19 nt, 20 nt, 21 nt, 22 nt, 23 nt, 24 nt, or 25 nt.

In some cases, the duplex-forming segment of a Cpfl guide RNA can comprise the nucleotide sequence 5'-AAUUUCUACUGUUGUAGAU-3' (SEQ ID NO: 1139).

### Polvcation-based endosomal escape polymers

Polymers suitable for inclusion in a complex of the present disclosure include polycation-containing polymers that provide for enhanced escape from an endosomal compartment in a eukaryotic cell. Such polymers are referred to herein as "endosomal disruptive polymers." A nucleic acid-conjugated colloidal metal nanoparticle is complexed with a Type II CRISPR system comprising a Cas9 polypeptide and a guide RNA, and the nucleic acid-conjugated colloidal metal nanoparticle/Type II CRISPR system complex is encapsulated in an endosomal disruptive polymer. In some cases, a Type II CRISPR system comprises: i) a Cas9 polypeptide; ii) a guide RNA; and iii) a donor template polynucleotide; and the nucleic acid-conjugated colloidal metal nanoparticle/Type II CRISPR system complex is encapsulated in an endosomal disruptive polymer.

In some cases, an endosomal disruptive polymer suitable for inclusion in a complex of the present disclosure is a cationic polymer selected from the group consisting of polyethylene imine, poly(arginine), poly(lysine), poly(histidine), poly-[2-{(2-aminoethyl)amino}-ethyl-aspartamide] (pAsp(DET)), a block co-polymer of poly(ethylene glycol) (PEG) and poly(arginine), a block co-polymer of PEG and poly(lysine), and a block co-polymer of PEG and poly{*N*-[*N*-(2-aminoethyl)-2-aminoethyl]aspartamide} (PEG-pAsp(DET)). In some cases, a complex of the present disclosure comprises poly{*N*-[*N*-(2-aminoethyl)-2-aminoethyl]aspartamide} (PEG-pAsp(DET)). In some cases, polyethylenimine (PEI) is used.

In some cases, a complex of the present disclosure further includes a silicate in the portion of the complex that encapsulates the nucleic acid-conjugated colloidal metal nanoparticle/Type II CRISPR system complex. In some cases, a nucleic acid-conjugated colloidal metal nanoparticle/Type II CRISPR system complex is encapsulated in alternating layers of an endosomal disruptive polymer and a silicate. In some cases, a nucleic acid-conjugated colloidal metal nanoparticle/Type II CRISPR system complex is encapsulated in a single layer of an endosomal disruptive polymer. In some cases, a nucleic acid-conjugated colloidal metal nanoparticle/Type II CRISPR system complex is encapsulated in two or more layer of an endosomal disruptive polymer.

### Donor template DNA

As noted above, in some cases, a complex of the present disclosure comprises: a) a nanoparticle-nucleic acid conjugate; b) a Type II or a Type V CRISPR system comprising a site-directed DNA-modifying polypeptide and a guide RNA; and c) a polycation-based endosomal escape polymer, and may optionally also comprise a donor polynucleotide (e.g., a donor DNA template).

By a "donor sequence" or "donor polynucleotide" or "donor DNA template" it is meant a nucleic acid sequence to be inserted at the cleavage site induced by a site-directed modifying polypeptide (e.g., a Cas9 polypeptide or a Cpfl polypeptide). The donor polynucleotide will contain sufficient homology to a target genomic sequence at the cleavage site, e.g. 70%, 80%, 85%, 90%, 95%, or 100% homology with the nucleotide sequences flanking the cleavage site, e.g. within about 50 bases or less of the cleavage site, e.g. within about 30 bases, within about 15 bases, within about 10 bases, within about 5 bases, or immediately flanking the cleavage site, to support homology-directed repair between it and the genomic sequence to which it bears homology. Approximately 25, 50, 100, or 200 nucleotides, or more than 200 nucleotides, of sequence homology between a donor and a genomic sequence (or any integral value between 10 and 200 nucleotides, or more) will support homology-directed repair. Donor sequences can be of any length, e.g. 10 nucleotides or more, 50 nucleotides or more, 100 nucleotides or more, 250 nucleotides or more, 500 nucleotides or more, 1000 nucleotides or more, 5000 nucleotides or more, etc.

The donor sequence is typically not identical to the genomic sequence that it replaces. Rather, the donor sequence may contain one or more single base changes, insertions, deletions, inversions or rearrangements with respect to the genomic sequence, so long as sufficient homology is present to support homology-directed repair. In some embodiments, the donor sequence comprises a non-homologous sequence flanked by two regions of homology, such that homology-directed repair between the target DNA region and the two flanking sequences results in insertion of the non-homologous sequence at the target region. Donor sequences may also comprise a vector backbone containing sequences that are not homologous to the DNA region of interest and that are not intended for insertion into the DNA region of interest. Generally, the homologous region(s) of a donor sequence will have at least 50% sequence identity to a genomic sequence with which recombination is desired. In certain embodiments, 60%, 70%, 80%, 90%, 95%, 98%, 99%, or 99.9% sequence identity is present. Any value between 1% and 100% sequence identity can be present, depending upon the length of the donor polynucleotide.

The donor sequence may comprise certain sequence differences as compared to the genomic sequence, e.g. restriction sites, nucleotide polymorphisms, selectable markers (e.g., drug resistance genes, fluorescent proteins, enzymes etc.), etc., which may be used to assess for successful insertion of the donor sequence at the cleavage site or in some cases may be used for other purposes (e.g., to signify expression at the targeted genomic locus). In some cases, if located in a coding region, such nucleotide sequence differences will not change the amino acid sequence, or will make silent amino acid changes (i.e., changes which do not affect the structure or function of the protein). Alternatively, these sequences differences may include flanking recombination sequences such as FLPs, loxP sequences, or the like, that can be activated at a later time for removal of the marker sequence.

The donor sequence may be provided to the cell as single-stranded DNA, single-stranded RNA, double-stranded DNA, or double-stranded RNA. It may be introduced into a cell in linear or circular form. If introduced in linear form, the ends of the donor sequence may be protected (e.g., from exonucleolytic degradation) by methods known to those of skill in the art. For example, one or more dideoxynucleotide residues are added to the 3' terminus of a linear molecule and/or self-complementary oligonucleotides are ligated to one or both ends. See, for example, Chang et al. (1987) Proc. Natl. Acad Sci USA 84:4959-4963; Nehls et al. (1996) Science 272:886-889. Additional methods for protecting exogenous polynucleotides from degradation include, but are not limited to, addition of terminal amino group(s) and the use of modified internucleotide linkages such as, for example, phosphorothioates, phosphoramidates, and O-methyl ribose or deoxyribose residues. As an alternative to protecting the termini of a linear donor sequence, additional lengths of sequence may be included outside of the regions of homology that can be degraded without impacting recombination. A donor sequence can be introduced into a cell as part of a vector molecule having additional sequences such as, for example, replication origins, promoters and genes encoding antibiotic resistance. Moreover, donor sequences can be introduced as naked nucleic acid, as nucleic acid complexed with an agent such as a liposome or poloxamer, or can be delivered by viruses (e.g., adenovirus, AAV), as described above for nucleic acids encoding a Cas9 guide RNA and/or a Cas9 fusion polypeptide and/or donor polynucleotide.

### METHODS

The present disclosure provides methods of making a complex of the present disclosure. The present disclosure provides methods of using a complex of the present disclosure. A complex of the present disclosure can be used to modify a target nucleic acid in a eukaryotic cell. A complex of the present disclosure can be used to modulate transcription of a target nucleic acid in a eukaryotic cell.

### Method of Making a Complex

Further aspects of the present disclosure include a method of making a complex of the present disclosure. In some cases, the nanoparticle is functionalized with a sulfur (e.g., a thiol moiety), and the nucleic acid is attached to the nanoparticle via the sulfur (e.g., via the thiol moiety). Once the nucleic acid is attached to the nanoparticle, the Type II site directed DNA modifying polypeptide (e.g., Cas9 polypeptide) or the Type V site directed DNA modifying polypeptide (e.g., Cpfl polypeptide) and the guide RNA are contacted with the nucleic acid-nanoparticle conjugate, to form a complex of the present disclosure.

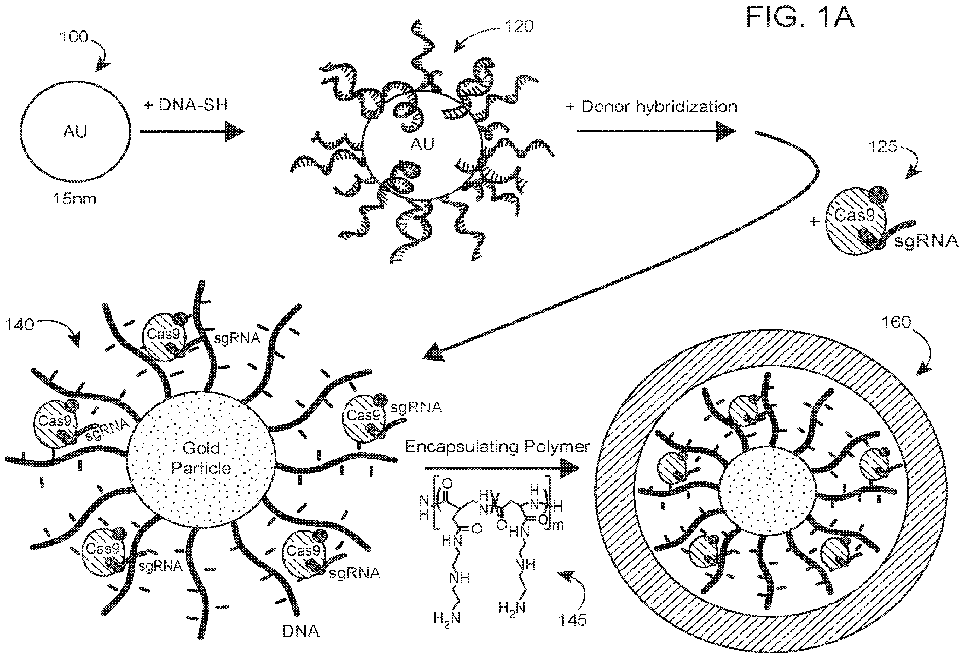

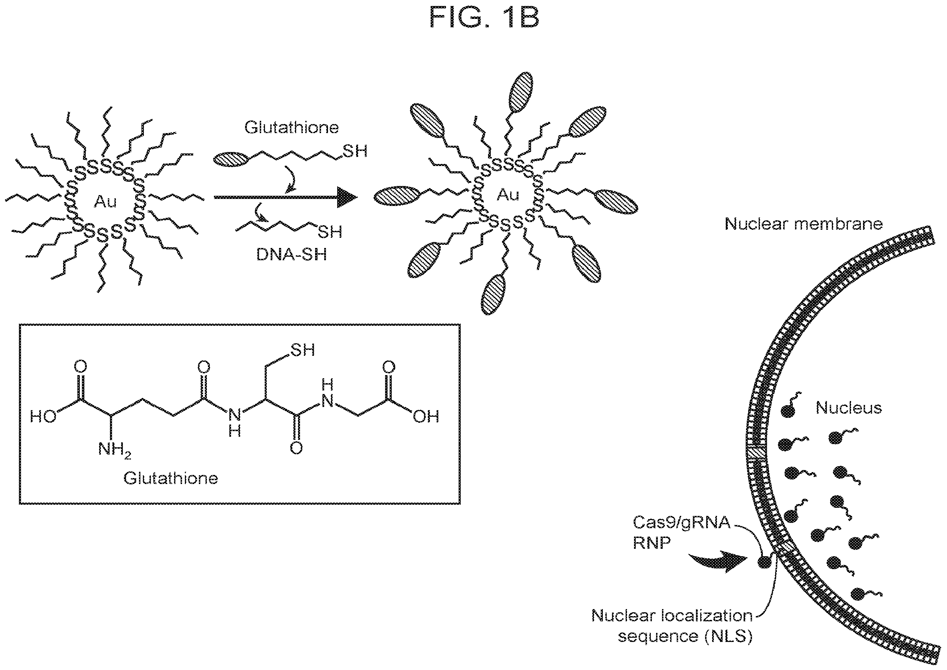

An implementation of the method is described with reference to FIG. 1A. The method may include loading a gold nanoparticle (GNP) conjugated to DNA via a thiol group (**120**) with a Cas9/gRNA ribonucleoprotein (RNP) (**125**) to produce a Cas9 RNP-DNA-GNP complex (**140**).

The GNP-DNA conjugate may be produced by reacting a GNP (**100**) with a DNA-thiol. The GNP may have a diameter of about 30 nm.

In some cases, the GNP-DNA conjugate (**120**) is hybridized with a donor single-stranded DNA before loading the Cas9 RNP.

After forming the Cas9 RNP-DNA-GNP complex (**140**), the complex may be coated with silicate and an endosomal disruptive polymer (**145**), such as a PAsp(DET) polymer to form an encapsulated Cas9 RNP-DNA-GNP complex (**160**).

### Method of Binding a Target Nucleic Acid and Methods of Modifying a Target Nucleic Acid

The present disclosure provides methods of binding a target nucleic acid present in a eukaryotic cell. The methods generally involve contacting a eukaryotic cell comprising a target nucleic acid with a complex of the present disclosure, wherein the complex enters the cell, and wherein the guide RNA and site-directed DNA-modifying polypeptide (e.g., a Cas9 polypeptide or a Cpfl polypeptide) (and, if present, a donor polynucleotide) are released from the complex in an endosome in the cell. Once released from the endosome, the guide RNA and site-directed DNA-modifying polypeptide (e.g., a Cas9 polypeptide or a Cpfl polypeptide) (and, if present, a donor polynucleotide) can bind a target nucleic acid, e.g., where the target nucleic acid is in the nucleus, in a mitochondrion, or in the cytoplasm. In some case, the cell is *in vitro.* In some cases, the cell is *in vivo.* In some cases, the cell is present in a multicellular organism. In some cases, where the complex comprises a dead Cas9 polypeptide, the dead Cas9 polypeptide modulates transcription from the target nucleic acid. In some cases, e.g., where the complex comprises a Cas9 fusion polypeptide, the Cas9 fusion polypeptide modifies the target nucleic acid. In some cases, where the complex comprises a Cas9 polypeptide, the Cas9 polypeptide cleaves the target nucleic acid. In some cases, where the complex comprises a Cpfl polypeptide, the Cpfl polypeptide cleaves the target nucleic acid.

As noted above, in some cases, the complex comprises a donor template polynucleotide. In these instances, the method comprises contacting the target nucleic acid with the donor template polynucleotide. In some cases, the donor polynucleotide (e.g., a DNA repair template) replaces at least a portion of a target nucleic acid, e.g., to repair a defect in the target nucleic acid.

The present disclosure provides methods of genetically modifying a eukaryotic target cell. The methods generally involve contacting the eukaryotic target cell with a complex of the present disclosure. The complex enters the cell, and the guide RNA, site-directed DNA-modifying polypeptide (e.g., a Cas9 polypeptide or a Cpfl polypeptide), and donor polynucleotide (if present) are released from the complex in an endosome in the cell. Once released from the endosome, the guide RNA and site-directed DNA-modifying polypeptide (e.g., a Cas9 polypeptide or a Cpfl polypeptide) (and, if present, a donor polynucleotide) can bind a target nucleic acid, e.g., where the target nucleic acid is in the nucleus, in a mitochondrion, or in the cytoplasm. In some case, the cell is *in vitro.* In some cases, the cell is *in vivo.* In some cases, the cell is present in a multicellular organism. In some cases, the target cell is an insect cell. In some cases, the target cell is an arachnid cell. In some cases, the target cell is a cell of or in an invertebrate. In some cases, the target cell is a protozoan cell. In some cases, the target cell is a plant cell. In some cases, the target cell is present in a plant or a plant tissue. In some cases, the target cell is an animal cell. In some cases, the target cell is present in an animal, e.g., a human, or a non-human animal. In some cases, the target cell is a mammalian cell. In some cases, the target cell is present in a mammal, e.g., in a human or a non-human mammal. In some cases, is a myoblast, a neuron, a chondrocyte, a lymphocyte, an epithelial cell, an adipocyte, or a keratinocyte. In some cases, the target cell is pluripotent cell. In some cases, the target cell is a stem cell, e.g., an embryonic stem cell, a neuronal stem cell, a hematopoietic stem cell, an adult stem cell, an induced stem cell, etc.

A method of the present disclosure can be used in combination with one or more other methods of delivering a Type II or Type V CRISPR system to a eukaryotic cell. For example, in some cases, a method of the present disclosure for genetically modifying a eukaryotic target cell comprises administering to an individual in need thereof a complex of the present disclosure; and administering a recombinant vector comprising a nucleotide sequence encoding one or more components of a Type II or Type V CRISPR system (e.g., a nucleotide sequence encoding a Cas9 polypeptide; a nucleotide sequence encoding a Cpfl polypeptide; a nucleotide sequence encoding a guide RNA). As another example, in some cases, a method of the present disclosure for genetically modifying a eukaryotic target cell comprises administering to an individual in need thereof a complex of the present disclosure; and administering an RNA comprising a nucleotide sequence encoding one or more components of a Type II or Type V CRISPR system (e.g., a nucleotide sequence encoding a Cas9 polypeptide; a nucleotide sequence encoding a Cpfl polypeptide; a nucleotide sequence encoding a guide RNA).

### Target cells of interest

In some of the above applications, the subject methods may be employed to induce target nucleic acid cleavage, target nucleic acid modification, and/or to bind target nucleic acids (e.g., for visualization, for collecting and/or analyzing, etc.) in mitotic or post-mitotic cells *in vivo* and/or *ex vivo* and/or *in vitro* (e.g., to disrupt production of a protein encoded by a targeted mRNA). Because the guide nucleic acid provides specificity by hybridizing to target nucleic acid, a mitotic and/or post-mitotic cell of interest in the disclosed methods may include a cell from any eukaryotic cell or organism (e.g. a cell of a single-cell eukaryotic organism, a plant cell, an algal cell, e.g., *Botryococcus braunii, Chlamydomonas reinhardtii, Nannochloropsis gaditana, Chlorella pyrenoidosa, Sargassum patens, C. agardh,* and the like, a fungal cell (e.g., a yeast cell), an animal cell, a cell from an invertebrate animal (e.g. fruit fly, cnidarian, echinoderm, nematode, an insect, an arachnid, etc.), a cell from a vertebrate animal (e.g., fish, amphibian, reptile, bird, mammal), a cell from a mammal, a cell from a rodent, a cell from a human, etc.), or a protozoan cell.

Any type of cell may be of interest (e.g. a stem cell, e.g. an embryonic stem (ES) cell, an induced pluripotent stem (iPS) cell, a germ cell; a somatic cell, e.g. a fibroblast, a hematopoietic cell, a neuron, a muscle cell, a bone cell, a hepatocyte, a pancreatic cell; an in vitro or in vivo embryonic cell of an embryo at any stage, e.g., a 1-cell, 2-cell, 4-cell, 8-cell, etc. stage zebrafish embryo; etc.). Cells may be from established cell lines or they may be primary cells, where "primary cells", "primary cell lines", and "primary cultures" are used interchangeably herein to refer to cells and cells cultures that have been derived from a subject and allowed to grow in vitro for a limited number of passages, i.e. splittings, of the culture. For example, primary cultures are cultures that may have been passaged 0 times, 1 time, 2 times, 4 times, 5 times, 10 times, or 15 times, but not enough times go through the crisis stage. In some cases, the primary cell lines are maintained for fewer than 10 passages in vitro. Target cells are in some cases unicellular organisms, or are grown in culture.

If the cells are primary cells, they may be harvest from an individual by any convenient method. For example, leukocytes may be conveniently harvested by apheresis, leukocytapheresis, density gradient separation, etc., while cells from tissues such as skin, muscle, bone marrow, spleen, liver, pancreas, lung, intestine, stomach, etc. are most conveniently harvested by biopsy. An appropriate solution may be used for dispersion or suspension of the harvested cells. Such solution will generally be a balanced salt solution, e.g. normal saline, phosphate-buffered saline (PBS), Hank's balanced salt solution, etc., conveniently supplemented with fetal calf serum or other naturally occurring factors, in conjunction with an acceptable buffer at low concentration, generally from 5-25 mM. Convenient buffers include HEPES, phosphate buffers, lactate buffers, etc. The cells may be used immediately, or they may be stored, frozen, for long periods of time, being thawed and capable of being reused. In such cases, the cells will usually be frozen in 10% or more DMSO, 50% or more serum, and about 40% buffered medium, or some other such solution as is commonly used in the art to preserve cells at such freezing temperatures, and thawed in a manner as commonly known in the art for thawing frozen cultured cells.

In some cases, a method of modifying a target nucleic acid comprises homology-directed repair (HDR). In some cases, use of a complex of the present disclosure to carry out HDR provides an efficiency of HDR of at least 1%, at least 2%, at least 3%, at least 4%, at least 5%, at least 10%, at least 15%, at least 20%, at least 25%, or more than 25%.

In some cases, a method of modifying a target nucleic acid comprises non-homologous end joining (NHEJ). In some cases, use of a complex of the present disclosure to carry out HDR provides an efficiency of NHEJ of at least 1%, at least 2%, at least 3%, at least 4%, at least 5%, at least 10%, at least 15%, at least 20%, at least 25%, or more than 25%.

### UTILITY

Methods of the present disclosure for binding and/or modifying a target nucleic acid in a eukaryotic cell are useful in a variety of therapeutic and research applications, including site directed DNA recombination for genome editing, gene inactivation, transcriptional attenuation and transcriptional enhancement.

Methods of the present disclosure for binding and/or modifying a target nucleic acid in a eukaryotic cell are useful for carrying out non-homologous end joining or homology-directed repair. Thus, for example, a method of the present disclosure for modifying a target nucleic acid in a eukaryotic cell is useful for modifying the genome of the cell, e.g., in the context of treating a disease caused by a mutation in the genome.

### KITS

The present disclosure provides a kit for carrying out a method of the present disclosure.

In some cases, a kit of the present disclosure comprises a complex comprising: a) a nanoparticle-nucleic acid conjugate; a Type II or a Type V CRISPR system comprising a site-directed DNA-modifying polypeptide and a guide RNA, and optionally also comprising a donor polynucleotide (e.g., a DNA donor template); and b) a polycation-based endosomal escape polymer. In some cases, a kit includes a recombinant expression vector that provides for *in vitro* production of a guide RNA.

In some cases, a kit of the present disclosure comprises a complex comprising: a) a nanoparticle-nucleic acid conjugate; a Cas9 polypeptide; and a guide RNA; and b) a polycation-based endosomal escape polymer. In some cases, a kit of the present disclosure comprises a complex comprising: a) a nanoparticle-nucleic acid conjugate; a Cpfl polypeptide; and a guide RNA; and b) a polycation-based endosomal escape polymer. In some cases, a kit includes a recombinant expression vector that provides for *in vitro* production of a guide RNA.

In some cases, a kit of the present disclosure comprises a complex comprising: a) a nanoparticle-nucleic acid conjugate; a Cas9 polypeptide; a guide RNA; and a donor DNA; and b) a polycation-based endosomal escape polymer. In some cases, a kit of the present disclosure comprises a complex comprising: a) a nanoparticle-nucleic acid conjugate; a Cpfl polypeptide; a guide RNA; and a donor DNA; and b) a polycation-based endosomal escape polymer. In some cases, a kit includes a recombinant expression vector that provides for *in vitro* production of a guide RNA.

In some cases, a kit of the present disclosure includes a colloidal metal nanoparticle conjugated to a nucleic acid. In some cases, a kit of the present disclosure includes: a) a colloidal metal nanoparticle conjugated to a nucleic acid; and b) a Cas9 polypeptide. In some cases, a kit of the present disclosure includes: a) a colloidal metal nanoparticle conjugated to a nucleic acid; b) a Ca9 polypeptide; and c) a guide RNA. In some cases, a kit includes a recombinant expression vector that provides for *in vitro* production of a guide RNA.

A kit of the present disclosure can include one or more additional components, e.g., a buffer, a nuclease inhibitor, a protease inhibitor, and the like. A kit of the present disclosure can include a positive control and/or a negative control.

In addition to above-mentioned components, a subject kit can further include instructions for using the components of the kit to practice the subject methods. The instructions for practicing the subject methods are generally recorded on a suitable recording medium. For example, the instructions may be printed on a substrate, such as paper or plastic, etc. As such, the instructions may be present in the kits as a package insert, in the labeling of the container of the kit or components thereof (i.e., associated with the packaging or subpackaging) etc. In other embodiments, the instructions are present as an electronic storage data file present on a suitable computer readable storage medium, e.g. CD-ROM, diskette, flash drive, etc. In yet other embodiments, the actual instructions are not present in the kit, but means for obtaining the instructions from a remote source, e.g. via the internet, are provided. An example of this embodiment is a kit that includes a web address where the instructions can be viewed and/or from which the instructions can be downloaded. As with the instructions, this means for obtaining the instructions is recorded on a suitable substrate.

### EXAMPLES

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Celsius, and pressure is at or near atmospheric. Standard abbreviations may be used, e.g., bp, base pair(s); kb, kilobase(s); pl, picoliter(s); s or sec, second(s); min, minute(s); h or hr, hour(s); aa, amino acid(s); kb, kilobase(s); bp, base pair(s); nt, nucleotide(s); i.m., intramuscular(ly); i.p., intraperitoneal(ly); s.c., subcutaneous(ly); and the like.

Described below is a delivery vehicle, termed GX or CRISPR-Gold, which can efficiently deliver Cas9 protein, guide RNA and donor oligonucleotides into cells in culture and in vivo, and catalyze site specific DNA hydrolysis and recombination. CRISPR-Gold includes gold nanoparticles that are complexed with a ribonucleoprotein (RNP) comprising Cas9 and a guide RNA(s) and optionally also a donor DNA template, and may also optionally include an endosomal disruptive agent. CRISPR-Gold can be internalized by cells via endocytosis. After endocytosis, the endosomal disruptive agent (if present) releases the CRISPR-Gold into the cytoplasm. Glutathione in the cytoplasm can catalyze release of Cas9 RNP (and, if present, donor DNA) from the CRISPR-Gold through thiol exchange with the gold.

### MATERIALS AND METHODS

The following materials and methods were used in the Examples, below.

Materials. Oligonucleotides were purchased from Integrated DNA Technologies (IDT, Coralville, IA). Gold nanoparticles (15 nm) were purchased from BBI solutions. Sodium citrate and 4-(2-hydroxyethyl) piperazine-1-ethanesulfonate (HEPES) were purchased from Mandel Scientific (Guelph, ON). Sodium silicate and cardiotoxin were purchased from Sigma Aldrich (St. Louis, MO). Phusion High-fidelity DNA Polymerase was purchased from NEB (Ipswich, MA). The Megascript T7 kit, the Megaclear kit, PageBlue solution, propidium iodide, and the PureLink genomic DNA kit were purchased from Thermo Fischer (Waltham, MA). Mini-PROTEAN TGX Gels (4-20%) were purchased from Bio-Rad (Hercules, CA). MTeSR-1 media gentle cell dissociation reagent was purchased from StemCell Technologies (Vancouver, Canada). Matrigel was purchased from BD Biosciences (San Jose, CA). DMEM media, non-essential amino acids, penicillin-streptomycin, DPBS and 0.05% trypsin were purchased from Life Technologies (Carlsbad, CA). EMD Millipore Amicon Ultra-4 100kDa was purchased from Millipore (Germany).

### Expression and purification of Cas9

The full-length catalytically active *Streptococcus pyogenes* Cas9 was cloned into a custom pET-based expression vector encoding an N-terminal 6xHis-tag followed by maltose-binding protein (MBP) and a TEV protease cleavage site, as well as two SV40 nuclear localization signal (NLS) peptides at its C-terminus. Recombinant Cas9 protein was expressed in *Escherichia coli* strain BL21 (DE3) (Novagen) and further purified to homogeneity as previously described. Myers et al. (2013) Integrative biology : quantitative biosciences from nano to macro 5, 1495-1506. Purified Cas9 protein was stored in 50 mM HEPES pH 7.5, 300 mM NaCl, 10% glycerol, 100 µM TCEP at -80°C. *S. pyogenes* Cas9 D10A nickase was expressed and purified following the same procedure. Cas9 protein concentration was determined by a NanoDrop spectrophotometer from the absorbance at 280 nm.

### In Vitro T7 Transcription of sgRNA

Oligonucleotide primers for sgRNA production were purchased from IDT, with the forward primer containing a T7 promoter sequence. The DNA template for *in vitro* sgRNA transcription was prepared by overlapping PCR. Briefly, the T7 forward template (20 nM), T7Rev-Long template (20 nM), T7 forward primer (1 µM), and T7 reverse primer (1 µM), were mixed with Phusion Polymerase (NEB) and PCR amplification was performed according to the manufacturer's protocol. RNA *in vitro* transcription was performed with the MEGAscript T7 kit (Thermo Fisher) and purification of the resulting RNA was conducted using the MEGAclear kit, following the manufacturer's protocol. The transcribed sgRNA was eluted into 50 mM HEPES pH 7.5, 300 mM NaCl, 10% (vol/vol) glycerol, and 100 µM TCEP. The concentration of sgRNA was determined with a Nanodrop 2000 and the final sgRNA products were stored at -80 °C for subsequent experiments.

### Synthesis of PAsp(DET)

Poly{*N*-[*N*-(2-aminoethyl)-2-aminoethyl] aspartamide} (PAsp(DET)) was synthesized as previously reported. Kim et al. (2010) J. Control. Release 145:141; and Miyata et al. (2008) J. Am. Chem. Soc. 130:16287. Briefly, poly((β-benzyl L-aspartate) (PBLA) was synthesized by the ring-opening polymerization of the β-benzyl-L-aspartate *N*-carboxy-anhydride (BLA-NCA) with initiation by *n*-butylamine. The polymerization proceeded for 48 hours at 37 °C under an argon atmosphere. The degree of polymerization of the benzyl-L-aspartate (BLA) unit was calculated to be 55 from the ¹H NMR spectrum (DMSO-*d₆*, 80°C). The resulting PBLA was reacted with diethylenetriamine (DET) to obtain PAsp(DET). After one hour of reaction, the reaction mixture was added dropwise into cold HCl. The polymer product was purified by dialysis against 0.01 M HCl and then against deionized water overnight at 4 °C. The dialyzed solution was lyophilized to obtain the final product.

### Synthesis of CRISPR-Gold

A representative synthesis of CRISPR-Gold is described in this section. Gold nanoparticles (GNP) (15 nm in diameter, 450 nM) were reacted with a 5' thiol modified single stranded oligonucleotide (DNA-SH), 25 bases in length (200 µM), which had a region complementary to the donor DNA sequence, the reaction was performed in an Eppendorf tube in 20 mM HEPES buffer, in a 100 µL volume. The NaCl concentration of the reaction was increased a 100 mM per hour up to 400 mM (final volume 150 µL) by adding 1M NaCl solution, and the reaction was allowed to proceed overnight. Unconjugated DNA-SH was removed by centrifugation at 17,000 g for 15 min, and washed two times with 20 mM HEPES buffer. The resulting GNP-DNA conjugate was hybridized with the donor oligonucleotide, generating GNP-Donor. The donor DNA (100 µM concentration, 10 µL) was added to the GNP-DNA solution in 20mM HEPES with 50mM NaCl (100 µL), and incubated at 65 °C for 10 min, and gradually brought to room temperature (-2 °C/min). The GNP-Donor solution was stored at 4 °C until further use. CRISPR-Gold was synthesized by a layer-by-layer method, right before the *in vitro* and *in vivo* experiments. Cas9 (8 µg in 10 µL) and gRNA (2 µg in 10 µL) were mixed in 80 µL of Cas9 buffer (50 mM Hepes (pH 7.5), 300 mM NaCl, 10% (vol/vol) glycerol, and 100 µM TCEP) for 5 min at RT, and this solution was then added to the GNP-Donor solution (0.45 pmole of GNP), generating GNP-Donor-Cas9 RNP. Freshly diluted sodium silicate (60 mM, 2 µL) was added to the GNP-Donor-Cas9 RNP solution and PAsp(DET) was added to generate a final concentration of 100 µg/mL and incubated for 15 min at RT to form the last layer of CRISPR-Gold.

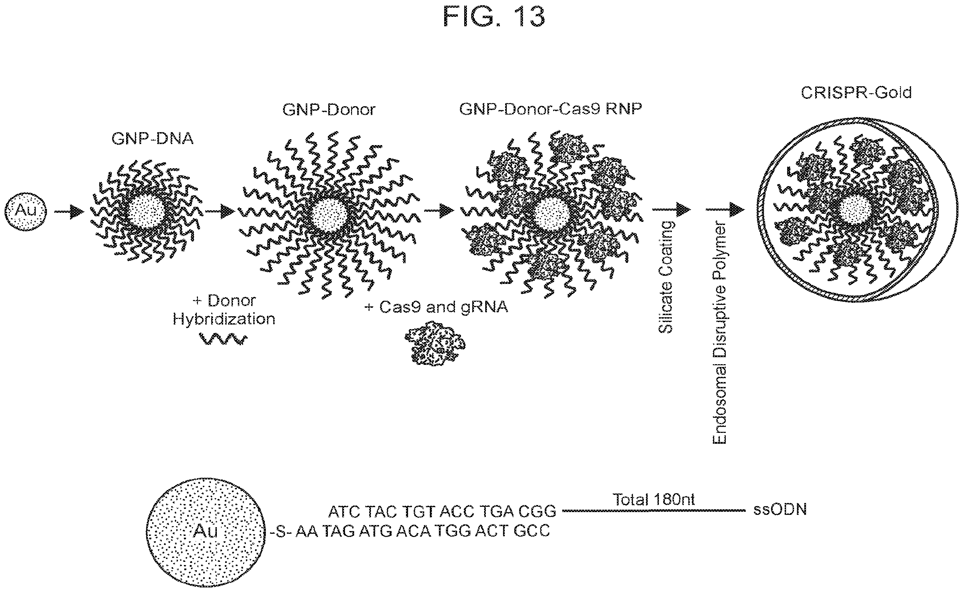

An example is shown in FIG. 13. Gold nanoparticles 15 nm in diameter were conjugated with a 5' thiol modified single stranded DNA (DNA-SH), and hybridized with single stranded donor DNA. A CXCR4 donor DNA sequence is presented as an example. The DNA-SH sequence is complementary to 18 nucleotides in the 3' end of the donor DNA. This CRISPR-Gold intermediate is sequentially complexed with Cas9 ribonucleoprotein (RNP), sodium silicate, and PAsp(DET) to form CRISPR-Gold.

### Absorbance spectra and zeta potential analysis of CRISPR-Gold

The synthetic intermediates in the synthesis of CRISPR-Gold, GNPs, GNP-DNA, GNP-DNA-donor DNA, GNP-Cas9 RNP and GNP-Cas9 RNP-Silicate were characterized by UV-vis spectroscopy. The absorbance spectra of each sample was measured with a UV-vis spectrophotometer (NanoDrop 2000, Thermo scientific). Zeta potential measurements were also made on each intermediate at 25 °C. Zeta potential measurements were made with a Zetasizer Nano ZS instrument (Malvern Instruments Ltd., Worcestershire, UK), and electrophoretic mobility was measured in a folded capillary cell (DTS 1060, Malvern Instruments), the zeta potential was calculated using the Smoluchowski equation.

Table 2 provides zeta potential analysis of CRISPR-Gold and its synthetic intermediates. Zeta potential measurements were performed on CRISPR-Gold and all of the synthetic intermediates generated during the construction of CRISPR-Gold. Zeta potential changes demonstrate the sequential synthesis of CRISPR-Gold. For example, GNP-Donor-Cas9 RNP-Silicate had a zeta potential of -18.9 mV and its surface charge density changes to +18.0 mV after the addition of the cationic polymer PAsp(DET).

**Table 2**

| **Samples** | **Zeta Potential (mV) ± S.D.** |
|---|---|
| GNP | -28.0 ± 14.2 |
| GNP-DNA | -24.1 ± 16.4 |
| GNP-Donor | -31.2 ± 15.8 |
| GNP-Donor-Cas9 protein | 0.8 ± 8.0 |
| GNP-Donor-Cas9 RNP | -13.2 ± 6.4 |
| GNP-Donor-Cas9 RNP-Silicate | -18.9 ± 5.2 |
| GNP-Donor-Cas9 RNP-Silicate-PAsp(DET) (CRISPR-Gold) | 18.0 ± 3.5 |

### Gel electrophoresis analysis of CRISPR-Gold to determine Cas9 protein content

The ability of CRISPR-Gold to complex Cas9 was determined via gel electrophoresis. 0.45 pmole of GNP-Donor was incubated with Cas9 (8 µg) and gRNA (2 µg) for 5 min at RT. The particles were centrifuged at 17,000 g for 10 min and the supernatant was removed. The pellet (GNP-Donor-Cas9 RNP) was washed with PBS and centrifuged at 17,000 g for 10 min, the supernatant and the pellets were collected and run on a gel, and analyzed for Cas9 content via densitometry, which demonstrated that GNP-Donor DNA binds Cas9 with high affinity. Similar analysis was performed on CRISPR-Gold. CRISPR-Gold was purified via centrifugation at 3,000 g for 5 min, and the particles and wash solution were analyzed via gel electrophoresis. Gel electrophoresis was performed using a 4-20% Mini-PROTEAN TGX Gel (Bio-rad) in Tris/SDS buffer, with loading dye containing 5% beta-mercaptoethanol. PageBlue solution (Thermo Fischer) staining was conducted and imaged with ChemiDoc MP using ImageLab software (Bio-rad). The percent of Cas9 RNP bound to the GNPs was determined by comparing the recovered Cas9 with the original amount mixed with the particles. The protein content in the particles was quantified via densitometry analysis on the respective gel bands.

### Enzymatic activity of Cas9 released from CRISPR-Gold

The enzymatic activity of Cas9 released from CRISPR-Gold was analyzed via gel electrophoresis. Purified samples of GNP-Cas9 RNP and CRISPR-Gold from S7 were prepared. They were incubated in 40 µL PBS containing 5 mM beta-mercaptoethanol at 37°C for 1 hour, to release Cas9 from the GNPs. The particles were centrifuged at 17,000 g for 10 min, and a 10 µL volume from the supernatants were collected and incubated with a PCR amplicon (200 ng) that contained a Cas9 cleavage site. After incubation at 37°C for 2 hr, the samples were analyzed by gel electrophoresis using a 4-20% Mini-PROTEAN TGX Gel (Bio-rad), stained with SYBR green (Thermo Fischer) and imaged with ChemiDoc MP using ImageLab software (Bio-rad).

### BFP expressing HEK cell culture

BFP-HEK cells were generated by infection of HEK293T cells with a BFP-containing lentivirus, followed by FACS-based enrichment, and clonal selection for cells expressing BFP with no silencing after 2-4 weeks. The lentivirus was generated by transfection of HEK293FT cells with a custom lentiviral vector containing a BFP gene driven by the pEF1 promoter, cloned into a Lenti XI DEST Blast backbone by Gateway cloning (Life Technologies, Inc.). Reporter cell lines were generated by infection of HEK293T cells with lentivirus, at low MOI (as estimated by FACS 3 days post-infection). BFP-positive cells were enriched by FACS, grown out, and sorted into clones by FACS. A clone with high constitutive BFP fluorescence (>99% BFP-positive) after expansion was selected as a reporter for BFP-GFP conversion by CRISPR-Gold-mediated HDR. To edit BFP-HEK to GFP, cells were plated at a density of 5×10⁴ cells per well in a 24 well plate, a day before CRISPR-Gold experiments, and cultured in DMEM with 10% fetal bovine serum (FBS), 1× MEM non-essential amino acids, and 100 µg/mL Pen Strep.

### Stem cell culture

Human H9 embryonic stem (hES) cells and human induced pluripotent stem (hiPS) cells were maintained in Matrigel-coated 6-well tissue culture plates. Cell culture plates were coated with Matrigel diluted to 12.5 µL/mL in DMEM and incubated for an hour at 37 °C¹. MTeSR-1 medium (StemCell Technologies) was added to the cells every day and the cells were passaged every 4-6 days. Gentle cell dissociation reagent (StemCell Technologies) was used for cell detachment, according to the manufacturer's protocol. Stem cells were passaged into 24 well plates, at a density of 2×10⁴ cells per well, 3 days prior to Cas9 transfection.

### Mouse primary bone marrow derived dendritic cell culture

Bone marrow cells were obtained from the tibias and femurs of mice. Bone marrow cells were plated in complete medium containing granulocyte-macrophage colony-stimulating factor (GM-CSF) (10 ng/mL; Peprotech) for 6 days to allow for differentiation into DCs. Cas9 transfection was conducted on Day 6.

### Cell transfection

For all of the gene editing experiments with CRISPR-Gold, electroporation, or lipofectamine, transfections were conducted on cells when they reached a density of 10⁵ cells per well (in 24 well plates). The sample sizes ranged between n = 3 to n = 6. This sample size was chosen based on previous papers on Cas9 protein delivery, which demonstrated that n = 3 to n = 6 was sufficient to generate statistically significant data.

### CRISPR-Gold treatment

CRISPR-Gold particles were formed as described above. For all of the in vitro cell experiments, 10⁵ cells in 0.5 mL were treated with 0.45 pmole GNP-Donor (determined by absorbance), Cas9 (8 µg), gRNA (2 µg), 2 µL sodium silicate (60 mM), and 10 µg PAsp(DET). Cas9 and gRNA solution were mixed in Cas9 buffer (50 mM Hepes (pH 7.5), 300 mM NaCl, 10% (vol/vol) glycerol, and 100 µM TCEP for 5 min at RT and added to the GNP-Donor solution. Freshly diluted sodium silicate (60 mM, 2 µL) was added to the GNP solution and incubated for 5 min at RT. The reaction mixture was centrifuged using an EMD Millipore Amicon Ultra-4 100 kDa at 3,000 rpm for 5 min to remove unbound sodium silicate. The recovered gold nanoparticles were resuspended in 20mM HEPES buffer (100 µL) and PAsp(DET) polymer was added to a final concentration of 100 µg/mL and incubated for 15 min at RT to form the last layer of CRISPR-Gold. In some cases, the concentration of donor DNA hybridized to CRISPR-Gold was varied between 0.5 µg to 6 µg per well treatment. CRISPR-Gold was added to the cells in fresh serum containing medium, and incubated for 16 hr and the medium was changed. The cells were incubated for a total of 3 days before genomic DNA extraction and analysis.

### Nucleofection

Cells were detached by 0.05 % trypsin or gentle dissociation reagent and spun down at 600 g for 3 min, and washed with PBS. Nucleofection was conducted using an Amaxa 96-well Shuttle system following the manufacturer's protocol, using 10 µL of Cas9 RNP and DNA donor (Cas9 : 100 pmole, gRNA : 120 pmole, DNA donor : 100 pmole). After the nucleofection, 500 µL of growth media was added and the cells were incubated at 37°C in tissue culture plates. The cell culture media were changed 16 hr after the nucleofection, and the cells were incubated for a total of 3 days before genomic DNA extraction and analysis.

### Lipofection

Lipofectamine transfection with Cas9 was performed following the protocol described in Zuris et al. *infra,* using 4.4 µg of Cas9, 1.2 µg of gRNA, and 1.2 µL of Lipofectamine 2000 in 100 µL total volume. Zuris et al. (2015) Nat. Biotechnol. 33:73. Additionally, donor DNA (250 ng) was mixed with lipofectamine (500 nL) and added to the transfection media, which contained the Cas9 RNP lipofectamine solution. The lipofection was conducted in OptiMEM media without serum, and an equal volume of 2x growth media was added to the cells after 1hr of lipofection to minimize cytotoxicity. The medium was changed 16 hr after the lipofection and the cells were incubated for a total of 3 days before genomic DNA extraction and analysis.

### Cellular uptake of CRISPR-Gold

The uptake of CRISPR-Gold in primary immune cells and bone marrow derived dendritic cells, obtained from the bone marrow of C57BL/6J mice, was determined. Bone marrow derived dendritic cells were cultured on Matrigel coated plates for imaging. CRISPR-Gold (0.45 pmole gold nanoparticle) was incubated with 10⁵ cells in culture media (500 µL) for 16 hr, and the media was changed. After washing 3 times, the cells were observed with a Zeiss inverted microscope and images were taken using Zen 2015 software. Additionally, the treated cells were detached and centrifuged at 600 g for 2 min in Eppendorf tubes, and imaged with a digital camera to determine changes in cell color.

### Flow cytometry analysis & fluorescence microscopy

Flow cytometry was used to quantify the expression levels of BFP and GFP in BFP-HEK cells treated with CRISPR-Gold. The flow cytometry experiment was performed on 2 independent times. The BFP-HEK cells were analyzed 7 days after Cas9 treatment. The cells were washed with PBS and detached by 0.05% trypsin. BFP and GFP expression was quantified using BD LSR Fortessa X-20 and Guava easyCyte™.

### Sanger sequencing of the BFP/GFP gene

The GFP+ population was sorted from BFP-HEK cells that had been treated with CRISPR-Gold (7 days after treatment). Cells were detached by 0.05% trypsin treatment and the GFP+ edited cells were sorted using a BD influx cell sorter (BD Biosciences) in the Berkeley flow cytometry facility. Genomic DNA was extracted from the GFP+ sorted cells and PCR amplification of the BFP/GFP gene was conducted. Sanger sequencing was conducted by Quintara Inc (CA, USA) and the sequence was analyzed with apE software.

### PCR amplification of genomic DNA from transfected cells

Genomic DNA of 2 × 10⁴ to 2 × 10⁵ cells was extracted 3 days after transfection using the Purelink genomic DNA kit (Thermo Fisher). The concentration of genomic DNA was measured with a Nanodrop spectrophotometer. The target genomic DNA sequences (BFP, CXCR, and dystrophin) were amplified using primer sets and Phusion polymerase with high efficiency (HE) or GC buffer according to the manufacturer's protocol. All primer sets were designed to anneal outside of the homology arms of the donor DNA in order to avoid amplifying the donor DNA. The PCR products were analyzed on a 1.5% (wt/vol) agarose gel casted with SYBR Safe (Thermo Fischer).

### Analysis of genome editing efficiency with restriction enzyme digestion and Surveyor assay

HDR was determined by the restriction enzyme digestion method and indel mutations were determined by the surveyor assay. The HDR efficiency in cells was determined with restriction enzyme digestion of PCR amplified target genes. Donor DNAs were designed to insert restriction enzyme sites, cleavable by either HindIII or DraI, into the target gene locus. The PCR amplicon of the CXCR4 locus and the PCR amplicon of the DMD locus were incubated with 10 units of HindIII and DraI, respectively. After 2 hr to 16 hr of incubation at 37 °C, the products were analyzed by gel electrophoresis using a 4-20% Mini-PROTEAN TGX Gel (Bio-rad) and stained with SYBR green (Thermo fischer). Individual band intensity was quantified using ImageJ and the HDR efficiency was calculated using the following equation: (b + c) / (a + b + c) × 100 (a = uncleaved PCR amplicon, b and c = the cleavage products). The surveyor assay was conducted to estimate the total DNA editing by cutting mismatched heteroduplex DNA from mutant DNA and wild-type DNA hybrid. Hybridization and SURVEYOR incubation were performed as described in Schumann et al. ((2015) Proc. Natl. Acad. Sci. USA 112:10437.

### Cell viability assays

The relative cell viabilities of cells transfected with CRISPR-Gold, nucleofection, and lipofection were determined with a cell counting kit (Dojindo) using regular culture media supplemented with 10% (v/v) CCK solution. The CCK assay was conducted 2 days after the transfection. Relative cell viability was defined as percent viability compared to untreated controls. An additional cell viability test was conducted on myoblasts using the propidium iodide assay. Staining of myoblasts treated with the various transfection methods was conducted 2 days after the transfection following the manufacturer's manual. Flow cytometry analysis was conducted with Guava easyCyte™.

### Sanger sequencing of hES edited with CRISPR-Gold

The CXCR4 PCR amplicon of CRISPR-Gold treated hES cells were cloned into plasmids using a Zero Blunt TOPO PCR cloning kit (Life Technologies), following the manufacturer's instruction. Briefly, TOP10 *E.coli* were transformed with plasmids containing the PCR amplicons and cultured on LB plates containing kanamycin. Sanger sequencing of the CXCR4 gene cloned into the *E.coli* colonies was conducted by Quintara Bioscience (CA, USA).

### In vivo delivery of CRISPR-Gold in mdx mice treated with cardiotoxin

Male C57BL/10ScSn (wild-type) mice and C57BL/10ScSn-*Dmdmdx*/J (mdx) mice that contain a nonsense mutation in exon 23 of the dystrophin gene were purchased from Jackson Laboratory. All animal studies were performed following authorized protocols and animals were treated in accordance with the policies of the animal ethics committee of the University of California at Berkeley and ACUC. Three groups of mdx mice were used for this experiment. The experimental groups were: Control (no GNP) = mice injected with Cas9 RNP and donor DNA without gold nanoparticle (n=3), Control (no gRNA) = mice injected with CRISPR-Gold without gRNA (n=1), CRISPR-Gold = mice injected with CRISPR-Gold, which received CRISPR-Gold with all materials (n=3).

CRISPR-Gold treatments were performed in 2 month old mdx mice in the tibialis anterior (TA) muscle (10 µL per muscle) and gastrocnemius muscles (10 µL per muscle), using a Hamilton syringe. The injection mix contained 10 µL of cardiotoxin (0.1mg/ml) mixed with 0.1mg/ml lidocaine hydrochloride. Two weeks after the injection, the muscles were harvested and analyzed.

CRISPR-Gold particles were formed as described above. For all mdx *in vivo* experiments, 6.75 pmole GNP, 120 µg Cas9, 30 µg gRNA, 30 µL sodium silicate (60mM), and 150 µg PAsp(DET) were injected per mouse. CRISPR-Gold particles were concentrated to a 60 µL total volume, which was distributed between the two hind legs, each hind leg received a total of 30 µL in 6 injection sites. Controls were injected with the same protocol. The experiments were conducted non-blinded and in a non-randomized way.

### Sequence of genomic DNA targeted in the mdx mouse and the sequence of the donor DNA

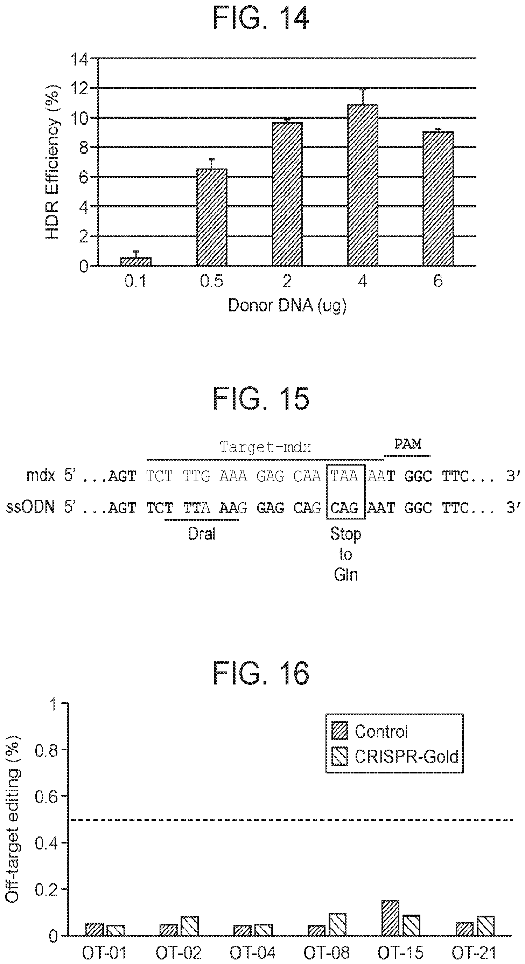

As shown in FIG. 15, donor DNA was designed to replace the stop codon in the mutated dystrophin gene. Non-sense mutation and donor DNA sequence designed to repair the mutation are marked in the pink box, nucleotides marked in green (A, G, G) are silent mutations that prevent Cas9 activity on the edited sequence. Repair with the donor DNA sequence generates a DraI restriction enzyme site (TTTAAA), which is used for HDR analysis.

### In vivo delivery of CRISPR-Gold in mdx mice without cardiotoxin treatment

Male C57BL/10ScSn (wild-type) mice and C57BL/10ScSn-*Dmdmdx*/J (mdx) mice that contain a nonsense mutation in exon 23 of the dystrophin gene were purchased from Jackson Laboratory. All animal studies were performed following authorized protocols and animals were treated in accordance with the policies of the animal ethics committee of the University of California at Berkeley. Two groups of mdx mice were used for this experiment. The experimental groups were: Negative Control = mdx mice without injection (n=6), Control (scrambled CRISPR-Gold) = mice injected with CRISPR-Gold with scrambled gRNAc (n=9), CRISPR-Gold = mice injected with CRISPR-Gold, which received CRISPR-Gold with all materials (n=9). The injections were conducted in 3 week old mdx mice in the tibialis anterior (TA) muscle (10 µL per muscle), gastrocnemius muscle (10 µL per muscle), and forelimb muscle (10 µL per muscle) using a Hamilton syringe. Two weeks after the injection, the mice received a second round of injections with exactly the same composition. Two weeks and 3 months after the second injection, mice were sacrificed and the muscles were analyzed by deep sequencing and for dystrophin protein.

A four limb hanging test was conducted on CRISPR-Gold treated mdx mice, at the age of 5 weeks, which is two weeks after the initial injection. Mice were placed on a hand made square apparatus with a grid structure. The apparatus was inverted and positioned 25 cm up from the cage to discourage intentional dropping. Soft bedding was prepared to prevent the mice from harming themselves if they fell. The maximum hanging time out of three trials was recorded for a duration of 600 sec. A fixed hanging limit was set at 600 sec. The maximum hanging time was divided by weight. The wild type mice were also tested at the age of 5 weeks. An unpaired student t test was conducted using Prism 7 software. The experiments were conducted in a blinded manner.

### Deep sequencing analysis of CRISPR-Gold treated muscle tissue

The genomic region of the Cas9 target sequence was amplified by PCR using Phusion high-fidelity polymerase according to the manufacturer's protocol. Target genes were amplified first with primer sets used for HDR detection and amplified again with deep sequencing primers to eliminate the potential of donor sequence amplification. The amplicons were purified using the ChargeSwitch PCR clean-up kit (Thermo Fischer). The Nextflex rapid illumine DNA-seq library prep kit was used to attach illumine adapters and PCR amplify the product for five cycles. PCR clean-up was performed one additional time. The Berkeley Sequencing facility performed DNA quantification using a Qubit 2.0 Fluorometer (Life Technologies, Carlsbad, CA). BioAnalyzer for size analysis and qPCR quantification followed. The library was sequenced with the Illumina HiSeq2500 in the Vincent Coates Genomic Sequencing Laboratory at UC Berkeley. The analysis was conducted using the CRISPR Genome Analyzer. Kim et al. (2010) J. Control. Release 145, 141-148.

### Immunohistochemistry of dystrophin protein

Collected gastrocnemius and tibialis anterior muscles were frozen sectioned and fixed with 4% paraformaldehyde for 15 min at RT. After blocking for 1hr, a primary antibody against dystrophin (Santa Cruz Biotechnology-47760 or 358922) was incubated with the samples overnight. After extensive washing with PBS, a secondary antibody (Santa Cruz Biotechnology-2005 or 362282) was incubated with the samples for 1 hr at RT. The samples were stained with DAPI staining and analyzed via microscopy. Laminin staining was conducted with same method using a primary antibody against laminin (Santa Cruz Biotechnology-74418) and secondary antibody (Santa Cruz Biotechnology-362257).

### High throughput imaging of a whole muscle section

Glass slides with whole muscle sections stained for dystrophin protein were imaged with Molecular Devices ImageXpress Micro. On average, about 288 images were taken per slide with a 10 x objective lens. The images were analyzed using MetaXpress software to merge the images and create a montage of the whole muscle sections.

### PCR amplification of genomic DNA from CRISPR-Gold edited muscle tissue

Muscle genomic DNA from either control mice (Cas9 RNP + donor DNA without GNP) or CRISPR-Gold treated mice was amplified with primers designed to only amplify the HDR edited sequence. PCR was conducted using the forward primer (AAAGGAGCAGCAGAATGGCT; SEQ ID NO: 1124), the reverse primer (CCACCAACTGGGAGGAAAG; SEQ ID NO: 1105), and Phusion polymerase with high GC buffer according to the manufacturer's protocol. The PCR products were analyzed on a 1.5% (wt/vol) agarose gel casted with SYBR Safe (Thermo fischer).

### Off-target deep sequencing analysis

Deep sequencing was performed on CRISPR-Gold treated mdx mice (with CTX and without CTX) to investigate the frequency of off-target genomic damage. Potential off-target loci were determined using CRISPR off-target prediction programs. PCR was conducted using primers listed in Table 3.

**Table 3 - Off-target primers**

| | |
|---|---|
| **OT-01_F** | TATGCCACTTCTTCAAAGAGATGAT (SEQ ID NO: 1125) |
| **OT-01_R** | AACAAGCAAACAATTCAAAGGATAG (SEQ ID NO: 1126) |
| **OT-02_F** | AAGAAGATATGGCATTGCTGGTA (SEQ ID NO: 1127) |
| **OT-02_R** | TCTGGAAACAAAAAGGCAATG (SEQ ID NO: 1128) |
| **OT-04_F** | CTATGAGTTTACCACCCTAATGTGC (SEQ ID NO: 1123) |
| **OT-04_R** | CTTATGCTTGTTCAGGCAAATACC (SEQ ID NO: 1130) |
| **OT-08_F** | TTTCTTGGAGCTGTAGTGTGTACTG (SEQ ID NO: 1131) |
| **OT-08_R** | GGAATAGAGTGAGCATTGTTCTGAT (SEQ ID NO: 1132) |
| **OT-15_F** | TTAAGCGGAAAGATAAGCTGAAGTA (SEQ ID NO: 1133) |
| **OT-15_R** | GGACCAATGTTACTGGAACACATAC (SEQ ID NO: 1134) |
| **OT-21_F** | CATGAAGATACAGAAACATCCCAGT (SEQ ID NO:1135) |
| **OT-21_R** | GGAGTGGCACCCTCCTTAC (SEQ ID NO: 1136) |

The amplicons were purified using the ChargeSwitch PCR clean-up kit (Thermo Fischer). The Nextflex rapid illumine DNA-seq library prep kit was used to attach illumine adapters and PCR amplify the product for five cycles. PCR clean-up was then performed a second time. The Berkeley Sequencing facility performed DNA quantification using a Qubit 2.0 Fluorometer (Life Technologies, Carlsbad, CA). BioAnalyzer for size analysis and qPCR quantification followed. The library was sequenced with the Illumina HiSeq2500 in the Vincent Coates Genomic Sequencing Laboratory at UC Berkeley using 150PE read. The analysis was conducted using CRISPR Genome Analyzer (54.80.152.219). Figure 16 presents the off-target mutation frequency of control (scrambled CRISPR-Gold injected mouse) and the CRISPR-Gold injected mouse samples (without CTX). Table 4 presents the off-target frequency of the control (naked Cas9 RNP and CTX injected mouse) and CRISPR-Gold injected mice (with CTX).

**Table 4**

| | **Control** | **Average Reads** | **CRISPR-Gold** | **Average Reads** |
|---|---|---|---|---|
| OT-01 | **0.573** | 1449020 | **0.172** ± 0.008 | 2122047 |
| OT-02 | **0.009** | 1455510 | **0.009** ± 0.001 | 1417348 |
| OT-04 | **0.008** | 1881566 | **0.005** ± 0.001 | 1606093 |
| OT-08 | **0.042** | 3496042 | **0.023** ± 0.008 | 326289 |
| OT-15 | **0.01** | 1347557 | **0.083** ± 0.066 | 1512108 |
| OT-21 | **0.009** | 3579136 | **0.011** ± 0.003 | 2569223 |

Table 4. CRISPR-Gold causes insignificant levels off-target mutations, which are in the range of deep sequencing error. The major predicted and reported off-target sites for the mdx gRNA were analyzed with deep sequencing to check for off-target mutations. The off-target mutations from the negative control composed of CRISPR-Gold with a scrambled gRNA and CRISPR-Gold are compared in the table. Every sample had more than 30,000 deep sequencing reads. CRISPR-Gold treated mice had very low levels of off-target site indel mutations, which were generally below the accuracy of deep sequencing. CRISPR-Gold samples (mean ± S.E, n=2).

Table 5 provides primers for HDR analysis

**Table 5**

| **Primers for HDR analysis** | |
|---|---|
| **mdx** | |
| Forward | GAGAAACTTCTGTGATGTGAGGACATATAAAG; (SEQ ID NO: 1100) |
| Reverse | CAATATCTTTGAAGGACTCTGGGTAAAATATC; (SEQ ID NO: 1101) |

| **CXCR** | |
|---|---|
| Forward | TTAATTCTCTTGTGCCCTTAGCCCACTACTTCAG; (SEQ ID NO: 1102) |
| Reverse | GGACAGGATGACAATACCAGGCAGGATAAGGCC; (SEQ ID NO: 1103) |

Table 6 provides sequencing primers

**Table 6**

| **Sequencing Primers** | |
|---|---|
| **mdx** | |
| Forward | GCGTGTTAGTGTAAATGAACTTCTA; (SEQ ID NO: 1104) |
| Reverse | CCACCAACTGGGAGGAAAG; (SEQ ID NO: 1105) |

| **CXCR** | |
|---|---|
| Forward | GAGAAGCATGACGGACAAGTACAGGCTG; (SEQ ID NO: 1106) |
| Reverse | TTGACTGTGTAGATGACATGGACTGCCT; (SEQ ID NO: 1107) |

| **BFP** | |
|---|---|
| Forward | TGTCCGGCGAGGGCGAGGGCGAT; (SEQ ID NO: 11 08) |
| Reverse | CGTCCTTGAAGAAGATGGTGCGC; (SEQ ID NO: 1109) |

Table 7 provides thiol oligonucleotides for conjugation to gold nanoparticles (GNP)

**Table 7**

| **Thiol oligonucleotides for conjugation to GNP** | | |
|---|---|---|
| BFP-SH | /5ThioMC6-D/GCGCTCCTGGACGTAGCCTTCGGGCAT; (SEQ ID NO:1110) | |
| mdx-SH | /5ThioMC6-D/ AAA TTCTGACAGA T A TTTCTGGCA T A TTTC; (SEQ ID NO:1111) | |
| CXCR4_SH | /5ThioMC6-D/AATAGATGACATGGACTGCC; (SEQ ID NO: 1112) | |

Table 8 provides nucleotide sequence of donor DNA

**Table 8**

| | |
|---|---|
| **MDX** | TGATATGAATGAAACTCATCAAATATGCGTGTTAGTGTAAATGAACTTCTAT TTAATTTTGAGGCTCTGCAAAGTTCTTTAAAGGAGCAGCAGAATGGCTTCA ACTATCTGAGTGACACTGTGAAGGAGATGGCCAAGAAAGCACCTTCAGAA |
| | ATATGCCAGAAATATCTGTCAGAATTT (SEQ ID NO: 1113) |
| **CXC R** | CCTGGTCATGGGTTACCAGAAGAAACTGAGAAGCATGACGGACAAGTACA GGCTGCACCTGTCAGTGGCCGACCTCCTAAGCTTGGATCCCTTTGTCATC ACGCTTCCCTTCTGGGCAGTTGATGCCGTGGCAAACTGGTACTTTGGGAA CTTCCTATGCAAGGCAGTCCATGTCATCTA (SEQ ID NO: 1114) |
| **BFP** | GATGCCACCTACGGCAAGCTGACCCTGAAGTTCATCTGCACCACCGGCAA GCTGCCCGTGCCCTGGCCCACCCTCGTGACCACCGTGACGTACGGCGTG CAGTGCTTCAGTCGCTACCCCGACCACATGAAGCAGCACGACTTCTTCAA GTCCGCCATGCCCGAAGGCTACGTCCAGGAGCGC (SEQ ID NO: 1115) |

Table 9 provides nucleotide sequence of sgRNA T7 template forward primer sequences

**Table 9**

| | |
|---|---|
| **CXCR** | GATCCTAATACGACTCACTATAGAAGCGTGATGACAAAGA GGGTTTTAGAGCTAGAAATAGCAAGTTAAAATAAGGC (SEQ ID NO: 1116) |
| **mdx** | GATCCTAATACGACTCACTATAGGTCTTTGAAAGAGCAATA AAAGTTTTAGAGCTAGAAATAGCAAGTTAAAATAAGGC (SEQ ID NO: 1117) |
| **GFP_L2** | GATCCTAATACGACTCACTATAGCTGAAGCACTGCACGCC ATGTTTTAGAGCTAGAAATAGCAAGTTAAAATAAGGC (SEQ ID NO: 1118) |
| **CXCR_F1 (Cas9-nickase)** | GATCCTAATACGACTCACTATAGTACAGGCTGCACCTGTC AGGTTTTAGAGCTAGAAATAGCAAGTTAAAATAAGGC (SEQ ID NO: 1119) |
| **DMD** | GATCCTAATACGACTCACTATAGTCTTTGAAAGAGCAACAA AAGTTTTAGAGCTAGAAATAGCAAGTTAAAATAAGGC (SEQ ID NO: 1120) |
| **Ai9_F** | GGATCCTAATACGACTCACTATAgAAAGAATTGATTTGATAC CGGTTTTAGAGCTAGAAATAGCAAGTTAAAATAAGGC (SEQ ID NO: 1121) |
| | GGATCCTAATACGACTCACTATAgGTATGCTATACGAAGTTA TTGTTTTAGAGCTAGAAATAGCAAGTTAAAATAAGGC (SEQ ID NO: 1122) |

### Example 1: Loading Gold Nanoparticles with Active Cas9/gRNA Ribonucleoproteins (RNPs)

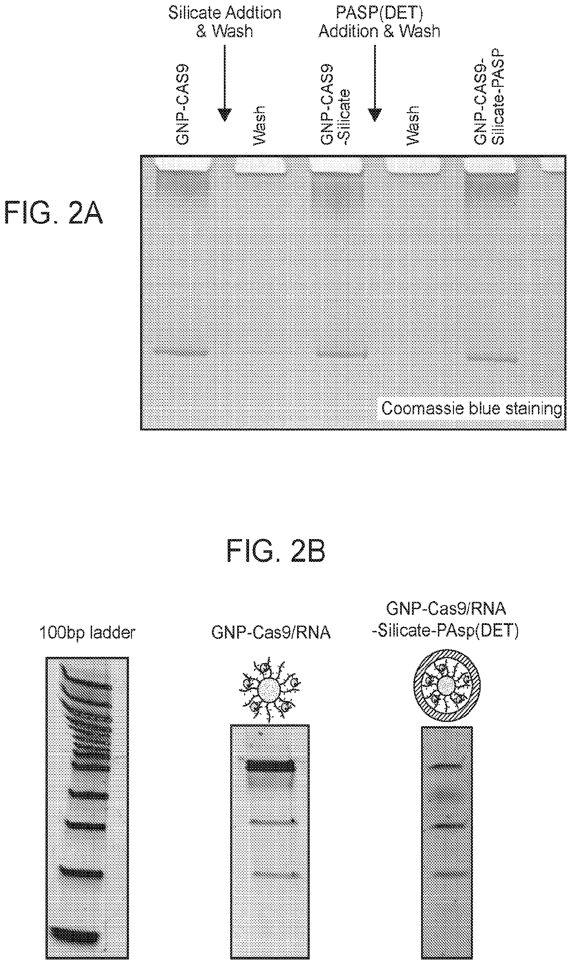

30 nm gold nanoparticles were reacted with thiol modified DNA, and then complexed with Cas9 and guide RNA. Then, to ensure that the Cas9-gold nanoparticles have endosomal disruptive capability, a layer by layer approach was used to encapsulate the Cas9 in consecutive layers of silicate and the endosomal disruptive polymer, (poly{*N*-[*N*-(2-aminoethyl)-2-aminoethyl]aspartamide} (PAsp(DET)). The degree of complexation was assessed by centrifuging the gold nanoparticles and quantifying the Cas9 that was pulled out of solution; the recovered Cas9 was also assayed for activity via cleavage of a vascular endothelial growth factor (VEGF) template DNA. FIGs. 2A and 2B demonstrate that DNA modified gold nanoparticles (GNPs) complexed with Cas9 with extremely high affinity, and that the complexed Cas9 was active against target DNA.

**FIGs. 2A and 2B****. Cas9/gRNA RNP loaded onto GNP and released with activity in reducing conditions.** (FIG. 2A) Cas9 loading gel shows efficient Cas9 loading to GNP. (FIG. 2B) In vitro cleavage gels shows that Cas9/gRNA RNP is released with good activity. Model VEGF DNA template is cleaved into two fragments by Cas9/gRNA RNP released from GNP with and without polymer layers.

### Example 2: Cas9 Delivery and Gene Editing Using GX (Cas9-Gold Nanoparticle Complexes)

Experiments were performed to determine if Cas9 delivered by gold could cause site-specific DNA cleavage in cells, using blue fluorescent protein (BFP)-expressing HEK293 cells.

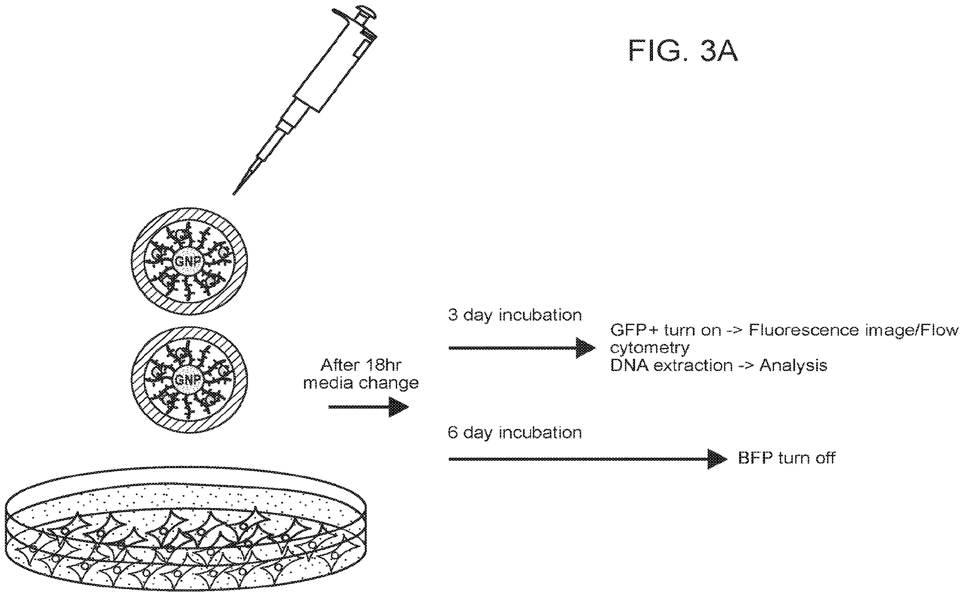

FIG. 3A depicts a schematic of protocol for testing *in vitro* Cas 9 delivery and activity by GNPs.

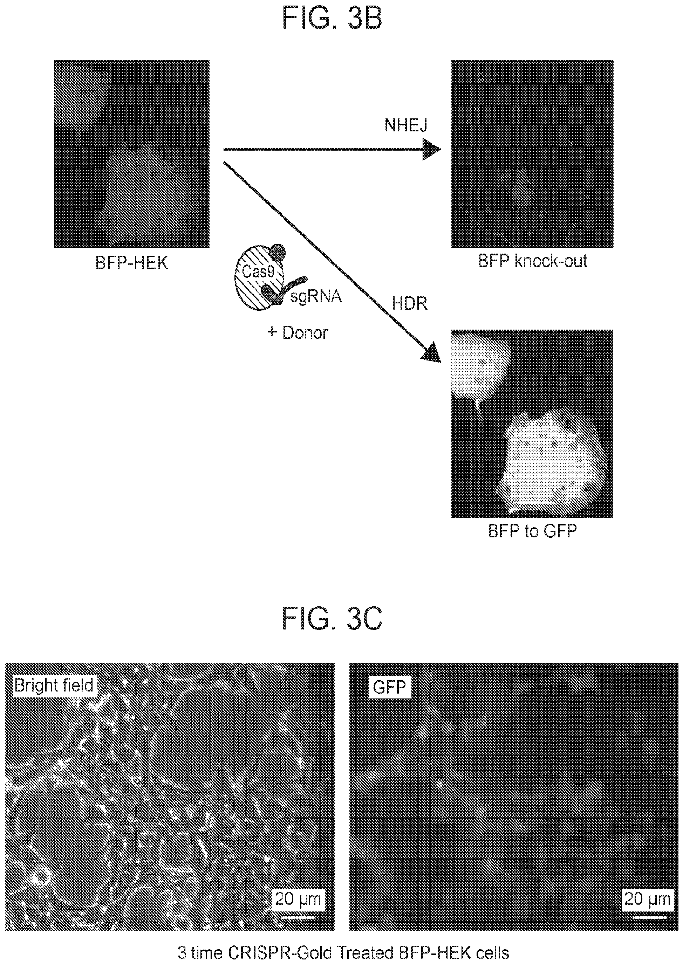

FIG. 3B. *In vitro* CRISPR-Gold treatment shows efficient editing in BFP-HEK cells. Scheme of BFP-HEK cell editing. BFP gene targeting with Cas9/gRNA produces two types of editing: non-homologous end joining (NHEJ) and homology directed repair (HDR). NHEJ in BFP-HEK cells frequently induces BFP knock-out and HDR with donor sequence converts BFP to GFP.

FIG. 3C. Fluorescence images show GFP expression from Gold-Cas9/donor treated BFP-HEK cells. Three times of CRISPR-Gold treatment induced GFP expression from the treated BFP-HEK cells. Left image is a bright field image. Right image is taken with GFP filter. Scale bar is 20 µm.

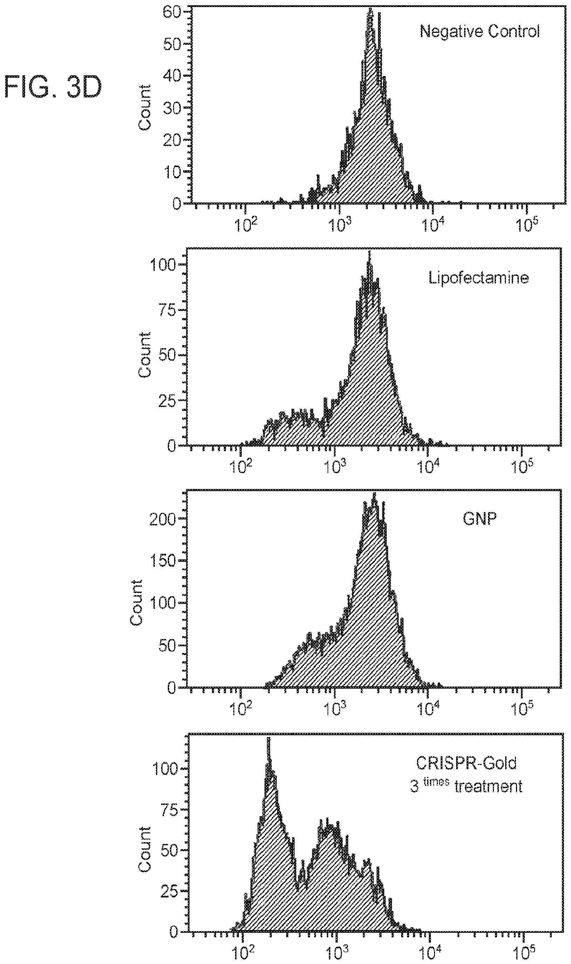

FIG. 3D-3E demonstrate that Cas9-gold nanoparticles can effectively deliver Cas9 into cells and induce gene editing in HEK293 cells. For example, HEK293 cells treated with Cas9-gold resulted in a new population, which were YFP negative and represented 10% of the total population, thus demonstrating that an insertion/deletion (indel) mutation occurred in these cells. Importantly, Cas9 gold nanoparticles had a Cas9 delivery efficiency that was comparable to conventional protein transfection reagents, such as Lipofectamine®, yet Cas9-gold nanoparticles had no toxicity up to a concentration of 100 micromoles/liter, which was significantly lower than other commonly used transfection reagents, such as Lipofectamine®. These results suggested that Cas9 gold can be given to cells, multiple times, to increase efficiency. Therefore, it was investigated if multiple delivery of Cas9 via GX would be able to increase the Cas9 delivery and mutation frequency.

The data presented in FIG. 3D show that BFP knock-out population appears 6 days after CRISPR-Gold (without donor) treatment to the BFP-HEK cells. Lipofectamine transfection was conducted as a positive control. Flow cytometry analysis shows that three times of CRISPR-Gold treatment generates very clear BFP knock-out population. FIG. 3D (bottom) demonstrates that treating cells with GX three times dramatically increased mutation efficiency to 50% after 3 treatments.

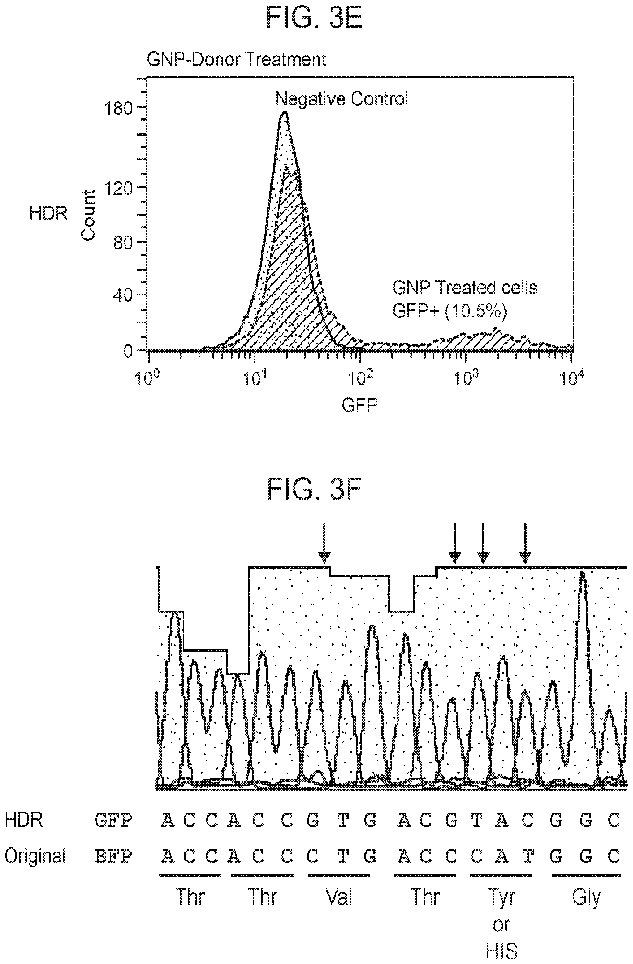

FIG. 3E. BFP gene editing to GFP was observed with flow cytometry from the Gold-GNP/donor treated BFP-HEK cells. Compared to negative control showing no GFP+ population, Gold-GNP treatment induced GFP expressing population from the BFP-HEK cells.

FIG. 3F. BFP to GFP genome editing was observed from sequencing result. GFP expressing cells from the CRISPR-Gold treated BFP-HEK cells were FACS sorted and sequence. Compared to the original BFP sequence, GFP expressing cells had 4 nucleotide editing, same as the donor DNA sequence, substituting histidine to tyrosine. Two other sequence editing was silent mutation to prevent Cas9 activity on the edited sequence.

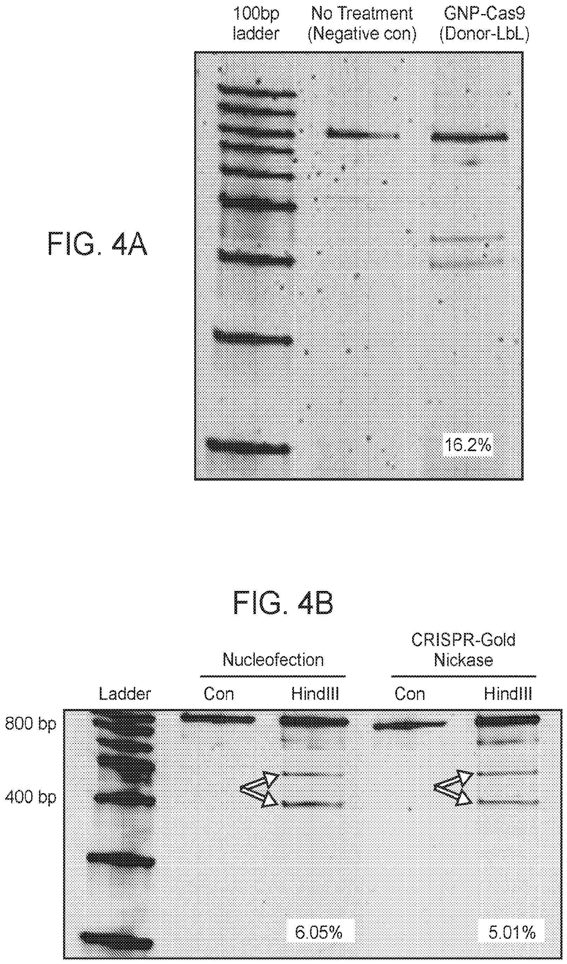

Experiments were performed to determine if gold nanoparticles could deliver Cas9, guide RNA and donor oligonucleotides into cells and generate homology directed repair (HDR) in target cells. For these experiments a guide was designed that cut the VEGF-R gene and a donor oligonucleotide was designed that introduced a HindIII nuclease site. Initial experiments were performed on HEK 293 cells to determine if the multifunctional gold nanoparticles could stimulate HDR. FIG. 4A demonstrates that gold nanoparticles were very efficient at catalyzing HDR, and approximately 15-16 % of the targets were HDR positive.

**FIG. 4A****. Endogenous genes are edited with Gold-GNP.** CRISPR-Gold edited CXCR gene in HEK cells. A restriction enzyme HindIII cut site was incorporated into the CXCR4 gene and HDR efficiency was quantified by CXCR4 PCR cleavage by HindIII. 16.2% HDR was achieved with Gold-Cas9/donor.

### Example 3: Cas9 Delivery and Gene Editing in Embryonic Stem Cells and Primary Myoblasts Using GX (Cas9-Gold Nanoparticle Complexes)

Experiments were performed to determine if gold nanoparticles could deliver Cas9, guide RNA and donor DNA into human embryonic stem (hES) cells and induce HDR. FIG. 4B demonstrates that Cas9-gold nanoparticles were exceptionally efficient at inducing HDR in embryonic stem cells. For example, Cas9-gold generated HDR in 4.5% of target cells, which was comparable to electroporation. Importantly, Lipofectamine® had only a 0.1% HDR efficiency in these cells, and gold nanoparticles were thus much better at generating HDR than conventional protein transfection reagents.

**FIG. 4B****.** Gold-Cas9/donor efficiently edited CXCR gene in human embryonic stem (hES) cells. Nucleofection was conducted as a positive control with twice or more amounts of Cas9, gRNA, and donor DNA than Gold-Cas9. Gold-Cas9/donor DNA delivery edited hES cells and Cas9-Nickase induced 5.01% HDR, which was comparable to the HDR efficiency of nucleofection.

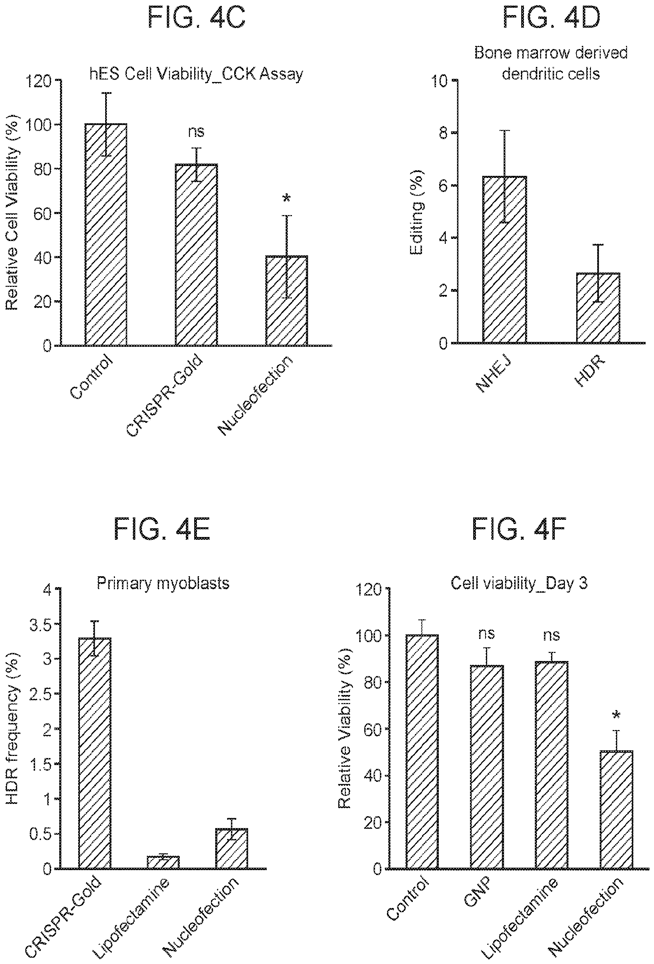

**FIG. 4C****.** CRISPR-Gold keeps high cell viability compared to nucleofection. Nucleofection caused 60% of cell death 2 days after the transfection. However, CRISPR-Gold maintained comparable cell viability to untreated control, mean ± S.E, n=6. *, p < 0.05, ns = statistically not significant to control.

**FIG. 4D****.** Primary mouse bone marrow derived dendritic cells were edited with CRISPR-Gold. The dendritic cells are suspension cells freshly isolated from mouse bone marrow. CRISPR-Gold induced 6% of NHEJ and 3% of HDR in target dystrophin sequence.

Finally it was investigated if gold nanoparticles could deliver Cas9 and donor oligonucleotides to muscle cells, e.g., myoblasts that contain the muscular dystrophy mutation, obtained from the MDX mouse, and catalyze HDR in muscle cells. FIG. 4E-4F demonstrate that GX can efficiently deliver Cas9, guide RNA and donor DNA into myoblasts with minimal toxicity. For example, a gene correction rate of 3.5% was observed with GX, which was higher than electroporation. In addition, under these conditions, GX had minimal toxicity, whereas electroporation had approximately 50% toxicity.

**FIG. 4E-4F.** (FIG. 4D) Primary myoblasts from mdx mouse were efficiently edited with Gold-Cas9/donor. Point mutation of the mouse myoblasts was edited to normal sequence with efficiency close to 3.3%, which is significantly higher percentage than the HDR efficiency of nucleofection. mean ± S.E, n=3. *, p < 0.05. (FIG. 4F) Compared to very low cell viability of nucleofected primary myoblasts, Gold-Cas9 and Lipofectamine® treated cells had comparable cell viability to the cells without any treatment, mean ± S.E, n=6. *, p < 0.05, ns = statistically not significant to control.

### Example 4: In Vivo Delivery of Cas9 by Gold Nanoparticles

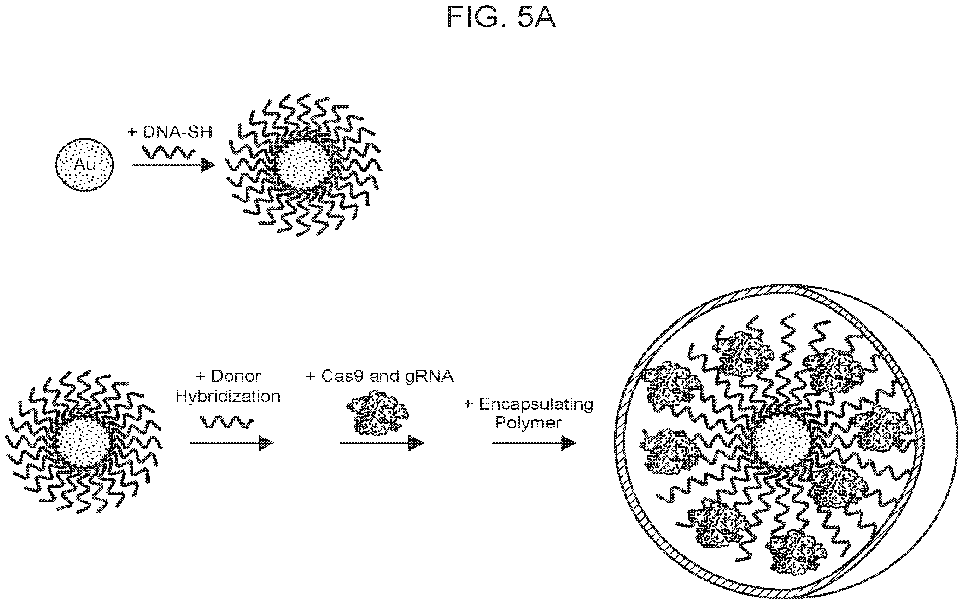

**FIG. 5A****.** Synthetic scheme of CRISPR-Gold. DNA-thiol conjugated to Gold Nanoparticle (GNP) is hybridized with donor single strand DNA. Cas9/gRNA ribonucleoproteins (RNPs) are loaded and the particle is coated with Silicate and PASp(DET) polymer, which helps cellular uptake and endosomal escape.

Experiments were performed to determine if Cas9 labeled with Alexa 647 and delivered by gold nanoparticles can be internalized by muscle tissue after an intramuscular injection.

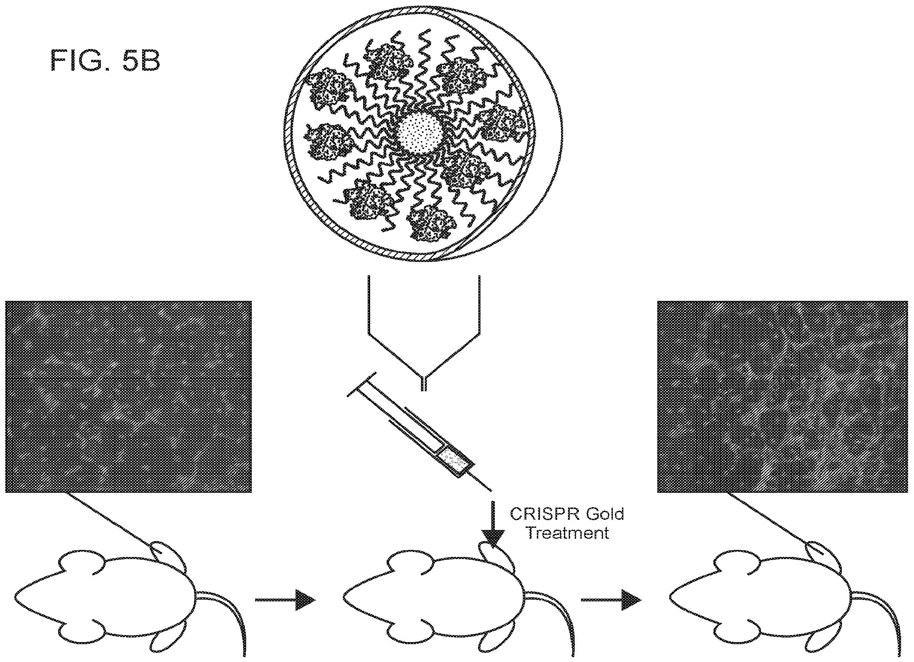

**FIG. 5B** is a schematic depiction of the experiment.

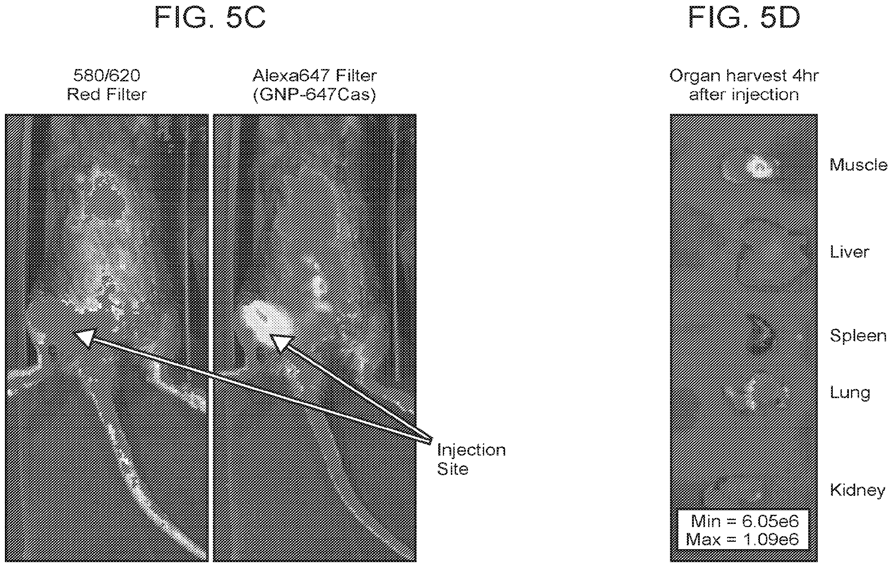

FIG. 5C and 5D demonstrate that gold nanoparticles can deliver Cas9, guide RNA and donor DNA into muscle cells after an intramuscular injection. For example, Cas9 delivered via gold nanoparticles had approximately 60% retention in the muscle tissue after injection.

**FIG. 5C-5D****. In vivo delivery of Gold-Alexa647 Cas9 shows retention of Alexa647 Cas9 in the muscle injection site.** (FIG. 5C) IVIS® image of Gold-Alexa647 Cas9 injected mouse 4 hr after injection shows significant Alexa647 signal from the muscle injection site. Ex580/Em620 filter shows that there was no auto-fluorescence. Ex640/em680 filter showed significant fluorescence in the injection site. (FIG. 5D) Injected Gold-Alexa647 Cas9 retained in muscle. Organs were harvested 4 hr after injection and imaged with IVIS®. Strong fluorescence was observed only from the muscle.

### Example 5: Activity of Cas9 Delivered In vivo by Gold Nanoparticles

Experiments are performed to determine if GX can deliver functional Cas9, donor DNA and guide RNA and correct the MDx mutation in mice and regenerate functional muscle tissue. Mice are injected with Cas9, donor DNA and gold nanoparticles, using guide RNA that target the MDx mutation. Two weeks later the mice are harvested and analyzed for dystrophin protein production, via histology.

### Example 6: Correction of DMD-mdx dystrophin in vivo

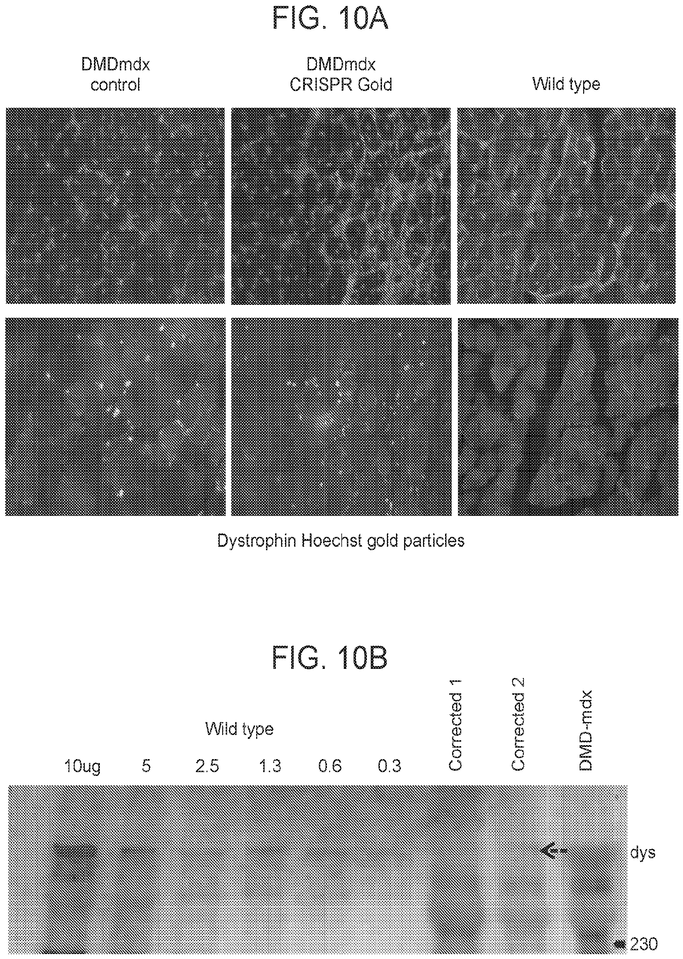

**CRISPR-gold particles correct DMD-mdx dystrophin in vivo.** Mutation-correcting nanoparticles along with cardiotoxin were injected into young (2 month old) DMD-mdx TA muscle (2 sites of 5 µl) and gastrocnemius muscles (four sites of 5 µl), or into the same sites in wild-type mice; the mice were allowed to heal for 2 weeks. Particles lacking cas9 were used as a negative control. Muscle was dissected, sectioned, and immunostained. The results are depicted in FIG. 10A-10B. Sectioned muscle was immunostained for Dystrophin (red, FIG. 10A) with Hoechst staining nuclei blue. Nanoparticle aggregates auto-fluoresce on the green and red (but not blue) channels. Dystrophin protein (lacking in DMD/MDX) becomes expressed in most/all muscle fibers (myofibers) at the site of CRISPR-gold particles injection after a single application (red outlines of the myofibers show re-expressed dystrophin protein, which is in the same area of muscle as the auto-fluorescent CRISPR-gold particles). Negative control particles, which had no Cas9 did nor restore the expression of dystrophin. Positive control shows dystrophin expression and localization in the wild type mouse muscle.

As depicted in FIG. 10B, muscle sections were solubilized and denatured in Laemmli buffer, resolved by sodium dodecyl sulfate-polyacrylamide gel electrophoresis (SDS-PAGE) and Western blotted for dystrophin protein. A serial dilution of wild-type muscle protein was loaded to determine the amount of corrected dystrophin protein. Qualitatively, the amount of corrected dystrophin by Western analysis correlates with the muscle cross-sectional area of dystrophin staining seen by immunofluorescent histology.

### Example 7: Translational potential of CRISPR-Gold as a therapeutic for DMD

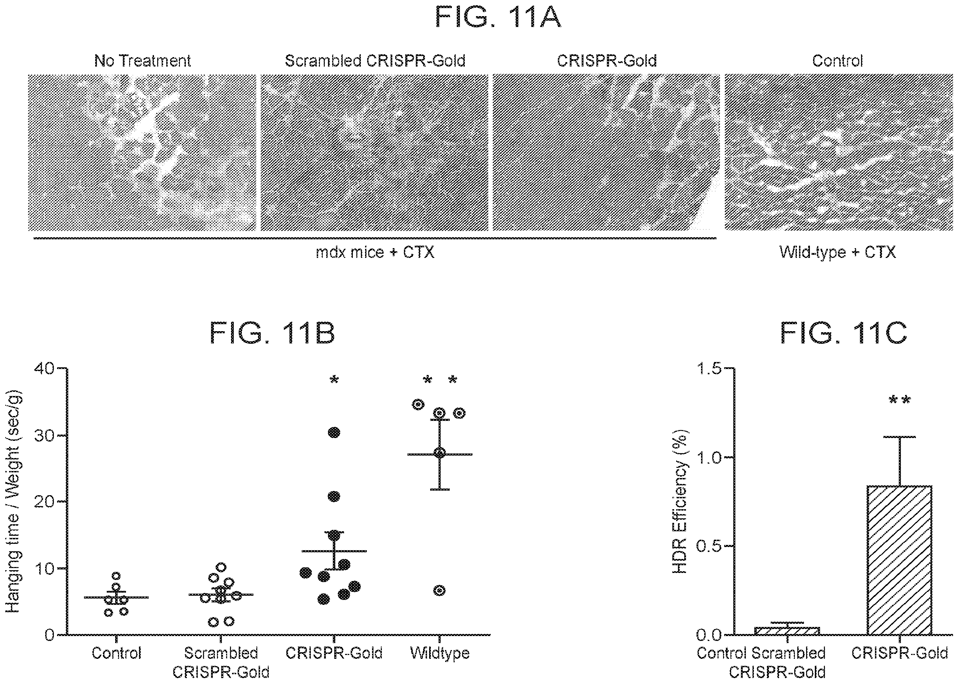

**CRISPR-Gold injection improves muscle fibrosis *in vivo.*** TA muscles of 2 months old *mdx* or wild-type mice were cryosectioned to 10 microns and stained with trichrome two weeks after injection with CRISPR-Gold and cardiotoxin. The results are depicted in Figure 11A. Sections from animals injected with cardiotoxin and a scrambled CRISP-Gold or cardiotoxin alone were used as controls. Fibrotic tissue appears in blue, while muscle fibers appear red. Qualitatively, the reduced level of fibrotic tissue staining in sections from the CRISPR-Gold and cardiotoxin treated animal indicates improved tissue health.

**CRISPR-Gold injection enhances muscle strength and agility.** Three week old *mdx* mice were injected in TA muscle, gastrocnemius muscle and forelimb muscle with 10 microns per muscle of mutation correcting nanoparticles without cardiotoxin. A four limb hanging test was conducted on 5 week old mice (2 weeks after the injection) by placing the mice on a handmade square apparatus with a grid structure. The apparatus was inverted and placed 25 cm up from the cage to discourage intentional dropping. The maximum hanging time out of three trials was recorded for the duration of 600 sec and divided by the weight of the tested mouse. Negative control mice (*mdx* mice without injection), control mice (*mdx* mice injected with CRISPR-Gold with scrambled gRNAc) and wild-type were animals were also tested at the age of 5 weeks. The results depicted in Figure 11B show a 100% increase in hanging time per weight in the four limb hanging test in comparison to *mdx* control mice. Figure 11C shows that HDR in the dystrophin gene of CRISPR-Gold treated mice occurred at a rate of 0.8% compared to the control mice. The lower rate of HDR was expected because of the absence of cardiotoxin.

### Example 8: Gene Editing Using Various GX Components

Experiments were performed to determine HDR efficiency if components of the Cas9-Gold Nanoparticle complexes were varied.

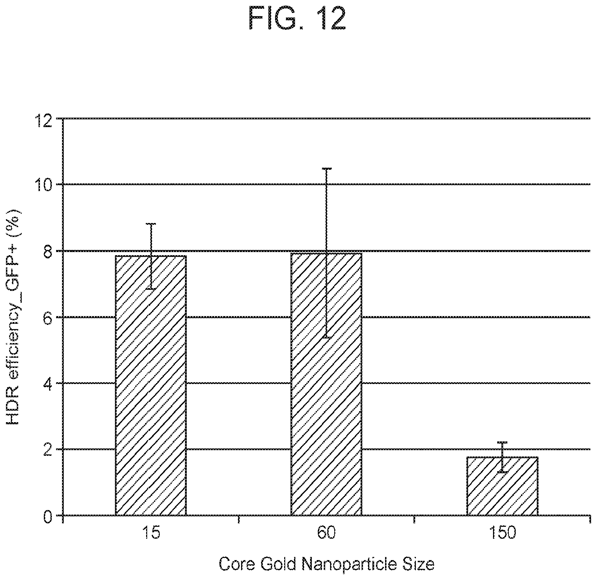

FIG 12. BFP-HEK cells were efficiently edited with CRISPR-Gold loaded onto gold nanoparticles of various sizes. 15 nm, 60 nm and 150 nm gold nanoparticles were reacted with thiol modified DNA, complexed with Cas9 and guide RNA, and encapsulated with PAsp(DET). GFP expressing populations were observed using flow cytometry. As shown in FIG. 12, using various sizes of gold nanoparticles, HDR efficiencies of from about 1.8 to about 8% were achieved.

Silver nanoparticles can deliver Cas9. DNA conjugation and particle formation using a method similar to CRISPR-Gold formation substituting gold nanoparticles with silver nanoparticles results in BFP-HEK cells efficiently edited at a rate of 4-8% gene editing.

Experiments were performed to determine if a polymer other than PAsp(DET) could be used to form GX. Replacing the endosomal disruptive polymer PAsp(DET) with polyethylenimine (PEI), Cas9-Gold nanoparticles were formed and induced HDR at a rate of 1%.

CRISPR-Gold can also deliver Cas9 derivatives. SpCas9-H1, eSpCas9 and Cpfl with its derivative crRNA were delivered and gene editing was performed using the GX delivery vehicle. Example 8: Effect of the amount of donor DNA on the HDR frequency of CRISPR-Gold treatment

CRISPR-Gold was made with various amounts of donor DNA, and added to BFP-HEK cells. Each well received 8 µg of Cas9 protein. The HDR frequency was determined by quantifying the percent of GFP+ population from CRISPR-Gold treated BFP-HEK cells. The amount of Donor DNA has a correlation with HDR efficiency. The HDR efficiency reaches a maximum at 4 µg of Donor DNA per 8 µg of Cas9. As shown in FIG. 14, the HDR frequency of CRISPR-Gold treatment is dependent on the amount of donor DNA in CRISPR-Gold.

## Claims

1. A complex comprising:
a) a nanoparticle-nucleic acid conjugate;
b) a Type II or a Type V CRISPR system comprising:
i) a Cas9 polypeptide or a Cpfl polypeptide; and
ii) a guide RNA; and
c) an endosomal disruptive polymer.

2. The complex of claim 1, wherein the nanoparticle comprises a biocompatible polymer.

3. The complex of claim 2, wherein the nanoparticle is selected from a gold nanoparticle, a silver nanoparticle, a platinum nanoparticle, an aluminum nanoparticle, a palladium nanoparticle, a copper nanoparticle, a cobalt nanoparticle, an indium nanoparticle, and a nickel nanoparticle.

4. The complex of any one of claims 1 to 3, wherein the endosomal disruptive polymer is a cationic polymer selected from the group consisting of poly-[2-{(2-aminoethyl)amino}-ethyl-aspartamide] (pAsp(DET)) polyethylene imine, poly(arginine), poly(lysine), poly(histidine), a block co-polymer of poly(ethylene glycol) (PEG) and poly(arginine), a block co-polymer of PEG and poly(lysine), and a block co-polymer of PEG and poly{*N*-[*N*-(2-aminoethyl)-2-aminoethyl]aspartamide} (PEG-pAsp(D ET)).

5. The complex of any one of claims 1 to 4, wherein the endosomal disruptive polymer is poly{*N*-[*N*-(2-aminoethyl)-2-aminoethyl]aspartamide} (PAsp(DET).

6. The complex of any one of claims 1 to 5, wherein the nanoparticle has a diameter in the range of 5 nm to 100 µm or in the range of 10 nm to 150 nm.

7. The complex of any one of claims 1 to 6, wherein the nucleic acid conjugated to the nanoparticle comprises a nucleotide sequence having at least 80% nucleotide sequence identity to a contiguous stretch of 10 to 20 nucleotides present in a target nucleic acid, or present in the guide RNA.

8. The complex of any one of claims 1 to 7, wherein the complex further comprises a donor polynucleotide, optionally wherein the donor polynucleotide comprises two sequences that hybridize to the sequence targeted by the guide RNA.

9. The complex of any one of claims 1 to 8, wherein the complex further comprises a silicate.

10. The complex of any one of claims 1 to 9, wherein the complex comprises a Cas9 polypeptide comprising an amino acid sequence having at least 75% amino acid sequence identity to an amino acid sequence set forth in FIG. 6A-6J, wherein the Cas9 polypeptide is enzymatically active, or wherein the Cas9 polypeptide exhibits reduced enzymatic activity relative to a wild-type Cas9 polypeptide and retains target nucleic acid binding activity, optionally wherein the Cas9 polypeptide comprises a nuclear localization signal.

11. The complex of any one of claims 1 to 9, wherein the complex comprises a Cpfl polypeptide comprising an amino acid sequence having at least 75% amino acid sequence identity to the amino acid sequence set forth in FIG. 9; wherein the Cpfl polypeptide is enzymatically active, or wherein the Cpfl polypeptide exhibits reduced enzymatic activity relative to a wild-type Cpfl polypeptide and retains target nucleic acid binding activity, optionally wherein the Cpfl polypeptide comprises a nuclear localization signal.

12. The complex of any one of claims 1-11, wherein the guide RNA is a single-molecule guide RNA.

13. A method of producing the complex of any of claims 1 to 12, the method comprising:
a) contacting a Type II or a Type V CRISPR system with a nanoparticle (NP)-nucleic acid conjugate, wherein the Type II CRISPR system comprises a ribonucleoprotein (RNP) comprising a Cas9 polypeptide and a guide RNA (gRNA), wherein the Type V CRISPR system comprises an RNP comprising a Cpfl polypeptide and a gRNA, wherein said contacting is carried out under conditions sufficient to generate a NP-nucleic acid-RNP complex, thereby forming a NP-nucleic acid-RNP complex; and
b) encapsulating the NP-nucleic acid-RNP complex within one or more layers of an endosomal disruptive polymer.

14. The method of claim 13, wherein the RNP further comprises a donor polynucleotide.

15. The method of claim 13, wherein nanoparticle is a colloidal metal nanoparticle, optionally wherein the nanoparticle is a gold nanoparticle.

16. The complex of any one of claims 1-12, wherein the nanoparticle is conjugated to the nucleic acid through a thiol linker.

17. The complex of claim 16, wherein the nanoparticle is a gold nanoparticle.

## Patentansprüche

1. Komplex, umfassend:
a) ein Nanopartikel-Nucleinsäure-Konjugat;
b) ein Typ-II- oder Typ-V-CRISPR-System, umfassend:
i) ein Cas9-Polypeptid oder ein Cpf1-Polypeptid; und
ii) eine Guide-RNA; und
c) ein endosomenzerstörendes Polymer.

2. Komplex nach Anspruch 1, wobei das Nanopartikel ein biokompatibles Polymer umfasst.

3. Komplex nach Anspruch 2, wobei das Nanopartikel aus einem Goldnanopartikel, einem Silbernanopartikel, einem Platinnanopartikel, einem Aluminiumnanopartikel, einem Palladiumnanopartikel, einem Kupfernanopartikel, einem Kobaltnanopartikel, einem Indiumnanopartikel und einem Nickelnanopartikel ausgewählt ist.

4. Komplex nach einem der Ansprüche 1 bis 3, wobei das endosomenzerstörende Polymer ein kationisches Polymer ist, das aus der aus Poly[2-{(2-aminoethyl)amino}ethylaspart-amid] (pAsp(DET)), Polyethylenimin, Poly(arginin), Poly(lysin), Poly(histidin), einem Blockcopolymer von Poly(ethylenglykol) (PEG) und Poly(arginin), einem Blockcopolymer von PEG und Poly(lysin) und einem Blockcopolymer von PEG und Poly-{N-[N-(2-aminoethyl)-2-aminoethyl]aspartamid} (PEG-pASp(DET)) bestehenden Gruppe ausgewählt ist.

5. Komplex nach einem der Ansprüche 1 bis 4, wobei das endosomenzerstörende Polymer Poly{N-[N-(2-aminoethyl)-2-aminoethyl]aspartamid} (PAsp(DET)) ist.

6. Komplex nach einem der Ansprüche 1 bis 5, wobei das Nanopartikel einen Durchmesser im Bereich von 5 nm bis 100 µm oder im Bereich von 10 nm bis 150 nm aufweist.

7. Komplex nach einem der Ansprüche 1 bis 6, wobei die an das Nanopartikel konjugierte Nucleinsäure eine Nucleotidsequenz mit zumindest 80 % Nucleotidsequenzidentität mit einem zusammenhängenden Stück aus 10 bis 20 Nucleotiden umfasst, das in einer Zielnucleinsäure vorhanden ist oder in der Guide-RNA vorhanden ist.

8. Komplex nach einem der Ansprüche 1 bis 7, wobei der Komplex weiters ein Donorpolynucleotid umfasst, wobei das Donorpolynucleotid gegebenenfalls zwei Sequenzen umfasst, die an die Sequenz hybridisieren, auf welche die Guide-RNA abzielt.

9. Komplex nach einem der Ansprüche 1 bis 8, wobei der Komplex weiters ein Silicat umfasst.

10. Komplex nach einem der Ansprüche 1 bis 9, wobei der Komplex ein Cas9-Polypeptid umfasst, das eine Aminosäuresequenz mit zumindest 75 % Aminosäuresequenzidentität mit einer Aminosäuresequenz wie in Fig. 6A-6J dargelegt umfasst, wobei das Cas9-Polypeptid enzymatisch aktiv ist oder wobei das Cas9-Polypeptid verringerte enzymatische Aktivität im Vergleich zu einem Wildtyp-Cas9-Polypeptid aufweist und Zielnucleinsäure-Bindungsaktivität beibehält, wobei das Cas9-Polypeptid gegebenenfalls ein Kernlokalisierungssignal umfasst.

11. Komplex nach einem der Ansprüche 1 bis 9, wobei der Komplex ein Cpf1-Polypeptid umfasst, das eine Aminosäuresequenz mit zumindest 75 % Aminosäuresequenzidentität mit der in Fig. 9 dargelegten Aminosäuresequenz umfasst; wobei das Cpf1-Polypeptid enzymatisch aktiv ist oder wobei das Cpf1-Polypeptid verringerte enzymatische Aktivität im Vergleich zu einem Wildtyp-Cpf1-Polypeptid aufweist und Zielnucleinsäure-Bindungsaktivität beibehält, wobei das Cpf1-Polypeptid gegebenenfalls ein Kernlokalisierungssignal umfasst.

12. Komplex nach einem der Ansprüche 1-11, wobei die Guide-RNA eine Guide-RNA aus einem einzelnen Molekül ist.

13. Verfahren zur Herstellung eines Komplexes nach einem der Ansprüche 1 bis 12, wobei das Verfahren Folgendes umfasst:
a) Inkontaktbringen eines Typ-II- oder Typ-V-CRISPR-Systems mit einem Nanopartikel- (NP-) Nucleinsäure-Konjugat, wobei das Typ-II-CRISPR-System ein Ribonucleoprotein (RNP) umfasst, das ein Cas9-Polypeptid und eine Guide-RNA (gRNA) umfasst, wobei das Typ-V-CRISPR-System ein RNP umfasst, das ein Cpf1-Polypeptid und eine gRNA umfasst, wobei das Inkontaktbringen unter Bedingungen ausgeführt wird, die ausreichen, um einen NP-Nucleinsäure-RNP-Komplex zu bilden, wodurch ein NP-Nucleinsäure-RNP-Komplex gebildet wird, und
b) Verkapseln des NP-Nucleinsäure-RNP-Komplexes mit einer oder mehreren Schichten eines endosomenzerstörenden Polymers.

14. Verfahren nach Anspruch 13, wobei das RNP weiters ein Donorpolynucleotid umfasst.

15. Verfahren nach Anspruch 13, wobei das Nanopartikel ein kolloidales Metallnanopartikel ist, wobei das Nanopartikel gegebenenfalls ein Goldnanopartikel ist.

16. Komplex nach einem der Ansprüche 1-12, wobei das Nanopartikel durch einen Thiol-Linker an die Nucleinsäure konjugiert ist.

17. Komplex nach Anspruch 16, wobei das Nanopartikel ein Goldnanopartikel ist.

## Revendications

1. Complexe comprenant :
a) un conjugué nanoparticule-acide nucléique ;
b) un système CRISPR de Type II ou de Type V comprenant :
i) un polypeptide Cas9 ou un polypeptide Cpf1 ; et
ii) un ARN guide ; et
c) un polymère perturbateur endosomal.

2. Complexe selon la revendication 1, dans lequel la nanoparticule comprend un polymère biocompatible.

3. Complexe selon la revendication 2, dans lequel la nanoparticule est choisie parmi une nanoparticule d'or, une nanoparticule d'argent, une nanoparticule de platine, une nanoparticule d'aluminium, une nanoparticule de palladium, une nanoparticule de cuivre, une nanoparticule de cobalt, une nanoparticule d'indium, et une nanoparticule de nickel.

4. Complexe selon l'une quelconque des revendications 1 à 3, dans lequel le polymère perturbateur endosomal est un polymère cationique choisi parmi le groupe constitué de poly-[2-{(2-aminoéthyl)amino}-éthyl-aspartamide](pAsp(DET))polyéthylène imine, poly(arginine), poly(lysine), poly(histidine), d'un copolymère bloc de poly(éthylène glycol) (PEG) et poly(arginine), d'un copolymère bloc de PEG et poly(lysine), et d'un copolymère bloc de PEG et de poly{N-[N-(2-aminoéthyl)-2-aminoéthyl]aspartamide} (PEG-pAsp(DET)).

5. Complexe selon l'une quelconque des revendications 1 à 4, dans lequel le polymère perturbateur endosomal est poly{N-[N-(2-aminoéthyl)-2-aminoéthyl]aspartamide} (PAsp(DET).

6. Complexe selon l'une quelconque des revendications 1 à 5, dans lequel la nanoparticule a un diamètre dans la plage de 5 nm à 100 µm ou dans la plage de 10 nm à 150 nm.

7. Complexe selon l'une quelconque des revendications 1 à 6, dans lequel l'acide nucléique conjugué à la nanoparticule comprend une séquence nucléotidique ayant au moins 80 % d'identité de séquence nucléotidique avec un tronçon contigu de 10 à 20 nucléotides présents dans un acide nucléique cible, ou présents dans l'ARN guide.

8. Complexe selon l'une quelconque des revendications 1 à 7, dans lequel le complexe comprend en outre un polynucléotide donneur, facultativement dans lequel le polynucléotide donneur comprend deux séquences qui s'hybrident à la séquence ciblée par l'ARN guide.

9. Complexe selon l'une quelconque des revendications 1 à 8, dans lequel le complexe comprend en outre un silicate.

10. Complexe selon l'une quelconque des revendications 1 à 9, dans lequel le complexe comprend un polypeptide Cas9 comprenant une séquence d'acides aminés ayant au moins 75 % d'identité de séquence d'acides aminés avec une séquence d'acides aminés représentée sur les Figures 6A-6J, dans lequel le polypeptide Cas9 est enzymatiquement actif, ou dans lequel le polypeptide Cas9 présente une activité enzymatique réduite par rapport à un polypeptide Cas9 de type sauvage et conserve une activité de liaison d'acide nucléique cible, facultativement dans lequel le polypeptide Cas9 comprend un signal de localisation nucléaire.

11. Complexe selon l'une quelconque des revendications 1 à 9, dans lequel le complexe comprend un polypeptide Cpf1 comprenant une séquence d'acides aminés ayant au moins 75 % d'identité de séquence d'acides aminés avec la séquence d'acides aminés représentée sur la Figure 9 ; dans lequel le polypeptide Cpf1 est enzymatiquement actif, ou dans lequel le polypeptide Cpf1 présente une activité enzymatique réduite par rapport à un polypeptide Cpf1 de type sauvage et conserve une activité de liaison d'acide nucléique cible, facultativement dans lequel le polypeptide Cpf1 comprend un signal de localisation nucléaire.

12. Complexe selon l'une quelconque des revendications 1 à 11, dans lequel l'ARN guide est un ARN guide à molécule unique.

13. Procédé de production du complexe selon l'une quelconque des revendications 1 à 12, le procédé comprenant les étapes consistant à
a) mettre en contact un système CRISPR de Type II ou de Type V avec un conjugué nanoparticule (NP)-acide nucléique dans lequel le système CRISPR de Type II comprend une ribonucléoprotéine (RNP) comprenant un polypeptide Cas9 et un ARN guide (ARNg), dans lequel le système CRISPR de Type V comprend une RNP comprenant un polypeptide Cpf1 et un ARNg, dans lequel ladite mise en contact est effectuée dans des conditions suffisantes pour générer un complexe NP-acide nucléique-RNP, formant ainsi un complexe NP-acide nucléique-RNP ; et
b) encapsuler le complexe NP-acide nucléique-RNP dans une ou plusieurs couches d'un polymère perturbateur endosomal.

14. Procédé selon la revendication 13, dans lequel la RNP comprend en outre un polynucléotide donneur.

15. Procédé selon la revendication 13, dans lequel la nanoparticule est une nanoparticule métallique colloïdale, facultativement dans lequel la nanoparticule est une nanoparticule d'or.

16. Complexe selon l'une quelconque des revendications 1 à 12, dans lequel la nanoparticule est conjuguée à l'acide nucléique par l'intermédiaire d'un liant thiol.

17. Complexe selon la revendication 16, dans lequel la nanoparticule est une nanoparticule d'or.
